(19) Europäisches Patentamt
European Patent Office
Office européen des brevets

(11) EP 3 410 508 A1

(12) EUROPEAN PATENT APPLICATION
published in accordance with Art. 153(4) EPC

(43) Date of publication:
05.12.2018 Bulletin 2018/49

(21) Application number: 17744207.6

(22) Date of filing: 24.01.2017

(51) Int Cl.:
*H01L 51/50* (2006.01)
*C07F 15/00* (2006.01)
*C07D 409/14* (2006.01)
*C07D 233/58* (2006.01)
*C09K 11/06* (2006.01)

(86) International application number:
PCT/JP2017/002377

(87) International publication number:
WO 2017/130977 (03.08.2017 Gazette 2017/31)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 29.01.2016 JP 2016015799

(71) Applicant: **Sumitomo Chemical Company Limited**
**Tokyo 104-8260 (JP)**

(72) Inventors:
- **SASADA, Toshiaki**
**Tsukuba-shi**
**Ibaraki 300-3294 (JP)**
- **ABE, Taichi**
**Tsukuba-shi**
**Ibaraki 300-3294 (JP)**
- **YOSHIDA, Tomoyasu**
**Tsukuba-shi**
**Ibaraki 300-3294 (JP)**
- **SAITO, Takakazu**
**Tsukuba-shi**
**Ibaraki 300-3294 (JP)**

(74) Representative: **J A Kemp**
**14 South Square**
**Gray's Inn**
**London WC1R 5JJ (GB)**

# (54) COMPOSITION, PHOSPHORESCENT COMPOUND, AND LIGHT-EMITTING ELEMENT

(57) Disclosed is a composition in which a phosphorescent compound represented by formula (1) and a host material are blended with each other, wherein the amount of chlorine atoms contained as impurities in the phosphorescent compound is 3.5 ppm by mass or less with respect to the total amount of solid contents blended in the composition:

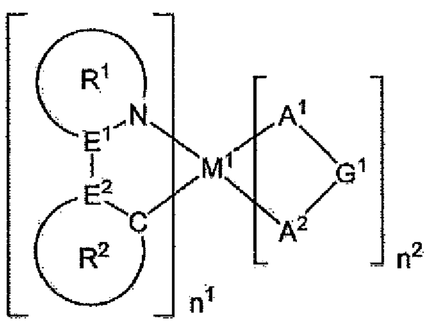

(1)

wherein, $M^1$ represents an iridium atom or the like; $n^1$ represents an integer of 1 or more, $n^2$ represents an integer of 0 or more, and $n^1 + n^2$ is 2 or 3; $E^1$ and $E^2$ represent a carbon atom or a nitrogen atom; $R^1$ ring represents a 5-membered aromatic heterocyclic ring and $R^2$ ring represents an aromatic hydrocarbon ring or the like; $A^1$-$G^1$-$A^2$ represents an anionic bidentate ligand; $A^1$ and $A^2$ represent a nitrogen atom or the like; and $G^1$ represents a single bond or the like.

EP 3 410 508 A1

## Description

### Technical Field

The present invention relates to a composition, a phosphorescent compound, and a light-emitting device.

### Background Art

An organic electroluminescent device (hereinafter also referred to as "light-emitting device") can be suitably used for display and lighting applications. Research and development into such devices is being actively carried out. This light-emitting device comprises organic layers such as a light-emitting layer and a charge transporting layer, and the like.

Patent Literature 1 describes a light-emitting device comprising a light-emitting layer comprising an iridium complex (M0) and a compound (H0) represented by the following formulas.

[Chemical Formula 1]

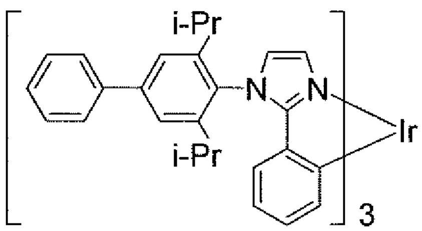

Iridium Complex (M0)

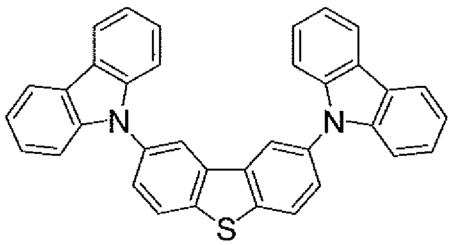

Compound (H0)

### Citation List

### Patent Literature

Patent Literature 1: US Patent Application Publication No.

### Summary of Invention

### Technical Problem

However, the light-emitting device described in Patent Literature 1 mentioned above does not always have a sufficient suppression of the initial degradation.

Therefore, it is an object of the present invention to provide a composition and a phosphorescent compound useful in the fabrication of a light-emitting device whose initial degradation is sufficiently decreased. Furthermore, it is another object of the present invention to provide a light-emitting device whose initial degradation is sufficiently decreased.

### Solution to Problem

The present inventors have studied diligently in order to achieve the objects described above and found as a result that chlorine atoms have a great effect on the initial degradation of light-emitting devices comprising an organic layer comprising a particular composition or an organic layer in which a particular phosphorescent compound is blended, and further found that the initial degradation of the light-emitting devices can be decreased by controlling the amount of chlorine atoms to be a particular amount, thereby completing the present invention. Patent Literature 1 has no statement that the amount of chlorine atoms contained in a light-emitting layer has an effect on the initial degradation.

Accordingly, the present invention provides the following [1] to [19].

[1] A composition in which a phosphorescent compound represented by formula (1) and a host material are blended with each other, wherein the amount of chlorine atoms contained as impurities in the phosphorescent compound is 3.5 ppm by mass or less with respect to the total amount of solid contents blended in the composition.

[Chemical Formula 2]

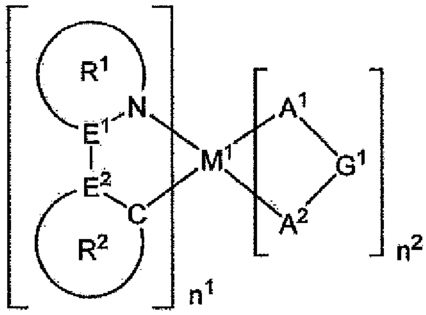

(1)

[In the formula,

$M^1$ represents a rhodium atom, a palladium atom, an iridium atom or a platinum atom.

$n^1$ represents an integer of 1 or more, $n^2$ represents an integer of 0 or more, and $n^1 + n^2$ is 2 or 3. When $M^1$ is a rhodium atom or an iridium atom, $n^1 + n^2$ is 3, and when $M^1$ is a palladium atom or a platinum atom, $n^1 + n^2$ is 2.

$E^1$ and $E^2$ each independently represent a carbon atom or a nitrogen atom, provided that at least one of $E^1$ and $E^2$ is a carbon atom.

$R^1$ ring represents a 5-membered aromatic heterocyclic ring, and the ring may have a substituent. When there are a plurality of the substituents, they may be the same or different, or may be bonded to each other to form a ring together with the atoms to which the substituents are bonded. When there are a plurality of $R^1$ rings, they may be the same or different.

$R^2$ ring represents an aromatic hydrocarbon ring or an aromatic heterocyclic ring, and these rings each may have a substituent. When there are a plurality of the substituents, they may be the same or different, or may be bonded to each other to form a ring together with the atoms to which they are bonded. When there are a plurality of $R^2$ rings, they may be the same or different.

The substituent that the $R^1$ ring may have and the substituent that the $R^2$ ring may have may be bonded to each other to form a ring together with the atoms to which the substituents are bonded.

$A^1$-$G^1$-$A^2$ represents an anionic bidentate ligand, $A^1$ and $A^2$ each independently represent a carbon atom, an oxygen atom or a nitrogen atom, and these atoms each may be atoms constituting a ring. $G^1$ represents a single bond or an atomic group constituting the bidentate ligand together with $A^1$ and $A^2$. When there are a plurality of $A^1$-$G^2$-$A^2$, they may be the same or different.]

[2] The composition according to [1], wherein the total amount of chlorine atoms contained as impurities in the phosphorescent compound and chlorine atoms contained as impurities in the host material is 3.5 ppm by mass or less with respect to the total amount of solid contents blended in the composition.

[3] The composition according to [2], wherein the total amount of chlorine atoms is 0.8 ppm by mass or less with respect to the total amount of solid contents blended in the composition.

[4] The composition according to [2], wherein the total amount of chlorine atoms is 0.01 ppm by mass or more and 3.0 ppm by mass or less with respect to the total amount of solid contents blended in the composition.

[5] The composition according to any one of [2] to [4], wherein the total amount of chlorine atoms is 0.1 ppm by mass or more and 0.8 ppm by mass or less with respect to the total amount of solid contents blended in the composition.

[6] The composition according to any one of [1] to [5], wherein the phosphorescent compound is a compound represented by formula (1-A).

[Chemical Formula 3]

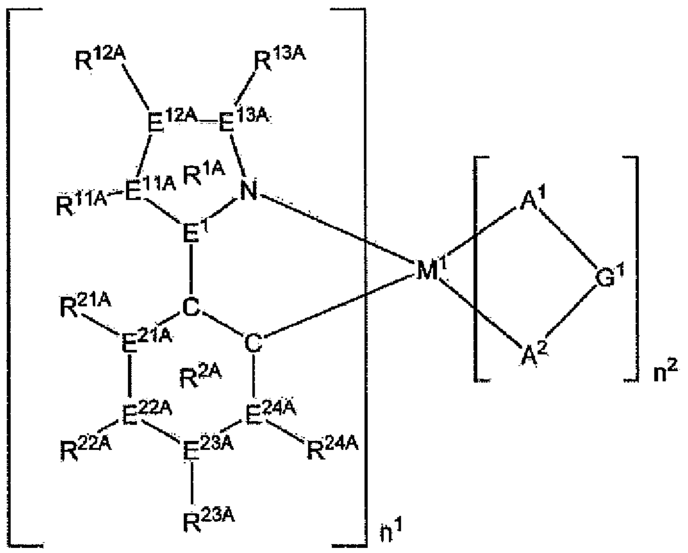

(1-A)

[In the formula,

$M^1$, $n^1$, $n^2$, $E^1$, and $A^1$-$G^1$-$A^2$ represent the same meanings as described above.

$E^{11A}$, $E^{12A}$, $E^{13A}$, $E^{21A}$, $E^{22A}$, $E^{23A}$, and $E^{24A}$ each independently represent a nitrogen atom or a carbon atom. When there are a plurality of $E^{11A}$, $E^{12A}$, $E^{13A}$, $E^{21A}$, $E^{22A}$ $E^{23A}$, and $E^{24A}$, they may be the same or different at each occurrence. When $E^{11A}$ is a nitrogen atom, $R^{11A}$ may be present or absent. When $E^{12A}$ is a nitrogen atom, $R^{12A}$ may be present or absent. When $E^{13A}$ is a nitrogen atom, $R^{13A}$ may be present or absent. When $L^{21A}$ is a nitrogen atom, $R^{21A}$ is absent. When $E^{22A}$ is a nitrogen atom, $R^{22A}$ is absent. When $E^{23A}$ is a nitrogen atom, $R^{23A}$ is absent. When $E^{24A}$ is a nitrogen atom, $R^{24A}$ is absent.

$R^{11A}$, $R^{12A}$, $R^{13A}$, $R^{21A}$, $R^{22A}$, $R^{23A}$, and $R^{24A}$ each independently represent a hydrogen atom, an alkyl group, a cycloalkyl group, an alkoxy group, a cycloalkoxy group, an aryl group, an aryloxy group, a monovalent heterocyclic group, a substituted amino group, or a fluorine atom, and these groups each may have a substituent. When there are a plurality of $R^{11A}$, $R^{12A}$, $R^{13A}$, $R^{21A}$ , $R^{22A}$ , $R^{23A}$ and $R^{24A}$ , they may each be the same or different at each occurrence. $R^{11A}$ and $R^{12A}$, $R^{12A}$ and $R^{13A}$, $R^{11A}$ and $R^{21A}$, $R^{21A}$ and $R^{22A}$, $R^{22A}$ and $R^{23A}$, and $R^{23A}$ and $R^{24A}$ each may be bonded to each other to form a ring together with the atoms to which they are bonded.

$R^{1A}$ ring represents a triazole ring or a diazole ring, constituted of a nitrogen atom, $E^1$, $E^{11A}$, $E^{12A}$, and $E^{13A}$.

$R^{2A}$ ring represents a benzene ring, a pyridine ring, or a pyrimidine ring, constituted of two carbon atoms, $E^{21A}$, $E^{22A}$, $E^{23A}$, and $E^{24A}$.]

[7] The composition according to [6], wherein the phosphorescent compound is a compound represented by formula (1-A1), a compound represented by formula (1-A2), a compound represented by formula (1-A3), or a compound represented by formula (1-A4).

[Chemical Formula 4]

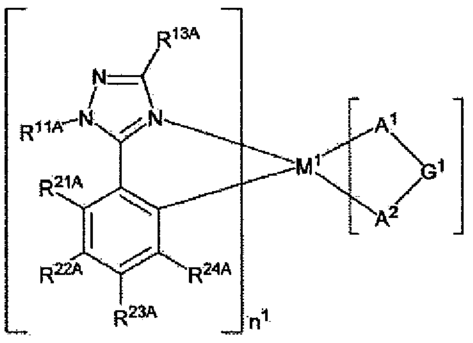

(1-A1)

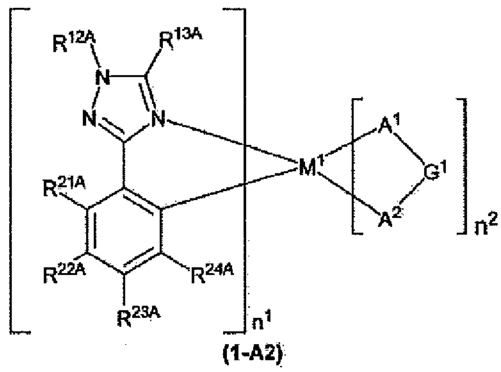

(1-A2)

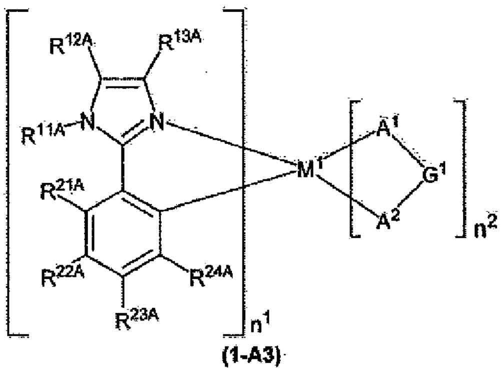

(1-A3)

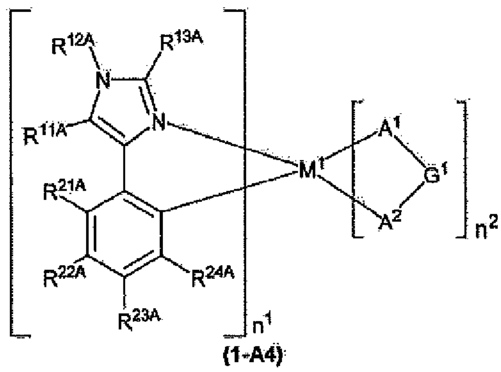

(1-A4)

[In the formula, $M^1$, $n^1$, $n^2$, $R^{11A}$, $R^{12A}$, $R^{13A}$, $R^{21A}$, $R^{22A}$, $R^{23A}$, $R^{24A}$, and $A^1$-$G^1$-$A^2$ represent the same meanings as described above.]

[8] The composition according to [7], wherein the phosphorescent compound is a compound represented by formula (1-A3) and wherein the $R^{11A}$ is an aryl group that may have a substituent.

[9] The composition according to any of [1] to [8], wherein the host material is a compound represented by formula (H-1).

[Chemical Formula 5]

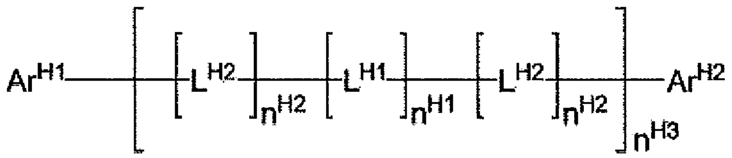

(H-1)

[In the formula,

$Ar^{H1}$ and $Ar^{H2}$ each independently represent an aryl group or a monovalent heterocyclic group, and these groups each may have a substituent.

$n^{H1}$ and $n^{H2}$ each independently represent 0 or 1. When there are a plurality of $n^{H1}$, they may be the same or different. The plurality of $n^{H2}$ may be the same or different.

$n^{H3}$ represents an integer of 0 or more.

$L^{H1}$ represents an arylene group, a divalent heterocyclic group, or a group represented by $-[C(R^{H11})_2]n^{H11}-$, and these groups each may have a substituent. When there are a plurality of $L^{H1}$, they may be the same or different.

$n^{H11}$ represents an integer of 1 or more and 10 or less. $R^{H11}$ represents a hydrogen atom, an alkyl group, a cycloalkyl group, an alkoxy group, a cycloalkoxy group, an aryl group, or a monovalent heterocyclic group, and these groups each may have a substituent. The plurality of $R^{H11}$ may be the same or different, or may be bonded to each other to form a ring together with the carbon atom to which they are bonded.

$L^{H2}$ represents a group represented by $-N(-L^{H21}-R^{H21})-$. When there are a plurality of $L^{H2}$, they may be the

same or different.

$L^{H21}$ represents a single bond, an arylene group, or a divalent heterocyclic group, and these groups each may have a substituent. $R^{H21}$ represents a hydrogen atom, an alkyl group, a cycloalkyl group, an aryl group, or a monovalent heterocyclic group, and these groups each may have a substituent.]

[10] The composition according to any one of [1] to [8], further comprising at least one material selected from the group consisting of a hole transporting material, a hole injecting material, an electron transporting material, an electron injecting material, a light emitting material, an antioxidant, and a solvent.

[11] A phosphorescent compound represented by formula (1), wherein the amount of chlorine atoms contained as impurities is 15 ppm by mass or less with respect to the total amount of the phosphorescent compound.

[Chemical Formula 6]

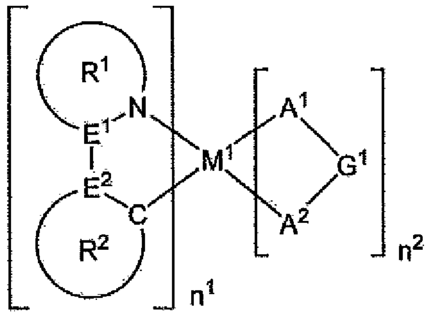

(1)

[In the formula,

$M^1$ represents a rhodium atom, a palladium atom, an iridium atom, or a platinum atom.

$n^1$ represents an integer of 1 or more, $n^2$ represents an integer of 0 or more, and $n^1 + n^2$ is 2 or 3. When $M^1$ is a rhodium atom or an iridium atom, $n^1 + n^2$ is 3. When $M^1$ is a palladium atom or a platinum atom, $n^1 + n^2$ is 2.

$E^1$ and $E^2$ each independently represent a carbon atom or a nitrogen atom, provided that at least one of $E^1$ and $E^2$ is a carbon atom.

$R^1$ ring represents a 5-membered aromatic heterocyclic ring, and the ring may have a substituent. When there are a plurality of the substituents, they may be the same or different, or may be bonded to each other to form a ring together with the atoms to which they are bonded. When there are a plurality of $R^1$ rings, they may be the same or different.

$R^2$ ring represents an aromatic hydrocarbon ring or an aromatic heterocyclic ring, and these rings each may have a substituent. When there are a plurality of substituents, they may be the same or different, or may be bonded to each other to form a ring together with the atoms to which they are bonded. When there are a plurality of $R^2$ rings, they may be the same or different.

The substituent that the $R^1$ ring may have and the substituent that the $R^2$ ring may have may be bonded to each other to form a ring together with the atoms to which they are bonded.

$A^1$-$G^1$-$A^2$ represents an anionic bidentate ligand. $A^1$ and $A^2$ each independently represent a carbon atom, an oxygen atom, or a nitrogen atom, and these atoms each may be atoms constituting a ring. $G^1$ represents a single bond or an atomic group constituting the bidentate ligand together with $A^1$ and $A^2$. When there are a plurality of $A^1$-$G^1$-$A^2$, they may be the same or different.]

[12] The phosphorescent compound according to [11], wherein the phosphorescent compound represented by formula (1) is a compound represented by formula (1-A).

[Chemical Formula 7]

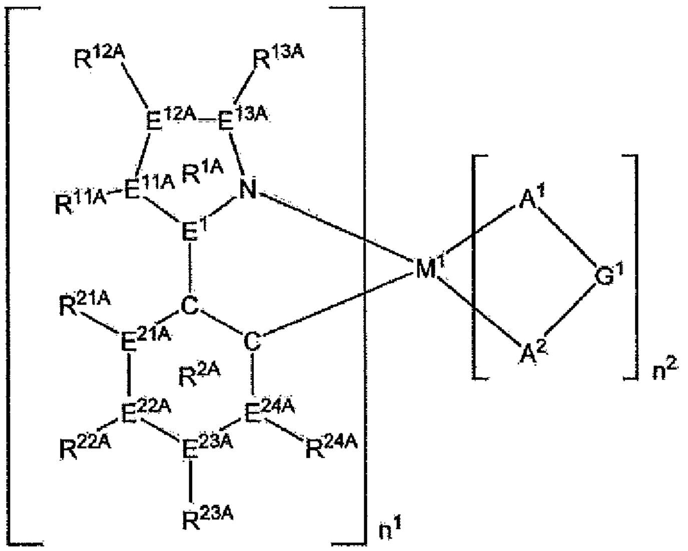

(1-A)

[In the formula,

$M^1$, $n^1$, $n^2$, $E^1$, and $A^1$-$G^1$-$A^2$ represent the meanings the same as described above.

$E^{11A}$, $E^{12A}$, $E^{13A}$, $E^{21A}$, $E^{22A}$, $E^{23A}$, and $E^{24A}$ each independently represent a nitrogen atom or a carbon atom. When there are a plurality of $E^{11A}$, $E^{12A}$, $E^{13A}$, $E^{21A}$, $E^{22A}$, $E^{21A}$, and $E^{24A}$, they may each be the same or different at each occurrence. When $E^{11A}$ is a nitrogen atom, $R^{11A}$ may be present or absent. When $E^{12A}$ is a nitrogen atom, $R^{12A}$ may be present or absent. When $E^{13A}$ is a nitrogen atom, $R^{13A}$ may be present or absent. When $E^{21A}$ is a nitrogen atom, $R^{21A}$ is absent. When $E^{22A}$ is a nitrogen atom, $R^{22A}$ is absent. When $E^{23A}$ is a nitrogen atom, $R^{23A}$ is absent. When $E^{24A}$ is a nitrogen atom, $R^{24A}$ is absent.

$R^{11A}$, $R^{12A}$, $R^{13A}$, $R^{21A}$, $R^{22A}$, $R^{23A}$ and $R^{24A}$ each independently represent a hydrogen atom, an alkyl group, a cycloalkyl group, an alkoxy group, a cycloalkoxy group, an aryl group, an aryloxy group, a monovalent heterocyclic group, a substituted amino group, or a fluorine atom, and these groups each may have a substituent. When there are a plurality of $R^{11A}$, $R^{12A}$, $R^{13A}$, $R^{21A}$, $R^{22A}$, $R^{23A}$ and $R^{24A}$ , they may each be the same or different at each occurrence. $R^{11A}$ and $R^{12A}$, $R^{12A}$ and $R^{13A}$, $R^{11A}$ and $R^{21A}$, $R^{21A}$ and $R^{22A}$, $R^{22A}$ and $R^{23A}$, and $R^{23A}$ and $R^{24A}$ each may be bonded to each other to form a ring together with the atoms to which they are bonded.

$R^{1A}$ ring represents a triazole ring or a diazole ring, constituted of a nitrogen atom, $E^1$, $E^{11A}$, $E^{12A}$, and $E^{13A}$.

$R^{2A}$ ring represents a benzene ring, a pyridine ring, or a pyrimidine ring, constituted of two carbon atoms, $E^{21A}$, $E^{22A}$, $E^{23A}$, and $E^{24A}$.]

[13] The phosphorescent compound according to [12], wherein the phosphorescent compound is a compound represented by formula (1-A1), a compound represented by formula (1-A2), a compound represented by formula (1-A3), or a compound represented by formula (1-A4).

[Chemical Formula 8]

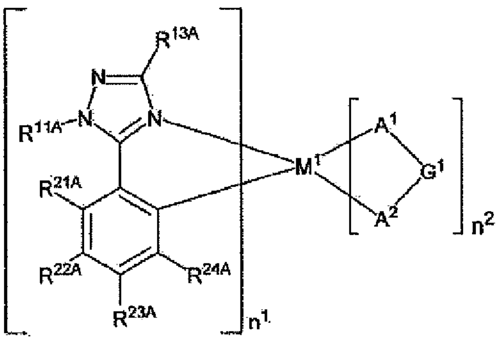

(1-A1)

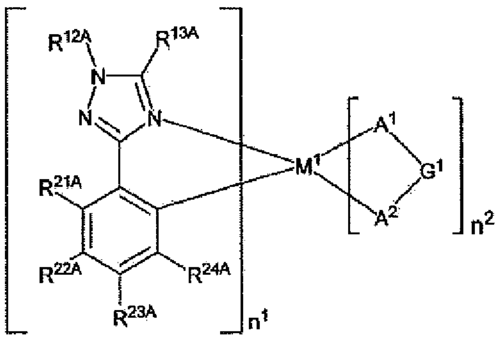

(1-A2)

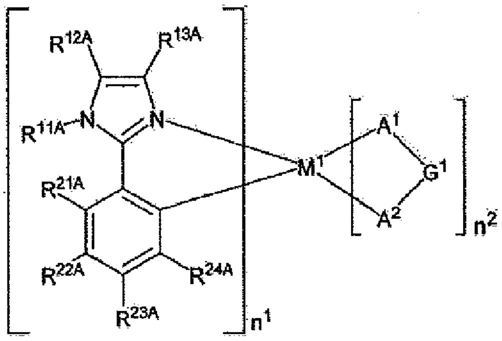

(1-A3)

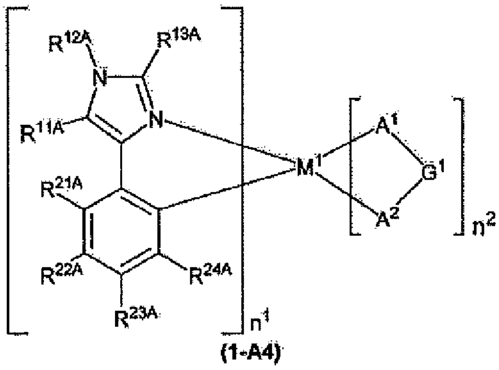

(1-A4)

[In the formula, $M^1$, $n^1$, $n^2$, $R^{11A}$, $R^{12A}$, $R^{13A}$, $R^{21A}$, $R^{22A}$, $R^{23A}$, $R^{24A}$, and $A^1$-$G^1$-$A^2$ represent the meanings the same as described above.]

[14] The phosphorescent compound according to [13], wherein the phosphorescent compound is a compound represented by formula (1-A3) and wherein the $R^{11A}$ is an aryl group that may have a substituent.

[15] The phosphorescent compound according to any one of [11] to [14], wherein the amount of chlorine atoms contained as the impurities is 0.9 ppm by mass or less.

[16] The phosphorescent compound according to any one of [11] to [14], wherein the amount of chlorine atoms contained as the impurities is 0.01 ppm by mass or more and 12 ppm by mass or less.

[17] The phosphorescent compound according to [15] or [16], wherein the amount of chlorine atoms contained as the impurities is 0.1 ppm by mass or more and 0.9 ppm by mass or less.

[18] A light-emitting device comprising an organic layer comprising a composition according to any of [1] to [10].

[19] A light-emitting device comprising an organic layer in which a phosphorescent compound according to any one of [11] to [17] is blended.

**Advantageous Effects of Invention**

According to the present invention, a composition and a phosphorescent compound useful in the fabrication of a light-emitting device whose initial degradation is sufficiently suppressed can be provided. Moreover, according to the present invention, a light-emitting device whose initial degradation is sufficiently suppressed can be provided.

**Description of Embodiments**

Preferable embodiments of the present invention will now be described in detail.

<Description of Common Terms>

Unless otherwise stated, terms commonly used in the present specification have the following meanings.

Me represents a methyl group, Et represents an ethyl group, Bu represents a butyl group, i-Pr represents an isopropyl group, and t-Bu represents a tert-butyl group.

The hydrogen atom may be a heavy hydrogen atom or a light hydrogen atom.

In the formula representing a phosphorescent compound and a metal complex, a solid line representing a bond

to the central metal means a covalent bond or a coordinate bond.

The term "polymer compound" means a polymer having molecular weight distribution and having a polystyrene-equivalent number-average molecular weight of $1 \times 10^3$ to $1 \times 10^8$.

The polymer compound may be any of a block copolymer, a random copolymer, an alternating copolymer, and a graft copolymer, or may even be some other form.

If a polymerization active group remains intact at the terminal group of the polymer compound, light-emitting properties and luminescence lifetime may deteriorate if such a polymer compound is used to fabricate the light-emitting device, and therefore, it is preferable that the terminal group be a stable group. This terminal group is preferably a group covalently bonded to the main chain, and examples thereof include groups bonding to an aryl group or a monovalent heterocyclic group via a carbon-carbon bond.

The term "low molecular weight compound" means a compound that does not have a molecular weight distribution and that has a molecular weight of $1 \times 10^4$ or less.

The term "constitutional unit" means a unit occurring one or more times in a polymer compound.

The term "alkyl group" may be either linear or branched. The linear alkyl group usually has 1 to 50 carbon atoms, preferably 3 to 30 carbon atoms, and more preferably 4 to 20 carbon atoms, not including the carbon atoms of the substituent. The branched alkyl group usually has 3 to 50 carbon atoms, preferably 3 to 30 carbon atoms, and more preferably 4 to 20 carbon atoms, not including the carbon atoms of the substituent.

The alkyl group may have a substituent, and examples thereof include a methyl group, an ethyl group, a propyl group, an isopropyl group, a butyl group, a 2-butyl group, an isobutyl group, a tert-butyl group, a pentyl group, an isoamyl group, 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group, and a dodecyl group, and groups obtained by substituting a hydrogen atom of these groups with a cycloalkyl group, an alkoxy group, a cycloalkoxy group, an aryl group, a fluorine atom, and the like, and examples thereof include a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group, and a 6-ethyloxyhexyl group.

The term "cycloalkyl group" usually has 3 to 50 carbon atoms, preferably 3 to 30 carbon atoms, and more preferably 4 to 20 carbon atoms, not including the carbon atoms of the substituent.

The cycloalkyl group may have a substituent, and examples thereof include a cyclohexyl group, a cyclohexylmethyl group, and a cyclohexylethyl group.

The term "aryl group" means the atomic group remaining after removing from an aromatic hydrocarbon one hydrogen atom that is directly bonded to a carbon atom constituting the ring. The aryl group usually has 6 to 60 carbon atoms, preferably 6 to 20 carbon atoms, and more preferably 6 to 10 carbon atoms, not including the carbon atoms of the substituent.

The aryl group may have a substituent, and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group, and a 4-phenylphenyl group, and groups obtained by substituting a hydrogen atom of these groups with an alkyl group, a cycloalkyl group, an alkoxy group, a cycloalkoxy group, an aryl group, a fluorine atom, and the like.

The term "alkoxy group" may be any of linear and branched. The linear alkoxy group usually has 1 to 40 carbon atoms, and preferably 4 to 10 carbon atoms, not including the carbon atoms of the substituent. The branched alkoxy group usually has 3 to 40 carbon atoms, and preferably 4 to 10 carbon atoms, not including the carbon atoms of the substituent.

The alkoxy group may have a substituent, and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, an octyloxy group, a 2-ethylhexyloxy group, a nonyloxy group, a decyloxy group, a 3,7-dimethyloctyloxy group, and a lauryloxy group, and groups obtained by substituting a hydrogen atom of these groups with a cycloalkyl group, an alkoxy group, a cycloalkoxy group, an aryl group, a fluorine atom, and the like.

The term "cycloalkoxy group" usually has 3 to 40 carbon atoms, and preferably 4 to 10 carbon atoms, not including the carbon atoms of the substituent.

The cycloalkoxy group may have a substituent, and examples thereof include a cyclohexyloxy group.

The term "aryloxy group" usually has 6 to 60 carbon atoms, and preferably 6 to 48, not including the carbon atoms of the substituent.

The aryloxy group may have a substituent, and examples thereof include a phenoxy group, a 1-naphthyloxy group, a 2-naphthyloxy group, a 1-anthracenyloxy group, a 9-anthracenyloxy group, and a 1-pyrenyloxy group, and groups obtained by substituting a hydrogen atom of these groups with an alkyl group, a cycloalkyl group, an alkoxy group, a cycloalkoxy group, a fluorine atom, and the like.

The term "p-valent heterocyclic group" (p represents an integer of 1 or more) means an atomic group remaining after removing from a heterocyclic compound p atoms of hydrogen among the hydrogen atoms that are directly bonded

to carbon atoms or hetero atoms constituting the ring. Among p-valent heterocyclic groups, preferable are "p-valent aromatic heterocyclic groups", which are the atomic groups remaining after p atoms of hydrogen among the hydrogen atoms directly bonded to carbon atoms or hetero atoms constituting the ring are removed from an aromatic heterocyclic compound.

The term "aromatic heterocyclic compound" means, for example, a compound in which the heterocyclic ring itself exhibits aromaticity, such as oxadiazole, thiadiazole, thiazole, oxazole, thiophene, pyrrole, phosphole, furan, pyridine, pyrazine, pyrimidine, triazine, pyridazine, quinoline, isoquinoline, carbazole, dibenzophosphole, and the like, or a compound in which an aromatic ring is condensed to a heterocyclic ring even if the heterocyclic ring itself does not exhibit aromaticity, such as phenoxazine, phenothiazine, dibenzoborole, dibenzosilole, benzopyran, and the like.

The monovalent heterocyclic group usually has 2 to 60 carbon atoms, and preferably 4 to 20 carbon atoms, not including the carbon atoms of the substituent.

The monovalent heterocyclic group may have a substituent, and examples thereof include a thienyl group, a pyrrolyl group, a furyl group, a pyridyl group, a piperidinyl group, a quinolinyl group, an isoquinolinyl group, a pyrimidinyl group and a triazinyl group, and groups obtained by substituting a hydrogen atom of these groups with an alkyl group, a cycloalkyl group, an alkoxy group, a cycloalkoxy group, and the like.

The term "halogen atom" means a fluorine atom, a chlorine atom, a bromine atom, or an iodine atom.

The term "amino group" may have a substituent, and a substituted amino group is preferable. As the substituent that the amino group has, an alkyl group, a cycloalkyl group, an aryl group, or a monovalent heterocyclic group is preferable.

Examples of the substituted amino group include a dialkylamino group, a dicycloalkylamino group, and a diarylamino group.

Examples of the amino group include a dimethylamino group, a diethylamino group, a diphenylamino group, a bis(4-methylphenyl)amino group, a bis(4-tert-butylphenyl)amino group, and a bis(3,5-di-tert-butylphenyl)amino group.

The term "alkenyl group" may be any of linear and branched. The linear alkenyl group usually has 2 to 30 carbon atoms, and preferably 3 to 20 carbon atoms, not including the carbon atoms of the substituent. The branched alkenyl group usually has carbon atoms 3 to 30, and preferably 4 to 20 carbon atoms, not including the carbon atoms of the substituent.

The term "cycloalkenyl group" usually has 3 to 30 carbon atoms, and preferably 4 to 20 carbon atoms, not including the carbon atoms of the substituent.

The alkenyl group and the cycloalkenyl group each may have a substituent, and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group, and a 7-octenyl group, and these groups having a substituent.

The term "alkynyl group" may be any of linear and branched. The alkynyl group usually has 2 to 20 carbon atoms, and preferably 3 to 20 carbon atoms, not including the carbon atoms of the substituent. The branched alkynyl group usually has 4 to 30 carbon atoms, and preferably 4 to 20 carbon atoms, not including the carbon atoms of the substituent.

The term "cycloalkynyl group" usually has 4 to 30 carbon atoms, and preferably 4 to 20 carbon atoms, not including the carbon atoms of the substituent.

The alkynyl group and the cycloalkynyl group each may have a substituent, and examples thereof include an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and these groups having a substituent.

The term "arylene group" means an atomic group remaining after removing from an aromatic hydrocarbon two hydrogen atoms that are directly bonded to a carbon atom constituting the ring. The arylene group usually has 6 to 60 carbon atoms, preferably 6 to 30 carbon atoms, and more preferably 6 to 18 carbon atoms, not including the carbon atoms of the substituent.

The arylene group may have a substituent, and examples thereof include a phenylene group, a naphthalenediyl group, an anthracenediyl group, a phenanthrenediyl group, a dihydrophenanthrenediyl group, a naphthacenediyl group, a fluorenediyl group, a pyrenediyl group, a perylenediyl group, a chrysenediyl group, and these groups having a substituent, and preferable are groups represented by formulas (A-1) to (A-20). The arylene group may be a group obtained by bonding a plurality of these groups.

[Chemical Formula 9]

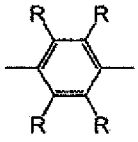
(A-1)
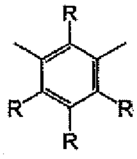
(A-2)
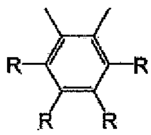
(A-3)
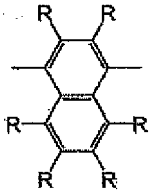
(A-4)
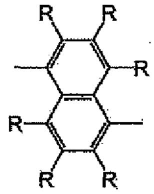
(A-5)
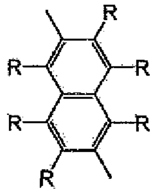
(A-6)

[Chemical Formula 10]

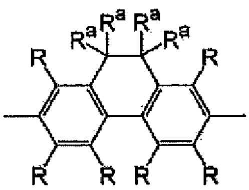
(A-7)
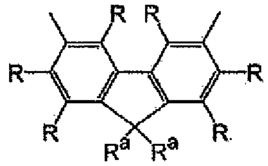
(A-8)
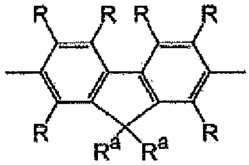
(A-9)
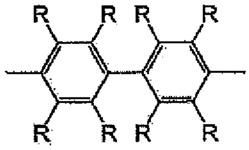
(A-10)

[Chemical Formula 11]

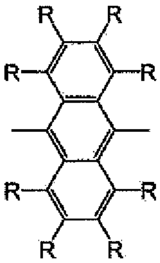
(A-11)
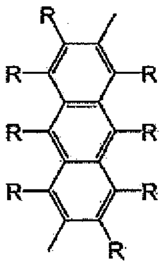
(A-12)
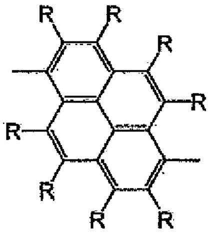
(A-13)
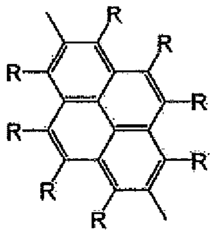
(A-14)
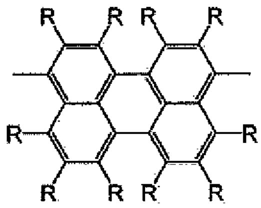
(A-15)

[Chemical Formula 12]

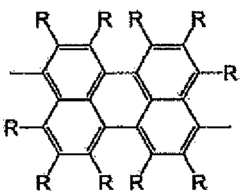
(A-16)
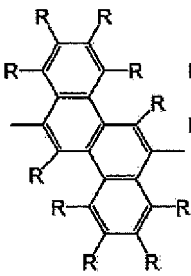
(A-17)
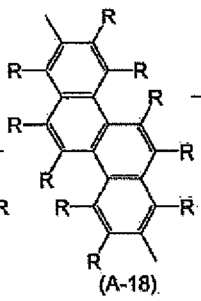
(A-18)
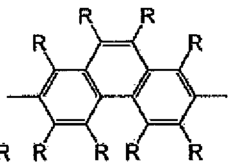
(A-19)
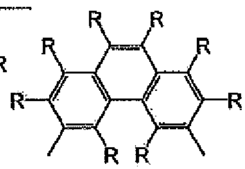
(A-20)

[In the formula, R and $R^a$ each independently represent a hydrogen atom, an alkyl group, a cycloalkyl group, an aryl group or a monovalent heterocyclic group. The plurality of R and $R^a$ each may be the same or different. The plurality of $R^a$ may be bonded to each other to form a ring together with the atoms to which they are bonded.]

The divalent heterocyclic group usually has 2 to 60 carbon atoms, preferably 3 to 20 carbon atoms, and more preferably 4 to 15 carbon atoms, not including the carbon atoms of the substituent.

The divalent heterocyclic group may have a substituent, and examples thereof include divalent groups obtained by removing from pyridine, diazabenzene, triazine, azanaphthalene, diazanaphthalene, carbazole, dibenzofuran, dibenzothiophene, dibenzosilole, phenoxazine, phenothiazine, acridine, dihydroacridine, furan, thiophene, azole, diazole and triazole two hydrogen atoms among the hydrogen atoms directly bonded to a carbon atom or a hetero atom constituting the ring. Preferable are groups represented by formulas (AA-1) to (AA-34). The divalent heterocyclic group may be a group obtained by bonding a plurality of these groups.

[Chemical Formula 13]

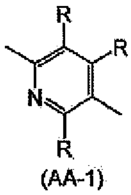
(AA-1)
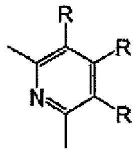
(AA-2)
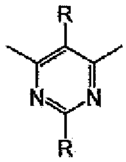
(AA-3)
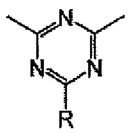
(AA-4)
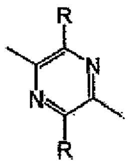
(AA-5)
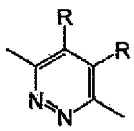
(AA-6)
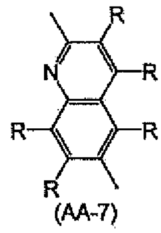
(AA-7)

[Chemical Formula 14]

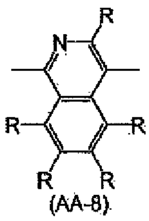
(AA-8)
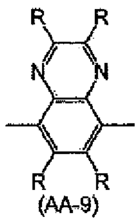
(AA-9)
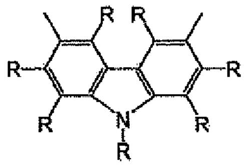
(AA-10)
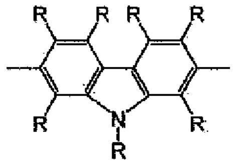
(AA-11)
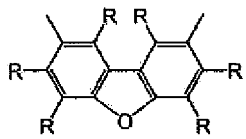
(AA-12)

[Chemical Formula 15]

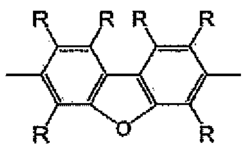
(AA-13)
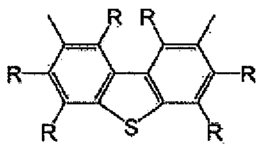
(AA-14)
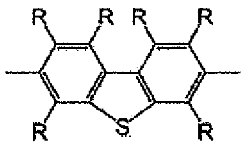
(AA-15)
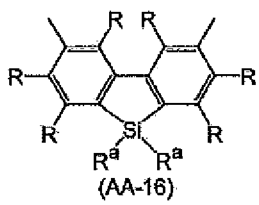
(AA-16)

[Chemical Formula 16]

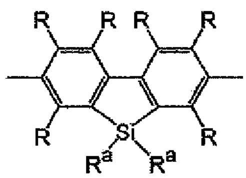
(AA-17)
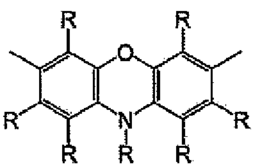
(AA-18)
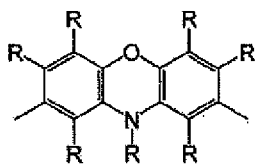
(AA-19)
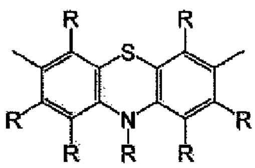
(AA-20)

[Chemical Formula 17]

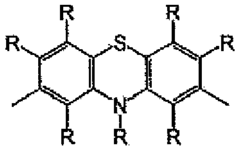
(AA-21)
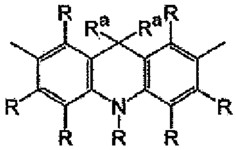
(AA-22)
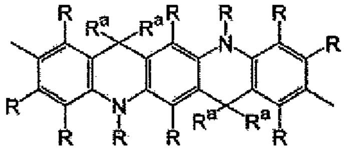
(AA-23)
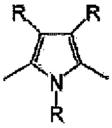
(AA-24)
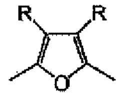
(AA-25)

[Chemical Formula 18]

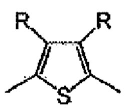
(AA-26)
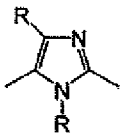
(AA-27)
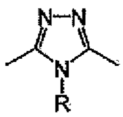
(AA-28)
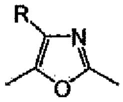
(AA-29)
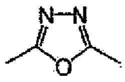
(AA-30)
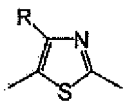
(AA-31)
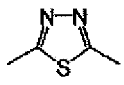
(AA-32)

[Chemical Formula 19]

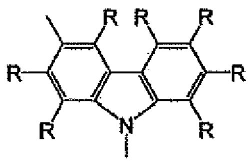
(AA-33)
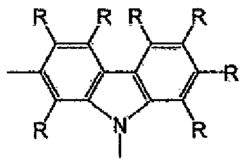
(AA-34)

[In the formula, R and $R^a$ represent the same meanings as described above.]

The term "crosslinkable group" means a group capable of producing a new bond when subjected to heat treatment, UV-irradiation treatment, near-UV irradiation treatment, visible light irradiation treatment, infrared irradiation treatment, a radical reaction, and the like. Preferable is a crosslinkable group represented by formulas (XL-1) to (XL-17) of the group of crosslinkable groups A.

(Group of Crosslinkable Groups A)

[Chemical Formula 20]

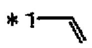
(XL-1)
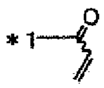
(XL-2)
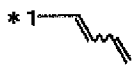
(XL-3)
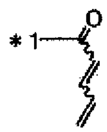
(XL-4)
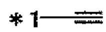
(XL-5)
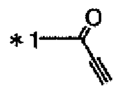
(XL-6)
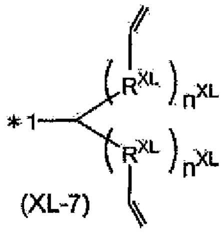
(XL-7)
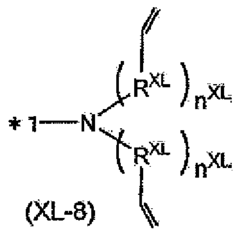
(XL-8)
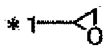
(XL-9)
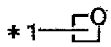
(XL-10)
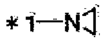
(XL-11)
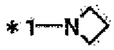
(XL-12)
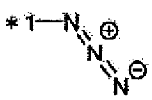
(XL-13)
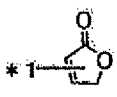
(XL-14)
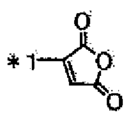
(XL-15)
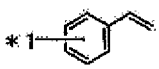
(XL-16)
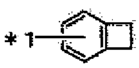
(XL-17)

[In the formula, $R^{XL}$ represents a methylene group, an oxygen atom, or a sulfur atom, and $n^{XL}$ represents an integer of 0 to 5. When there are a plurality of $R^{XL}$, they may be the same or different, and when there are a plurality of $n^{XL}$, they may be the same or different. *1 represents a bonding position. These crosslinkable groups each may have a substituent.]

The term "substituent" represents a fluorine atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group, or a cycloalkynyl group. The substituent may also be a crosslinkable group.

"Chlorine" means an element having an atomic number of 17. "Bromine" means an element having an atomic number of 35.

[Phosphorescent Compound Represented by Formula (1)]

First, the phosphorescent compound represented by formula (1) which is blended in the composition according to the present embodiment will be described.

The phosphorescent compound represented by formula (1) is a compound (metal complex) that exhibits phosphorescence luminescence usually at room temperature (25°C), and preferably a compound that exhibits luminescence from the excited triplet state at room temperature (25°C).

The phosphorescent compound represented by formula (1) is preferably a compound whose maximum peak wavelength of the emission spectrum is 380 nm or more and less than 495 nm , more preferably a compound whose maximum peak wavelength of the emission spectrum is 400 nm or more and less than 490 nm, further preferably a compound whose maximum peak wavelength of the emission spectrum is 420 nm or more and less than 485 nm, particularly preferably a compound whose maximum peak wavelength of the emission spectrum is 440 nm or more and less than 480 nm, and more particularly preferably a compound whose maximum peak wavelength of the emission spectrum is 460 nm or more and less than 475 nm.

The maximum peak wavelength of the emission spectrum of the phosphorescent compound can be evaluated by dissolving the phosphorescent compound in an organic solvent such as xylene, toluene, chloroform, or tetrahydrofuran to prepare a dilute solution ($1 \times 10^{-6}$ to $1 \times 10^{-3}$ % by mass), and measuring the PL spectrum of the dilute solution at room temperature. Xylene is preferable as the organic solvent for dissolving the phosphorescent compound.

The phosphorescent compound represented by formula (1) is constituted of $M^1$, which is a central metal, a ligand whose number is defined by the suffix $n^1$, and a ligand whose number is defined by the suffix $n^2$.

$M^1$ is preferably an iridium atom or a platinum atom, and more preferably an iridium atom, because the initial degradation of the light-emitting device according to the present embodiment is more suppressed.

When $M^1$ is a rhodium atom or an iridium atom, $n^1$ is preferably 2 or 3, and more preferably 3.

When $M^1$ is a palladium atom or a platinum atom, $n^1$ is preferably 2.

$E^1$ and $E^2$ are preferably carbon atoms.

The $R^1$ ring is preferably a 5-membered aromatic heterocyclic ring having 2 or more and 4 or less nitrogen atoms as ring atoms thereof, more preferably a diazole ring or a triazole ring, and further preferably a diazole ring, and these rings each may have a substituent.

The $R^2$ ring is preferably a 5-membered or 6-membered aromatic hydrocarbon ring or a 5-membered or 6-membered aromatic heterocyclic ring, more preferably a 6-membered aromatic hydrocarbon ring or a 6-membered aromatic heterocyclic ring, and further preferably a 6-membered aromatic hydrocarbon ring, and these rings each may have a substituent, provided that when the $R^2$ ring is a 6-membered aromatic heterocyclic ring, $E^2$ is a carbon atom.

Examples of the $R^2$ ring include a benzene ring, a naphthalene ring, a fluorene ring, a phenanthrene ring, an indene ring, a pyridine ring, a diazabenzene ring, and a triazine ring. The $R^2$ ring is preferably a benzene ring, a naphthalene ring, a fluorene ring, a pyridine ring, or a pyrimidine ring, more preferably a benzene ring, pyridine ring, or pyrimidine ring and further preferably a benzene ring, and these rings each may have a substituent.

A substituent that the $R^1$ ring and the $R^2$ ring may have is preferably a fluorine atom, an alkyl group, a cycloalkyl group, an alkoxy group, a cycloalkoxy group, an aryloxy group, an aryl group, a monovalent heterocyclic group, or a substituted amino group, more preferably an alkyl group, a cycloalkyl group, an alkoxy group, an aryl group, a monovalent heterocyclic group, or a substituted amino group, further preferably an alkyl group, a cycloalkyl group, an aryl group, or a monovalent heterocyclic group, and particularly preferably an aryl group, and these groups each may further have a substituent.

The aryl group in a substituent that the $R^1$ ring and the $R^2$ ring may have is preferably a phenyl group, a naphthyl group, an anthracenyl group, a phenanthrenyl group, a dihydrophenanthrenyl group, a fluorenyl group, or a pyrenyl group, more preferably a phenyl group, a naphthyl group or a fluorenyl group, and still more preferably a phenyl group, and these groups each may have a substituent.

Preferable examples of the monovalent heterocyclic group in a substituent that the $R^1$ ring and the $R^2$ ring may have include a pyridyl group, a pyrimidinyl group, a triazinyl group, a quinolinyl group, an isoquinolinyl group, a dibenzofuranyl group, a dibenzothienyl group, a carbazolyl group, an azacarbazolyl group, a diazacarbazolyl group, a phenoxazinyl group, and a phenothiazinyl group, more preferable are a pyridyl group, a pyrimidinyl group, a triazinyl group,

a carbazolyl group, an azacarbazolyl group, and a diazacarbazolyl group, and still more preferable are a pyridyl group, a pyrimidinyl group and a triazinyl group, and these groups each may have a substituent.

In the substituted amino group of a substituent that the $R^1$ ring and the $R^2$ ring may have, the substituent possessed by the amino group is preferably an aryl group or a monovalent heterocyclic group, and these groups each may further have a substituent. The examples and preferable ranges of the aryl group in the substituent that the amino group has are the same as the examples and preferable ranges of the aryl group in a substituent that the $R^1$ ring and the $R^2$ ring may have. The examples and preferable ranges of the monovalent heterocyclic group in the substituent that the amino group has are the same as the examples and preferable ranges of the monovalent heterocyclic group in a substituent that the $R^1$ ring and the $R^2$ ring may have.

The further optional substituents of a substituent that the $R^1$ ring and the $R^2$ ring may have are preferably an alkyl group, a cycloalkyl group, an aryl group, a monovalent heterocyclic group, an alkoxy group, a cycloalkoxy group, an aryloxy group, or a substituted amino group, more preferably an alkyl group, a cycloalkyl group, an aryl group, a monovalent heterocyclic group, or a substituted amino group, still more preferably an alkyl group, a cycloalkyl group, an aryl group, or a monovalent heterocyclic group, and particularly preferably an alkyl group, a cycloalkyl group, or an aryl group, and these groups each may further have a substituent.

The aryl group, monovalent heterocyclic group, or substituted amino group in a substituent that the $R^1$ ring and the $R^2$ ring may have is preferably a group represented by formula (D-A), (D-B), or (D-C), more preferably a group represented by formula (D-A) or (D-C), and particularly preferably a group represented by formula (D-C), because the initial degradation of the light-emitting device according to the present embodiment is more suppressed.

[Chemical Formula 21]

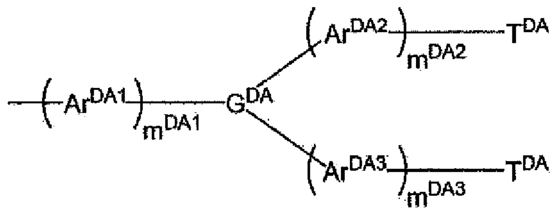

**(D-A)**

[In the formula,

$m^{DA1}$, $m^{DA2}$, and $m^{DA3}$ each independently represent an integer of 0 or more.

$G^{DA}$ represents a nitrogen atom, an aromatic hydrocarbon group, or a heterocyclic group, and these groups each may have a substituent.

$Ar^{DA1}$, $Ar^{DA2}$, and $Ar^{DA3}$ each independently represent an arylene group or a divalent heterocyclic group, and these groups each may have a substituent. When there are a plurality of $Ar^{DA1}$, $Ar^{DA2}$, and $Ar^{DA3}$, they may each be the same or different at each occurrence.

$T^{DA}$ represents an aryl group or a monovalent heterocyclic group, and these groups each may have a substituent. The plurality of $T^{DA}$ may be the same or different.]

[Chemical Formula 22]

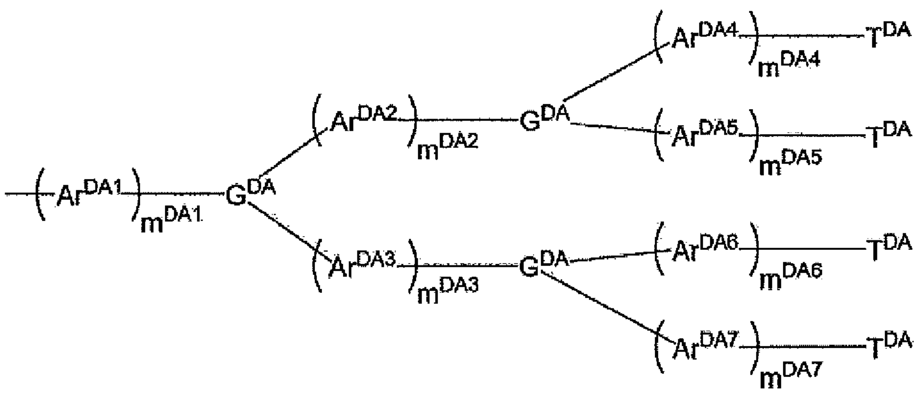

**(D-B)**

[In the formula,

$m^{DA1}$, $m^{DA2}$, $m^{DA3}$, $m^{DA4}$, $m^{DA5}$, $m^{DA6}$, and $m^{DA7}$ each independently represent an integer of 0 or more.
$G^{DA}$ represents a nitrogen atom, an aromatic hydrocarbon group, or a heterocyclic group, and these groups each may have a substituent. The plurality of $G^{DA}$ may be the same or different.
$Ar^{DA1}$, $Ar^{DA2}$, $Ar^{DA3}$, $Ar^{DA4}$, $Ar^{DA5}$, $Ar^{DA6}$ and $Ar^{DA7}$ each independently represent an arylene group or a divalent heterocyclic group, and these groups each may have a substituent. When there are a plurality of $Ar^{DA1}$, $Ar^{DA2}$, $Ar^{DA3}$, $Ar^{DA4}$, $Ar^{DA5}$, $Ar^{DA6}$, and $Ar^{DA7}$, they may each be the same or different at each occurrence.
$T^{DA}$ represents an aryl group or a monovalent heterocyclic group, and these groups each may have a substituent. The plurality of $T^{DA}$ may be the same or different.]

[Chemical Formula 23]

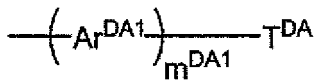
(D-C)

[In the formula,

$m^{DA1}$ represents an integer of 0 or more.
$Ar^{DA1}$ represents an arylene group or a divalent heterocyclic group, and these groups each may have a substituent. When there are a plurality of $Ar^{DA1}$, they may each be the same or different.
$T^{DA}$ represents an aryl group or a monovalent heterocyclic group, and these groups each may have a substituent.]

$m^{DA1}$, $m^{DA2}$, $m^{DA3}$, $m^{DA4}$, $m^{DA5}$, $m^{DA6}$, and $m^{DA7}$ are generally an integer of 10 or less, preferably an integer of 5 or less, more preferably an integer of 2 or less, and still more preferably 0 or 1. It is preferable that $m^{DA2}$, $m^{DA3}$, $m^{DA4}$, $m^{DA5}$, $m^{DA6}$, and $m^{DA7}$ are the same integer, and it is more preferable that $m^{DA1}$, $m^{DA2}$, $m^{DA3}$, $m^{DA4}$, $m^{DA5}$, $m^{DA6}$, and $m^{DA7}$ are the same integer.

$G^{DA}$ is preferably a group represented by formula (GDA-11) to (GDA-15), more preferably a group represented by formula (GDA-11) to (GDA-14), still more preferably a group represented by formula (GDA-11) or (GDA-14), and particularly preferably a group represented by formula (GDA-11).

[Chemical Formula 24]

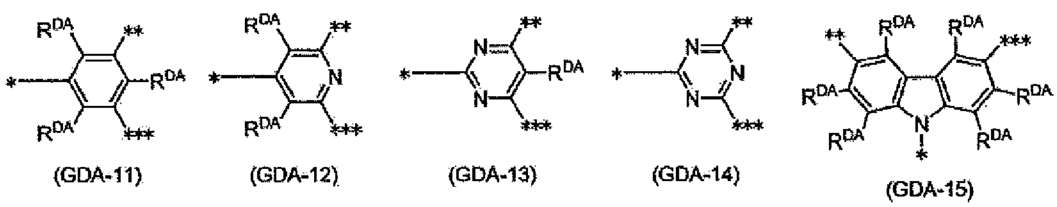
(GDA-11) (GDA-12) (GDA-13) (GDA-14) (GDA-15)

[In the formula,

*represents a bond with $Ar^{DA1}$ in formula (D-A), $Ar^{DA1}$ in formula (D-B), $Ar^{DA2}$ in formula (D-B), or $Ar^{DA3}$ in formula (D-B).
** represents a bond with $Ar^{DA2}$ in formula (D-A), $Ar^{DA2}$ in formula (D-B), $Ar^{DA4}$ in formula (D-B), or $Ar^{DA6}$ in formula (D-B).
*** represents a bond with $Ar^{DA3}$ in formula (D-A), $Ar^{DA3}$ in formula (D-B), $Ar^{DA5}$ in formula (D-B), or $Ar^{DA7}$ in formula (D-B).
$R^{DA}$ represents a hydrogen atom, an alkyl group, a cycloalkyl group, an alkoxy group, a cycloalkoxy group, an aryl group, or a monovalent heterocyclic group, and these groups each may further have a substituent. When there are a plurality of $R^{DA}$, they may be the same or different.]

$R^{DA}$ is preferably a hydrogen atom, an alkyl group, a cycloalkyl group, an alkoxy group, or a cycloalkoxy group, more preferably a hydrogen atom, an alkyl group, or a cycloalkyl group, and these groups each may have a substituent.

$Ar^{DA1}$, $Ar^{DA2}$, $Ar^{DA3}$, $Ar^{DA4}$, $Ar^{DA5}$, $Ar^{DA6}$, and $Ar^{DA7}$ are preferably a phenylene group, a fluorenediyl group, or a carbazolediyl group, more preferably a group represented by formula (A-1) to (A-3), (A-8), (A-9), (AA-10), (AA-11), (AA-33), or (AA-34), and still more preferably a group represented by formula (ArDA-1) to (ArDA-5), particularly preferably a group represented by formulas (ArDA-1) to (ArDA-3), and especially preferably a group represented by formula (ArDA-1).

[Chemical Formula 25]

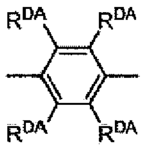
(ArDA-1)
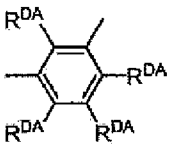
(ArDA-2)
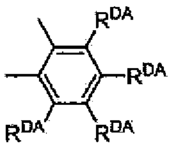
(ArDA-3)
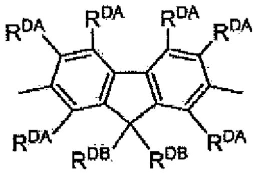
(ArDA-4)
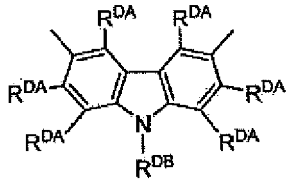
(ArDA-5)

[In the formula,

$R^{DA}$ represents the same meaning as described above.

$R^{DB}$ represents a hydrogen atom, an alkyl group, a cycloalkyl group, an aryl group, or a monovalent heterocyclic group, and these groups each may have a substituent. When there are a plurality of $R^{DB}$, they may be the same or different.]

$R^{DB}$ is preferably an alkyl group, a cycloalkyl group, an aryl group, or a monovalent heterocyclic group, more preferably an aryl group or a monovalent heterocyclic group, and still more preferably an aryl group, and these groups each may have a substituent.

$T^{DA}$ is preferably a group represented by formula (TDA-1) to (TDA-3), more preferably a group represented by formula (TDA-1).

[Chemical Formula 26]

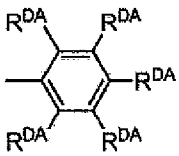
(TDA-1)
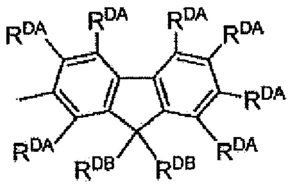
(TDA-2)
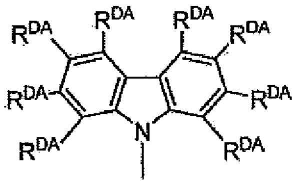
(TDA-3)

[In the formula, $R^{DA}$ and $R^{DB}$ represent the same meanings as described above.]

The group represented by formula (D-A) is preferably a group represented by formula (D-A1) to (D-A4), more preferably a group represented by formula (D-A1) or (D-A4).

[Chemical Formula 27]

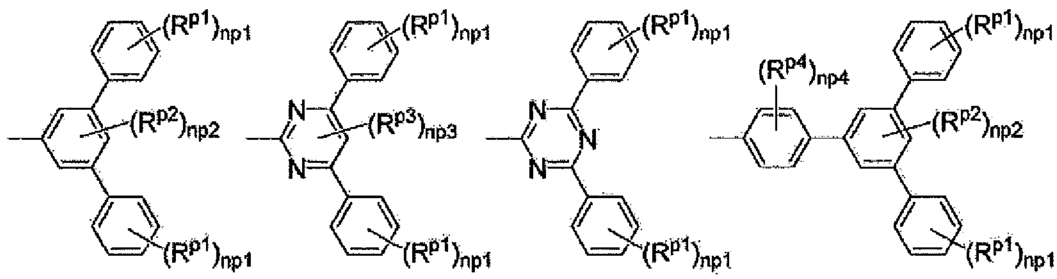

(D-A1) (D-A2) (D-A3) (D-A4)

[In the formula,

$R^{p1}$, $R^{p2}$, $R^{p3}$, and $R^{p4}$ each independently represent an alkyl group, a cycloalkyl group, an alkoxy group, a cycloalkoxy group, or a fluorine atom. When there are a plurality of $R^{p1}$, $R^{p2}$, and $R^{p4}$, they may each be the same or different at each occurrence.

np1 represents an integer of 0 to 5, np2 represents an integer of 0 to 3, np3 represents 0 or 1, and np4 represents an integer of 0 to 4. The plurality of np1 may be the same or different.]

The group represented by formula (D-B) is preferably a group represented by formula (D-B1) to (D-B3), and more preferably a group represented by formula (D-B1).

[Chemical Formula 28]

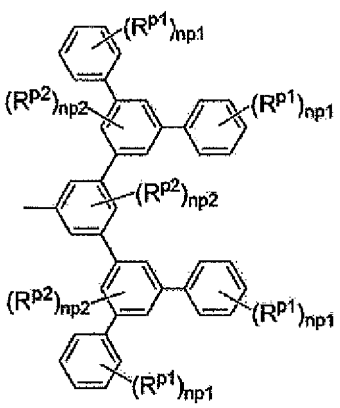

(D-B1)

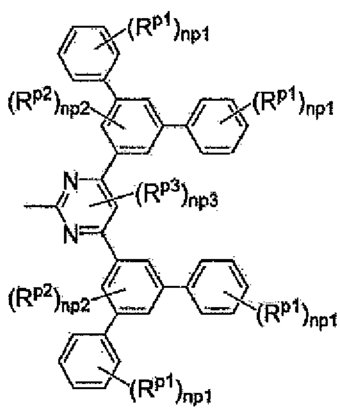

(D-B2)

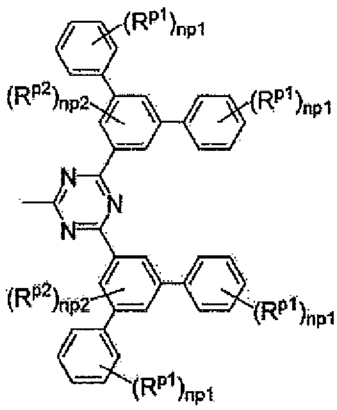

(D-B3)

[In the formula,

$R^{p1}$, $R^{p2}$, and $R^{p3}$ each independently represent an alkyl group, a cycloalkyl group, an alkoxy group, a cycloalkoxy group, or a fluorine atom. When there are a plurality of $R^{p1}$ and $R^{p2}$, they may each be the same or different at each occurrence.

np1 represents an integer of 0 to 5, np2 represents an integer of 0 to 3, np3 represents 0 or 1. When there are a plurality of np1 and np2, they may each be the same or different at each occurrence.]

The group represented by formula (D-C) is preferably a group represented by formula (D-C1) to (D-C4), more preferably a group represented by formula (D-C1) to (D-C3), more preferably a group represented by formula (D-C1) or (D-C2), and particularly preferably a group represented by formula (D-C2).

[Chemical Formula 29]

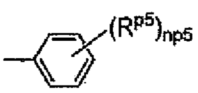
(D-C1)
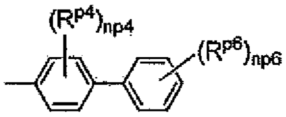
(D-C2)
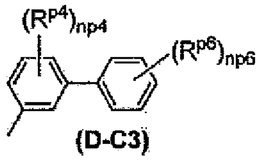
(D-C3)
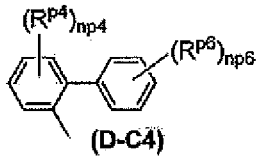
(D-C4)

[In the formula,

$R^{p4}$, $R^{p5}$, and $R^{p6}$ each independently represent an alkyl group, a cycloalkyl group, an alkoxy group, a cycloalkoxy group, or a fluorine atom. When there are a plurality of $R^{p4}$, $R^{p5}$, and $R^{p6}$, they may each be the same or different at each occurrence.

np4 represents an integer of 0 to 4, np5 represents an integer of 0 to 5, and np6 represents an integer of 0 to 5.]

np1 is preferably 0 or 1, more preferably 1. np2 is preferably 0 or 1, and more preferably 0. np3 is preferably 0. np4 is preferably an integer of 0 to 2. np 5 is preferably an integer of 1 to 3. np6 is preferably an integer of 0 to 2.

$R^{p1}$, $R^{p2}$, $R^{p3}$, $R^{p4}$, $R^{p5}$, and $R^{p6}$ are preferably an alkyl group or a cycloalkyl group, more preferably a methyl group, an ethyl group, an isopropyl group, a tert-butyl group, a hexyl group, a 2-ethylhexyl group, a cyclohexyl, a methoxy group, a 2-ethylhexyloxy group, a tert-octyl group or a cyclohexyloxy group, and still more preferably a methyl group, an ethyl group, an isopropyl group, a tert-butyl group, a hexyl group, a 2-ethylhexyl group, or a tert-octyl group.

Examples of the group represented by formula (D-A) include groups represented by formulas (D-A-1) to (D-A-12).

[Chemical Formula 30]

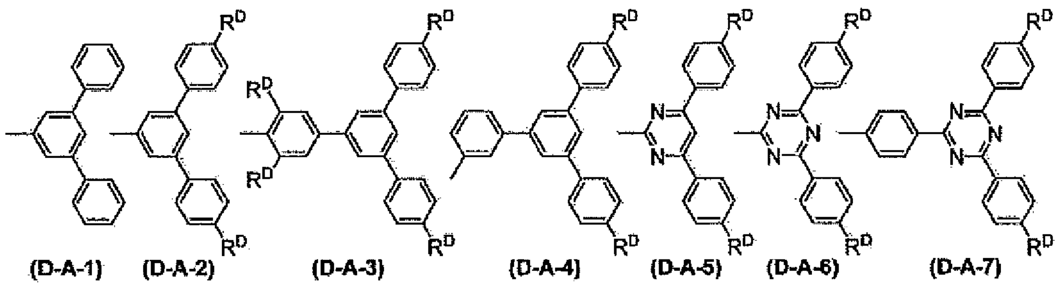
(D-A-1) (D-A-2) (D-A-3) (D-A-4) (D-A-5) (D-A-6) (D-A-7)

[Chemical Formula 31]

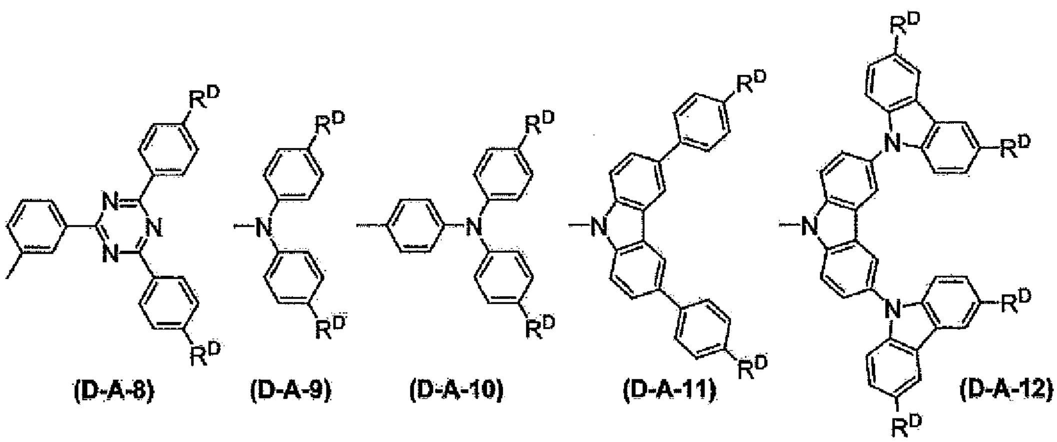
(D-A-8) (D-A-9) (D-A-10) (D-A-11) (D-A-12)

[In the formula, $R^D$ represents a methyl group, an ethyl group, an isopropyl group, a tert-butyl group, a hexyl group, a 2-ethylhexyl group, a tert-octyl group, a cyclohexyl group, a methoxy group, a 2-ethylhexyloxy group, or a cyclohexyloxy group. When there are a plurality of $R^D$, they may be the same or different.]

Examples of the group represented by formula (D-B) include groups represented by formulas (D-B-1) to (D-B-4).

[Chemical Formula 32]

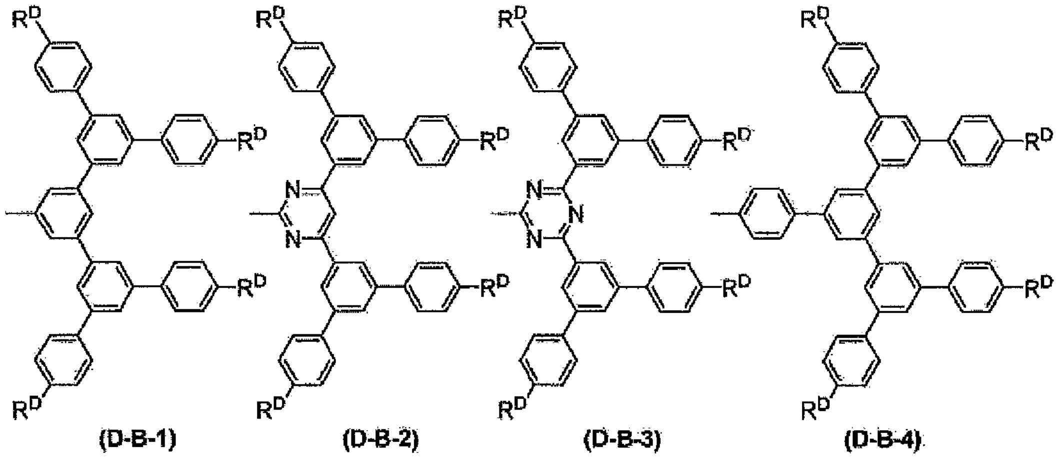

(D-B-1) (D-B-2) (D-B-3) (D-B-4)

[In the formula, $R^D$ represents the same meaning as described above.]

Examples of the group represented by formula (D-C) include groups represented by formulas (D-C-1) to (D-C-17).

[Chemical Formula 33]

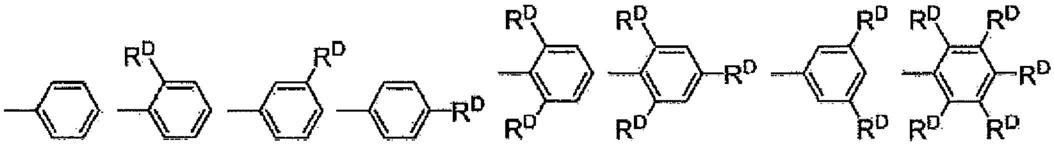

(D-C-1) (D-C-2) (D-C-3) (D-C-4) (D-C-5) (D-C-6) (D-C-7) (D-C-8)

[Chemical Formula 34]

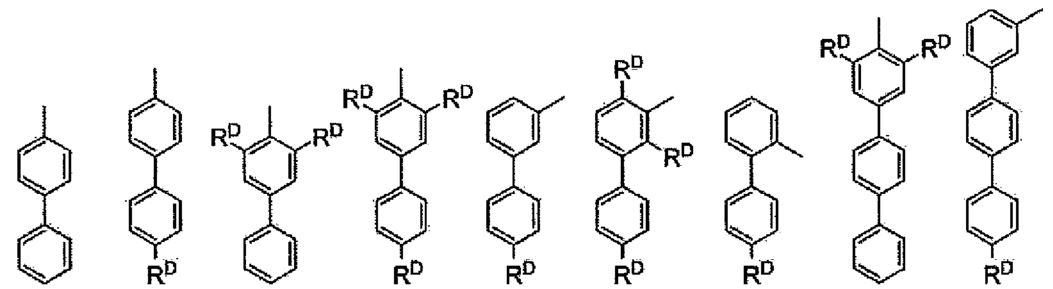

(D-C-9) (D-C-10) (D-C-11) (D-C-12) (D-C-13) (D-C-14) (D-C-15) (D-C-16) (D-C-17)

[In the formula, $R^D$ represents the same meaning as described above.]

$R^D$ is preferably a methyl group, an ethyl group, an isopropyl group, a tert-butyl group, a hexyl group, a 2-ethylhexyl group, or a tert-octyl group.

Because the initial degradation of the light-emitting device according to this embodiment is further suppressed, it is preferable that at least one ring selected from the group consisting of the $R^1$ ring and the $R^2$ ring has a substituent, and it is more preferable that the $R^1$ ring has a substituent.

The substituent that the at least one ring selected from the group consisting of the $R^1$ ring and the $R^2$ ring has is preferably an aryl group that may have a substituent or a monovalent heterocyclic group that may have a substituent,

more preferably an aryl group that may have a substituent, further preferably a group represented by the formulas (D-A1), (D-A4), (D-B1), or (D-C1) to (D-C4), particularly preferably a group represented by the formulas (D-C1) to (D-C3), and more particularly preferably a group represented by the formula (D-C2).

[Anionic Bidentate Ligand]

Examples of the anionic bidentate ligand represented by $A^1$-$G^1$-$A^2$ include ligands represented by the following formulas.

[Chemical Formula 35]

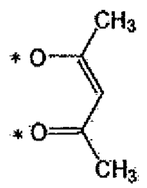
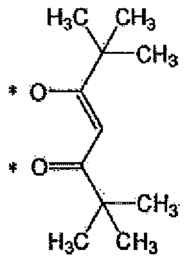
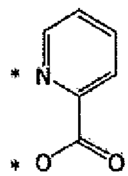
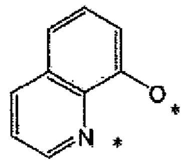
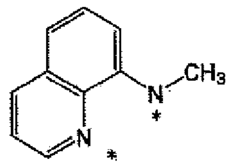

[Chemical Formula 36]

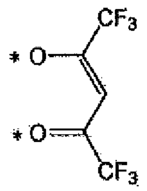
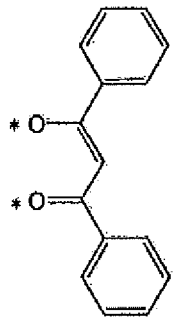
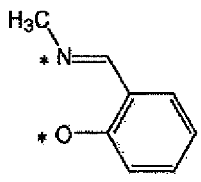
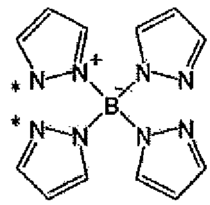

[In the formula, * represents a site that binds to $M^1$.]

The anionic bidentate ligand represented by $A^1$-$G^1$-$A^2$ may be a ligand represented by the following formulas.

[Chemical Formula 37]

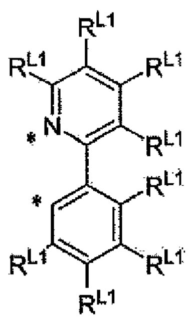
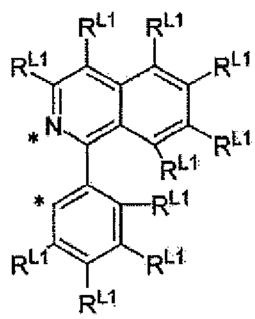
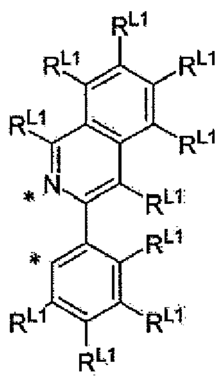
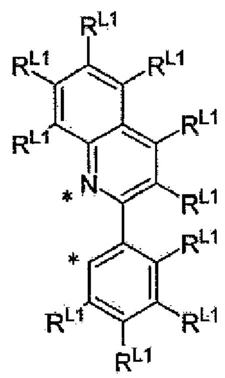

[In the formula,

*represents a site that binds to $M^1$.

$R^{L1}$ represents a hydrogen atom, an alkyl group, a cycloalkyl group, an aryl group, a monovalent heterocyclic group,

or a fluorine atom, and these groups each may have a substituent. The plurality of $R^{L1}$ may be the same or different.]

The phosphorescent compound represented by formula (1) is preferably a compound represented by the formula (1-A), because the initial degradation of the light-emitting device according to the present embodiment is more suppressed.

[Compound Represented by Formula (1-A)]

When the $R^{1A}$ ring is a diazole ring, it is preferably an imidazole ring in which $E^{11A}$ is a nitrogen atom or an imidazole ring in which $E^{12A}$ is a nitrogen atom, and more preferably an imidazole ring in which $E^{11A}$ is a nitrogen atom.

When the $R^{1A}$ ring is a triazole ring, it is preferably a triazole ring in which $E^{11A}$ and $E^{12A}$ are nitrogen atoms, or a triazole ring in which $E^{11A}$ and $E^{13A}$ are nitrogen atoms, and more preferably a triazole ring in which $E^{11A}$ and $E^{12A}$ are nitrogen atoms.

The $R^{1A}$ ring is preferably a diazole ring.

Examples and preferable ranges of the aryl group, monovalent heterocyclic group, and substituted amino group in $R^{11A}$, $R^{12A}$, $R^{13A}$, $R^{21A}$, $R^{22A}$, $R^{23A}$, and $R^{24A}$ are respectively the same as the examples and preferable ranges of the aryl group, monovalent heterocyclic group, and substituted amino group in a substituent that the $R^1$ ring and the $R^2$ ring may have.

Examples and preferable ranges of the substituent that $R^{11A}$, $R^{12A}$ $R^{13A}$, $R^{21A}$, $R^{22A}$, $R^{23A}$, and $R^{24A}$ may have are the same as the examples and preferable ranges of the substituent that the substituent that the $R^1$ ring and the $R^2$ ring may have may further have.

When $E^{11A}$ is a nitrogen atom and $R^{11A}$ is present, $R^{11A}$ is preferably an alkyl group, a cycloalkyl group, an aryl group, or a monovalent heterocyclic group, more preferably an aryl group or a monovalent heterocyclic group, further preferably an aryl group, particularly preferably a group represented by the formulas (D-A1), (D-A4), (D-B1), or (D-C1) to (D-C4), more particularly preferably a group represented by the formulas (D-C1) to (D-C3), and further more preferably a group represented by the formula (D-C2), and these groups each may have a substituent.

When $E^{11A}$ is a carbon atom, $R^{11A}$ is preferably a hydrogen atom, an alkyl group, a cycloalkyl group, an aryl group, a monovalent heterocyclic group, or a substituted amino group, more preferably a hydrogen atom, an alkyl group, a cycloalkyl group, or an aryl group, further preferably a hydrogen atom, an alkyl group, or a cycloalkyl group, and particularly preferably a hydrogen atom, and these groups each may have a substituent.

When $E^{12A}$ is a nitrogen atom and $R^{12A}$ is present, $R^{12A}$ is preferably an alkyl group, a cycloalkyl group, an aryl group, or a monovalent heterocyclic group, more preferably an aryl group or a monovalent heterocyclic group, further preferably an aryl group, particularly preferably a group represented by the formulas (D-A1), (D-A4), (D-B1), or (D-C1) to (D-C4), more particularly preferably a group represented by the formulas (D-C1) to (D-C3), further more preferably a group represented by the formula (D-C2), and these groups each may have a substituent.

When $E^{12A}$ is a carbon atom, $R^{12A}$ is preferably a hydrogen atom, an alkyl group, a cycloalkyl group, an aryl group, a monovalent heterocyclic group, or a substituted amino group, more preferably a hydrogen atom, an alkyl group, a cycloalkyl group, or an aryl group, further preferably a hydrogen atom, an alkyl group, or a cycloalkyl group, and particularly preferably a hydrogen atom, and these groups each may have a substituent.

When $E^{13A}$ is a nitrogen atom and $R^{13A}$ is present, $R^{13A}$ is preferably an alkyl group, a cycloalkyl group, an aryl group, or a monovalent heterocyclic group, more preferably an aryl group or a monovalent heterocyclic group, further preferably an aryl group, particularly preferably a group represented by the formulas (D-A1), (D-A4), (D-B1), or (D-C1) to (D-C4), more particularly preferably a group represented by the formulas (D-C1) to (D-C3), further more preferably a group represented by the formula (D-C2), and these groups each may have a substituent.

When $E^{13A}$ is a carbon atom, $R^{13A}$ is preferably a hydrogen atom, an alkyl group, a cycloalkyl group, an aryl group, a monovalent heterocyclic group, or a substituted amino group, more preferably a hydrogen atom, an alkyl group, a cycloalkyl group, or an aryl group, further preferably a hydrogen atom, an alkyl group, or a cycloalkyl group, particularly preferably a hydrogen atom, and these groups each may have a substituent.

When the $R^{2A}$ ring is a pyridine ring, a pyridine ring in which $E^{21A}$ is a nitrogen atom, a pyridine ring in which $E^{22A}$ is a nitrogen atom, or a pyridine ring in which $E^{23A}$ is a nitrogen atom is preferable, and a pyridine ring in which $E^{22A}$ is a nitrogen atom is more preferable.

When the $R^{2A}$ ring is a pyrimidine ring, a pyrimidine ring in which $E^{21A}$ and $E^{23A}$ are nitrogen atoms, or a pyrimidine ring in which $E^{22A}$ and $E^{24A}$ are nitrogen atoms is preferable, and a pyrimidine ring in which $E^{22A}$ and $E^{24A}$ are nitrogen atoms is more preferable.

The $R^{2A}$ ring is preferably a benzene ring.

$R^{21A}$, $R^{22A}$, $R^{23A}$, and $R^{24A}$ are preferably a hydrogen atom, an alkyl group, a cycloalkyl group, an alkoxy group,

a cycloalkoxy group, a fluorine atom, an aryl group, a monovalent heterocyclic group, or a substituted amino group, more preferably a hydrogen atom, an alkyl group, a cycloalkyl group, an aryl group, a monovalent heterocyclic group, or a substituted amino group, further preferably a hydrogen atom, an alkyl group, a cycloalkyl group, or an aryl group, particularly preferably a hydrogen atom, an alkyl group, or a cycloalkyl group, more particularly preferably a hydrogen atom, and these groups each may have a substituent.

Because the initial degradation of the light-emitting device according to the present embodiment is more suppressed, at least one selected from the group consisting of $R^{11A}$, $R^{12A}$, $R^{13A}$, $R^{21A}$, $R^{22A}$, $R^{23A}$, and $R^{24A}$ is preferably an aryl group that may have a substituent or a monovalent heterocyclic group that may have a substituent, more preferably an aryl group that may have a substituent, further preferably a group represented by the formulas (D-A1), (D-A4), (D-B1), or (D-C1) to (D-C4), particularly preferably a group represented by the formulas (D-C1) to (D-C3), and more particularly preferably a group represented by the formula (D-C2).

When at least one selected from the group consisting of $R^{11A}$, $R^{12A}$, $R^{13A}$, $R^{21A}$, $R^{22A}$, $R^{23A}$, and $R^{24A}$ is an aryl group that may have a substituent or a monovalent heterocyclic group that may have a substituent, preferably at least one selected from the group consisting of $R^{11A}$, $R^{12A}$, and $R^{13A}$ is an aryl group that may have a substituent or a monovalent heterocyclic group that may have a substituent, more preferably $R^{11A}$ or $R^{12A}$ is an aryl group that may have a substituent or a monovalent heterocyclic group that may have a substituent, and further preferably $R^{11A}$ is an aryl group that may have a substituent or a monovalent heterocyclic group that may have a substituent.

$R^{11A}$ and $R^{12A}$, $R^{12A}$ and $R^{13A}$, $R^{11A}$ and $R^{21A}$, $R^{21A}$ and $R^{22A}$, $R^{22A}$ and $R^{23A}$, and $R^{23A}$ and $R^{24A}$ each may be bonded to each other to form a ring together with the atoms to which they are bonded, but $R^{11A}$ and $R^{12A}$ and $R^{12A}$ and $R^{13A}$ preferably form no ring because the maximum peak wavelength of emission spectrum of the phosphorescent compound represented by formula (1) is a short wavelength. Moreover, because the synthesis of the phosphorescent compound represented by formula (1) becomes easy, preferably $R^{11A}$ and $R^{12A}$, $R^{12A}$ and $R^{13A}$, $R^{21A}$ and $R^{22A}$, $R^{22A}$ and $R^{23A}$, and $R^{23A}$ and $R^{24A}$ form no ring, and more preferably $R^{11A}$ and $R^{12A}$, $R^{12A}$ and $R^{13A}$, $R^{11A}$ and $R^{21A}$, $R^{21A}$ and $R^{22A}$, $R^{22A}$ and $R^{23A}$, and $R^{23A}$ and $R^{24A}$ form no ring.

Because the initial degradation of the light-emitting device according to the present embodiment is further suppressed, the compound represented by formula (1-A) is preferably a compound represented by formula (1-A1), a compound represented by formula (1-A2), a compound represented by formula (1-A3), or a compound represented by formula (1-A4), more preferably a compound represented by formula (1-A1) or a compound represented by formula (1-A3), and still more preferably a compound represented by formula (1-A3).

Examples of the compound represented by the formula (1-A1) include compounds represented by formulas (1-A1-1) to (1-A 1-11) shown in Table 1. The compound represented by the formula (1-A1) is preferably the compound represented by the formulas (1-A1-1) to (1-A1-9), and more preferably the compound represented by the formulas (1-A1-1) to (1-A1-7).

[Table 1]

| Formula | $M^1$ | $n^1$ | $R^{11A}$ | $R^{13A}$ | $R^{21A}$ | $R^{22A}$ | $R^{23A}$ | $R^{24A}$ | $n^2$ | $A^1$-$G^1$-$A^2$ |
|---|---|---|---|---|---|---|---|---|---|---|
| (1-A1-1) | Ir | 3 | Formula (D-C-11) | H | H | H | H | H | 0 | - |
| (1-A1-2) | Ir | 3 | Formula (D-C-5) | Me | H | Formula (D-A-1) | H | H | 0 | - |
| (1-A1-3) | Ir | 3 | Formula (D-A-3) | Me | H | H | H | H | 0 | - |
| (1-A1-4) | Ir | 3 | Formula (D-A-3) | Me | H | Formula (D-A-1) | H | H | 0 | - |
| (1-A1-5) | Ir | 3 | Formula (D-C-6) | Me | H | Formula (D-B-1) | H | H | 0 | - |
| (1-A1-6) | Ir | 3 | Formula (D-C-12) | Me | H | Formula (D-C-4) | H | H | 0 | - |
| (1-A1-7) | Ir | 3 | Formula (D-C-12) | H | H | H | Me | H | 0 | - |
| (1-A1-8) | Ir | 3 | Me | $C_3H_7$ | H | H | H | H | 0 | - |
| (1-A1-9) | Ir | 3 | Me | Me | H | Formula (D-A-4) | H | H | 0 | - |
| (1-A1-10) | Ir | 2 | Formula (D-C-11) | H | H | H | H | H | 1 | 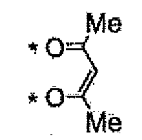 |
| (1-A1-11) | Pt | 2 | Formula (D-C-11) | H | H | H | H | H | 0 | - |

Examples of the compound represented by formula (1-A2) include compounds represented by formulas (1-A2-1) to (1-A2-8) shown in Table 2. The compound represented by the formula (1-A2) is preferably the compound represented by the formulas (1-A2-1) to (1-A2-6), and more preferably the compound represented by the formulas (1-A2-1) to (1-A2-4).

[Table 2]

| Formula | $M^1$ | $n^1$ | $R^{12A}$ | $R^{13A}$ | $R^{21A}$ | $R^{11A}$ | $R^{23A}$ | $R^{24A}$ | $n^2$ | $A^1$-$G^1$-$A^2$ |
|---|---|---|---|---|---|---|---|---|---|---|
| (1-A2-1) | Ir | 3 | Formula (D-C-11) | H | H | H | H | H | 0 | - |
| (1-A2-2) | Ir | 3 | Formula (D-C-6) | Me | H | Formula (D-A-1) | H | H | 0 | - |
| (1-A2-3) | Ir | 3 | Formula (D-C-12) | Me | H | Formula (D-C-4) | H | H | 0 | - |
| (1-A2-4) | Ir | 3 | Formula (D-C-5) | $C_3H_7$ | H | H | Formula (D-C-4) | H | 0 | - |
| (1-A2-5) | Ir | 3 | Me | $C_3H_7$ | H | H | H | H | 0 | - |
| (1-A2-6) | Ir | 3 | Me | $C_3H_7$ | H | Formula (D-A-1) | H | H | 0 | - |
| (1-A2-7) | Ir | 2 | Formula (D-C-11) | H | H | H | H | H | 1 | 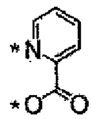 |
| (1-A2-8) | Pt | 2 | Formula (D-C-11) | H | H | H | H | H | 0 | - |

Examples of the compound represented by formula (1-A3) include compounds represented by formulas (1-A3-1) to (1-A3-11) shown in Table 3. The compound represented by the formula (1-A3) is preferably the compound represented by the formulas (1-A3-1) to (1-A3-9), and more preferably the compound represented by the formulas (1-A3-1) to (1-A3-8).

[Table 3]

| Formula | $M^1$ | $n^1$ | $R^{11A}$ | $R^{12A}$ | $R^{13A}$ | $R^{21A}$ | $R^{22A}$ | $R^{23A}$ | $R^{24A}$ | $n^2$ | $A^1$-$G^1$-$A^2$ |
|---|---|---|---|---|---|---|---|---|---|---|---|
| (1-A3-1) | Ir | 3 | Formula (D-C-6) | H | H | H | H | H | H | 0 | - |
| (1-A3-2) | Ir | 3 | Formula (D-C-11) | H | H | H | H | H | H | 0 | - |
| (1-A3-3) | Ir | 3 | Formula (D-A-3) | H | H | H | H | H | H | 0 | - |
| (1-A3-4) | Ir | 3 | Formula (D-C-5) | Me | $C_3H_7$ | H | H | H | H | 0 | - |
| (1-A3-5) | Ir | 3 | Formula (D-C-6) | H | Formula (D-C-3) | H | H | H | H | 0 | - |
| (1-A3-6) | Ir | 3 | Formula (D-C-5) | H | H | H | Formula (D-A-1) | H | H | 0 | - |
| (1-A3-7) | Ir | 3 | Formula (D-C-6) | H | H | H | Formula (D-A-4) | H | H | 0 | - |
| (1-A3-8) | Ir | 3 | Formula (D-C-14) | H | H | H | H | Formula (D-C-4) | H | 0 | - |
| (1-A3-9) | Ir | 3 | Formula (D-C-11) | H | H | F | H | $CF_3$ | H | 0 | - |
| (1-A3-10) | Ir | 2 | Formula (D-C-11) | H | H | H | H | H | H | 1 | 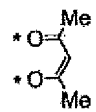 |
| (1-A3-11) | Pt | 2 | Formula (D-C-11) | H | H | H | H | H | H | 0 | - |

Examples of the compound represented by formula (1-A4) include compounds represented by formulas (1-A4-1) to (1-A4-8) shown in Table 4. The compound represented by the formula (1-A4) is preferably the compound represented by the formulas (1-A4-1) to (1-A4-6), and more preferably the compound represented by the formulas (1-A4-1) to (1-A4-5).

[Table 4]

| Formula | $M^1$ | $n^1$ | $R^{11A}$ | $R^{12A}$ | $R^{13A}$ | $R^{21A}$ | $R^{22A}$ | $R^{23A}$ | $R^{24A}$ | $n^2$ | $A^1$-$G^1$-$A^2$ |
|---|---|---|---|---|---|---|---|---|---|---|---|
| (1-A4-1) | Ir | 3 | H | Formula (D-C-11) | H | H | H | H | H | 0 | - |
| (1-A4-2) | Ir | 3 | H | Formula (D-C-5) | H | H | Formula (D-A-1) | H | H | 0 | - |
| (1-A4-3) | Ir | 3 | H | Formula (D-A-3) | H | H | H | H | H | 0 | - |
| (1-A4-4) | Ir | 3 | H | Formula (D-C-6) | H | H | H | Formula (D-C-1) | H | 0 | - |
| (1-A4-5) | Ir | 3 | Me | Formula (D-C-5) | Me | H | Me | H | H | 0 | - |
| (1-A4-6) | Ir | 3 | H | Formula (D-C-6) | H | F | H | $CF_3$ | H | 0 | - |
| (1-A4-7) | Ir | 2 | H | Formula (D-C-11) | H | H | H | H | H | 1 | 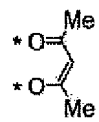 |
| (1-A4-8) | Pt | 2 | H | Formula (D-C-11) | H | H | H | H | H | 0 | - |

Examples of the compound represented by formula (1) include compounds represented by formulas (1-A1-1) to (1-A1-11), formulas (1-A2-1) to (1-A2-8), formulas (1-A3-1) to (1-A3-11), formulas (1-A4-1) to (1-A4-8), and formulas (1-A-1) to (1-A-5) below. The compound represented by the formula (1) is preferably the compound represented by formulas (1-A1-1) to (1-A1-9), formulas (1-A2-1) to (1-A2-6), formulas (1-A3-1) to (1-A3-9), formulas (1-A4-1) to (1-A4-6), or formulas (1-A-1) to (1-A-5), more preferably the compound represented by formulas (1-A1-1) to (1-A.1-7), formulas (1-A2-1) to (1-A2-4), formulas (1-A3-1) to (1-A3-8), formulas (1-A4-1) to (1-A4-5), or formulas (1-A-3) to (1-A-5), further preferably the compound represented by formulas (1-A1-1) to (1-A1-7) or formulas (1-A3-1) to (1-A3-8), and particularly preferably the compound represented by formulas (1-A3-1) to (1-A3-8).

[Chemical Formula 38]

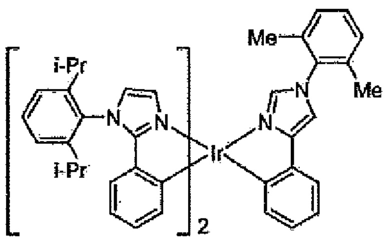

(1-A-1)

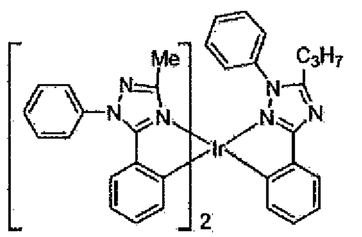

(1-A-2)

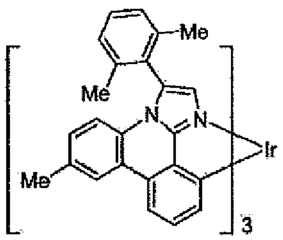

(1-A-3)

[Chemical Formula 39]

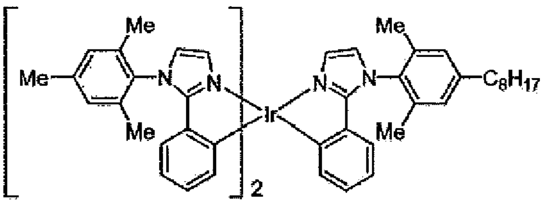

(1-A-4)

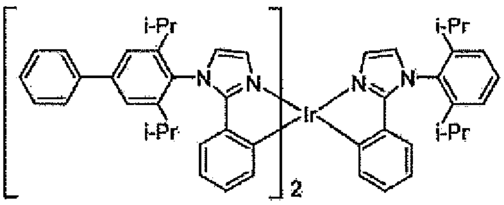

(1-A-5)

[Method for Obtaining Phosphorescent Compound Represented by Formula (1)]

The phosphorescent compound represented by formula (1) can be obtained from Aldrich, Luminescence Technology Corp., American Dye Source, or the like.

Moreover, methods for obtaining the compound other than that described above include those involving preparation by known methods described in literature such as International Publication No. WO 2006/121811, International Publication No. WO 2007/097153, Japanese Unexamined Patent Publication No. 2013-048190, International Publication No. WO 2004/101707, Japanese Unexamined Patent Publication No. 2013-147449, Japanese Unexamined Patent Publication No. 2013-147450, Japanese Unexamined Patent Publication No. 2013-147551.

[Amount ($C^1$) of Chlorine Atoms Contained in Phosphorescent Compound Represented by Formula (1)]

In the composition according to the present embodiment, the amount ($C^1$) of chlorine atoms contained as impurities in the phosphorescent compound represented by formula (1) is usually 15 ppm by mass or less with respect to the total amount of the phosphorescent compound. In the phosphorescent compound according to the present embodiment, the amount of chlorine atoms contained as impurities is preferably 13 ppm by mass or less, more preferably 9 ppm by mass or less, further preferably 5 ppm by mass or less, particularly preferably 1 ppm by mass or less, more particularly preferably 0.9 ppm by mass or less, and especially preferably 0 ppm by mass, because the initial degradation of the light-emitting device according to the present embodiment is suppressed.

Moreover, in the phosphorescent compound according to the present embodiment, the amount of chlorine atoms contained as impurities is preferably 0.01 ppm by mass or more and 12 ppm by mass or less, more preferably 0.05 ppm by mass or more and 11 ppm by mass or less, further preferably 0.1 ppm by mass or more and 10 ppm by mass or less, particularly preferably 0.5 ppm by mass or more and 9 ppm by mass or less, more particularly preferably 0.9 ppm by mass or more and 9 ppm by mass or less, because the light-emitting device according to the present embodiment has a good luminous efficiency.

Moreover, in the phosphorescent compound according to the present embodiment, the amount of chlorine atoms contained as impurities is preferably 0.01 ppm by mass or more and 5 ppm by mass or less, more preferably 0.05 ppm by mass or more and 1 ppm by mass or less, further preferably 0.1 ppm by mass or more and 0.9 ppm by mass or less, because the initial degradation of the light-emitting device according to the present embodiment is suppressed and the light-emitting device according to the present embodiment has a good luminous efficiency.

As used herein, the term "amount of chlorine atoms" can be measured by automatic combustion-ion chromatography. Accordingly, the term "amount of chlorine atoms" means the mass concentration of chlorine as measured by automatic combustion-ion chromatography. Moreover, the "amount of chlorine atoms" being "0 ppm by mass" means that the mass concentration of chlorine is below the detection limit when measured by automatic combustion-ion chromatography.

A specific method for calculating $C^1$ will be described with reference to Example D1 and Example D2 described below.

First, in Example D1, $C^1$ is 0 ppm by mass because the amount of chlorine atoms of the phosphorescent compound MC3 as measured by automatic combustion-ion chromatography is below the detection limit.

Next, in Example D2, the amounts of chlorine atoms in the phosphorescent compound MC3 and the phosphorescent compound MC2 as measured by automatic combustion-ion chromatography are below the detection limit (i.e., 0 ppm by mass) and 9 ppm by mass. Moreover, the mass ratio of the phosphorescent compound MC3 and the phosphorescent compound MC2 is phosphorescent compound MC3:phosphorescent compound MC2 = 22.5:2.5.

Accordingly, $C^1$ can be determined from the amount of chlorine atoms contained in each phosphorescent compound and its charged amount and demanded as follows.

$$C^1 = \{0 \times 22.5/(22.5 + 2.5)\} + \{9 \times 2.5/(22.5 + 2.5)\} = 0.9 \text{ ppm by mass}$$

Similarly to the specific method for calculating $C^1$ in Example D2 as described above, $C^1$ in Example D3 is determined as follows.

$$C^1 = \{0 \times 12.5/(12.5 + 12.5)\} + \{9 \times 12.5/(12.5 + 12.5)\} = 4.5 \text{ ppm by mass}$$

Similarly to the specific method for calculating $C^1$ in Example D1 as described above, $C^1$ in Example D4 is 9 ppm by mass.

Similarly to the specific method for calculating $C^1$ in Example D2 as described above, $C^1$ in Example D5 is determined as follows.

$$C^1 = \{9 \times 12.5/(12.5 + 12.5)\} + \{16 \times 12.5/(12.5 + 12.5)\} = 12.5 \text{ ppm by mass}$$

Similarly to the specific method for calculating $C^1$ in Example D1 as described above, $C^1$ in Comparative Example CD1 is 16 ppm by mass.

[Method for Reducing $C^1$]

Examples of the method for reducing $C^1$ include at least one method selected from among purification and processing by a dehalogenating agent.

[Purification]

Examples of purification include known methods for purification described in The fourth series of experimental chemistry (in Japanese) (1993, MaruzenJunkudo Bookstores Co., Ltd), The fifth series of experimental chemistry (in Japanese) (2007, MaruzenJunkudo Bookstores Co., Ltd), New series of experimental chemistry (in Japanese) (1975, MaruzenJunkudo Bookstores Co., Ltd), Manual for organic chemistry experiments (in Japanese) (1988, Kagaku-Dojin Publishing Co., Inc.).

Examples of purification include sublimation, extraction, reprecipitation, recrystallization, chromatography, or adsorption.

When the purification involves twice or more times of purification, methods thereof may be the same or different.

For sublimation, the degree of vacuum and the temperature for sublimation may be set as appropriate according to the material to be sublimated. The degree of vacuum is preferably $1 \times 10^{-10}$ to $1 \times 10^{5}$ Pa, more preferably $1 \times 10^{-7}$ to $1 \times 10^{2}$ Pa, further preferably $1 \times 10^{-5}$ to 1 Pa, and particularly preferably $1 \times 10^{-4}$ to $1 \times 10^{-2}$ Pa. Moreover, the temperature for sublimation is preferably -100°C to 1000°C, more preferably 0°C to 700°C, further preferably 100°C to 500°C, and particularly preferably 200°C to 350°C.

Extraction is preferably separating or solid-liquid extraction with a Soxhlet extractor, and more preferably separating.

Examples of a solvent to be used for extraction are the same as the examples of a solvent to be used for the reaction in the treatment with a dehalogenating agent described below.

The chromatography is preferably column chromatography.

Preferable examples of a filler to be used for column chromatography are silica gel or alumina.

Examples of a solvent to be used for chromatography are the same as the examples of a solvent to be used for the reaction in the treatment with a dehalogenating agent described below.

Examples of a solvent to be used for reprecipitation are the same as the examples of a solvent to be used for the reaction in the treatment with a dehalogenating agent described below.

Examples of a solvent to be used for recrystallization are the same as the examples of a solvent to be used

for the reaction in the treatment with a dehalogenating agent described below.

The adsorption is preferably treatment with an adsorbent. The adsorbent is preferably active carbon, silica gel, alumina, or celite.

The treatment with an adsorbent is usually performed in a solvent. Examples of a solvent to be used for the treatment with an adsorbent are the same as the examples of a solvent to be used for the reaction in the treatment with a dehalogenating agent described below.

[Treatment with Dehalogenating Agent]

Examples of the treatment with dehalogenating agent include known methods described in literature such as International Publication No. WO 2006/037458, Japanese Unexamined Patent Publication No. 2007-220772, Japanese Unexamined Patent Publication No. 2007-077078, and International Publication No. WO 2005/084083.

Examples of the treatment with a dehalogenating agent include methods involving the reduction with a hydride reducing agent and methods involving the reaction with a metal or organometallic compound.

Examples of the hydride reducing agent include alkali metal hydrides and alkaline earth metal hydrides such as sodium hydride, lithium hydride, calcium hydride, and magnesium hydride; aluminum hydride compounds such as lithium aluminum hydride (LAH), diisobutylaluminium hydride (DIBAL) and sodium bis(2-methoxyethoxy)aluminum hydride (Red-Al); boron hydride compounds such as diborane ($B_2H_6$), sodium borohydride ($NaBH_4$), and lithium triethylborohydride (Super-Hydride); silicon hydride compounds such as silane ($SiH_4$) and triethyl silane ($Et_3SiH$); and tin hydride compounds such as stannane ($SnH_4$) and tributyltin hydride (TBT).

In the methods involving the reaction with a metal, examples of the metal include lithium, sodium, magnesium, and zinc.

In the methods involving the reaction with an organometallic compound, examples of the organometallic compound include organolithium compounds such as butyllithium and phenyllithium; organic magnesium compounds such as Grignard reagents; and organozinc compounds such as diethyl zinc.

A preferable treatment with a dehalogenating agent is a method involving the reaction with a compound represented by the formula (Z1) because $C^1$ can be decreased more.

[Chemical Formula 40] $R^{Z1}$-$Z^{Z1}$ (Z1)

[In the formula,

$R^{Z1}$ represents an alkyl group, a cycloalkyl group, an aryl group, or a monovalent heterocyclic group, and these groups each may have a substituent.

$Z^{Z1}$ represents a group selected from the group consisting of the group of substituents Z.]

<Group of Substituents Z>

A group represented by $-B(OR^{C2})_2$ (wherein $R^{C2}$ represents a hydrogen atom, an alkyl group, a cycloalkyl group, or an aryl group, and these groups each may have a substituent; and the plurality of $R^{C2}$ may be the same or different, and may be connected to each other to form a ring structure together with the oxygen atoms to which they are bonded);

a group represented by $-BF_3Q'$ (wherein Q' represents Li, Na, K, Rb, or Cs);

a group represented by -MgY' (wherein Y' represents a chlorine atom, a bromine atom, or an iodine atom);

a group represented by -ZnY" (wherein Y" represents a chlorine atom, a bromine atom, or an iodine atom); and

a group represented by $-Sn(R^{C3})_3$ (wherein $R^{C3}$ represents a hydrogen atom, an alkyl group, a cycloalkyl group, or an aryl group, and these groups each may have a substituent; and the plurality of $R^{C3}$ may be the same or different, and may be connected to each other to form a ring structure together with the tin atom to which they are bonded).

Examples of the group represented by $-B(OR^{C2})_2$ include groups represented by the following formulas (W-1) to (W-10).

[Chemical Formula 41]

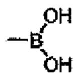
(W-1)
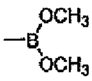
(W-2)
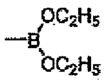
(W-3)
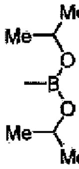
(W-4)
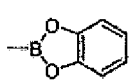
(W-5)
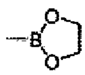
(W-6)
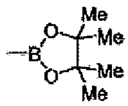
(W-7)
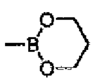
(W-8)
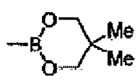
(W-9)
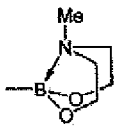
(W-10)

$R^{Z1}$ is preferably an aryl group or a monovalent heterocyclic group, more preferably an aryl group, and further preferably a phenyl group, and these groups each may have a substituent.

Substituents that $R^{Z1}$ may have are preferably a fluorine atom, an alkyl group, a cycloalkyl group, an aryl group, a monovalent heterocyclic group, an alkoxy group, a cycloalkoxy group, an aryloxy group, or a substituted amino group, more preferably an alkyl group, a cycloalkyl group, an alkoxy group, an aryl group, or a monovalent heterocyclic group, further preferably an alkyl group, a cycloalkyl group, or an alkoxy group, and particularly preferably an alkyl group or a cycloalkyl group, and these groups each may further have a substituent.

The group selected from the group of substituents Z is preferably a group represented by $-B(OR^{C2})_2$, and more preferably a group represented by the formula (W-7).

The treatment with a dehalogenating agent is usually performed in a solvent. Examples of the solvent to be used for the reaction include alcohol solvents such as methanol, ethanol, propanol, ethylene glycol, glycerin, 2-methoxyethanol, 2-ethoxyethanol; ether solvents such as diethyl ether, tetrahydrofuran (THF), dioxane, cyclopentyl methyl ether, and diglyme; halogen solvents such as methylene chloride and chloroform; nitrile solvents such as acetonitrile and benzonitrile; hydrocarbon solvents such as hexane, decalin, toluene, xylene, and mesitylene; amide solvents such as N,N-dimethylformamide and N,N-dimethylacetamide; acetone, dimethylsulfoxide, and water. One solvent may be used alone, or two or more solvents may be used in combination.

The amount of the solvent used is usually 10 to 100000 parts by mass when 100 parts by mass is defined as the total amount of the phosphorescent compound represented by formula (1).

In the treatment with a dehalogenating agent, the reaction time is usually from 30 minutes to 150 hours and the reaction temperature is usually between the melting point of the solvent in the reaction system and the boiling point.

In the treatment with a dehalogenating agent, a catalyst such as a palladium catalyst and a nickel catalyst may be used to promote the reaction. Examples of the palladium catalysts include palladium acetate, bis(triphenylphosphine)palladium (II) dichloride, tetrakis(triphenylphosphine)palladium (0), [1,1'-bis(diphenylphosphino)ferrocene]dichloropalladium (II), tris(dibenzylideneacetone)dipalladium (0). Examples of the nickel catalyst include tetrakis(triphenylphosphine)nickel (0), [1,3-bis(diphenylphosphino)propane]nickel (II) dichloride, and bis(1,4-cyclooctadiene)nickel (0). One catalyst may be used alone, or two or more catalysts may be used in combination.

The amount of the catalysts used is usually 0.00001 to 3 mol equivalent in terms of the amount of the transition metal with respect to the total number of moles of the phosphorescent compound represented by formula (1).

The palladium catalyst or nickel catalyst may be used together with a phosphorus compound such as triphenylphosphine, tri(o-tolyl)phosphine, tri(tert-butyl)phosphine, tricyclohexylphosphine, and 1,1'-bis(diphenylphosphino)ferrocene. One phosphorus compound may be used alone, or two or more phosphorus compounds may be used in combination.

In the treatment with a dehalogenating agent, a base and a phase transfer catalyst may be used to promote the reaction. Moreover, a catalyst and a base and/or a phase transfer catalyst may be used together as needed.

Examples of the base and phase transfer catalyst include inorganic bases such as sodium carbonate, potassium carbonate, cesium carbonate, potassium fluoride, cesium fluoride, and phosphate tripotassium; organic bases such as tetrabutyl ammonium fluoride, tetraethyl ammonium hydroxide, and tetrabutyl ammonium hydroxide; and phase transfer catalysts such as tetrabutyl ammonium chloride and tetrabutyl ammonium bromide. The base and the phase transfer catalyst each may each be used singly or in combination of two or more.

The amounts of the base and phase transfer catalyst used are each usually 0.001 to 100 mol equivalent with respect to the total number of moles of the phosphorescent compound represented by formula (1).

When the reaction is conducted two or more times in the treatment with a dehalogenating agent, they may be reacted under the same conditions or may be reacted under different conditions.

The method for reducing $C^1$ involves preferably treatment with a dehalogenating agent, more preferably both purification and treatment with a dehalogenating agent, further preferably both sublimation and/or recrystallization and treatment with a dehalogenating agent, and particularly preferably both recrystallization and treatment with a dehalogenating agent, because $C^1$ can be reduced more.

[Host material]

Next, the host material which is blended in the composition according to the present embodiment will be described.

The host material is a compound composed of main group element except chlorine.

The host material preferably has at least one function selected from the group consisting of luminous, hole injectability, hole transportability, electronic injectability, and electronic transportability, and more preferably has at least one function selected from the group consisting of hole injectability, hole transportability, electronic injectability, and electronic transportability.

The lowest excited triplet state ($T_1$) that the host material has is preferred at an energy level equal to $T_1$ that the phosphorescent compound represented by formula (1) has or a higher energy level because the light-emitting device according to the present embodiment has a good external quantum efficiency.

Examples of the host material include compounds composed of one or more types of atoms selected from hydrogen atoms, carbon atoms, Group 13 elements, Group 14 elements (except carbon atoms), Group 15 elements, Group 16 elements, and fluorine atoms; preferable examples are compounds composed of hydrogen atoms and carbon atoms or compounds composed of hydrogen atoms, carbon atoms, and one or more types of atoms selected from boron atoms, silicon atoms, nitrogen atoms, phosphorus atoms, oxygen atoms, sulfur atoms, selenium atoms, and fluorine atoms, more preferable examples are compounds composed of hydrogen atoms and carbon atoms or compounds composed of hydrogen atoms, carbon, and one or more types of atoms selected from nitrogen atoms, oxygen atoms, and sulfur atoms; further preferable examples are compounds composed of hydrogen atoms, carbon atoms, and one or more types of atoms selected from nitrogen atoms, oxygen atoms, and sulfur atoms; particularly preferable examples are compounds composed of hydrogen atoms, carbon atoms, and one or more types of atoms selected from nitrogen atoms and sulfur atoms; and more particularly preferable examples are compounds composed of hydrogen atoms, carbon atoms, nitrogen atoms, and sulfur.

Host materials are classified into low molecular weight compounds (hereinafter also referred to as "low molecular weight host materials".) and high molecular weight compounds (hereinafter also referred to as "high molecular weight host materials".) and preferably low molecular weight compounds.

[High molecular weight host material]

Examples of the high molecular weight host materials include high molecular weight compounds that are hole transporting materials described below and high molecular weight compounds that are electron transporting materials described below.

[Low molecular weight host material]

Examples of the low molecular weight host materials include compounds having an aromatic hydrocarbon ring composed of hydrogen atoms and carbon atoms and heterocyclic compounds composed of main group elements and heterocyclic compounds composed of main group elements are preferred.

Preferable examples of the heterocyclic compounds in the low molecular weight host materials are heterocyclic compounds composed of hydrogen atoms, carbon atoms, and one or more types of atoms selected from Group 13 elements, Group 14 elements, Group 15 elements, Group 16 elements, and fluorine atoms, more preferable examples are heterocyclic compounds composed of hydrogen atoms, carbon atoms, and one or more types of atoms selected from the group consisting of boron atoms, silicon atoms, nitrogen atoms, phosphorus atoms, oxygen atoms, sulfur atoms, selenium atoms, and fluorine atoms, further preferable examples are heterocyclic compounds composed of hydrogen atoms, carbon atoms, and one or more types of atoms selected from the group consisting of nitrogen atoms, oxygen atoms, and sulfur atoms, particularly preferable examples are heterocyclic compounds composed of atoms of hydrogen atoms, carbon atoms, and one or more types of atoms selected from the group consisting of nitrogen atoms and sulfur atoms.

The substituent that the aromatic hydrocarbon ring and heterocyclic ring may have in the low molecular weight host materials is preferably a fluorine atom, an alkyl group, a cycloalkyl group, an alkoxy group, a cycloalkoxy group, an aryloxy group, an aryl group, a monovalent heterocyclic group, or a substituted amino group, more preferably an alkyl group, a cycloalkyl group, an alkoxy group, an aryl group, a monovalent heterocyclic group, or a substituted amino group, more preferably an alkyl group, a cycloalkyl group, an aryl group, a monovalent heterocyclic group, or a substituted amino group, particularly preferably an alkyl group, an aryl group, or a monovalent heterocyclic group, more particularly preferably a monovalent heterocyclic group, and these groups each may further have a substituent.

The heterocyclic compounds in the low molecular weight host materials are preferably low molecular weight compounds having an aromatic heterocyclic ring that may have a substituent (i.e., aromatic heterocyclic compounds).

Preferable ranges of atoms composed of the aromatic heterocyclic ring in the low molecular weight host materials are the same as the preferable ranges of atoms composed of the heterocyclic ring in the low molecular weight host materials described above.

Preferable ranges of the substituent that the aromatic heterocyclic ring may have in the low molecular weight host materials are the same as the preferable ranges of the substituent in the heterocyclic ring may have in the low molecular weight host materials described above.

Examples of the aromatic hydrocarbon ring in the low molecular weight host materials include a benzene ring, a naphthalene ring, an anthracene ring, a fluorene ring, a spirobifluorene ring, a phenanthrene ring, a dihydrophenanthrene ring, a pyrene ring, a chrysene ring, and a triphenylene ring, the aromatic hydrocarbon ring is preferably a benzene ring, a naphthalene ring, a fluorene ring, a spirobifluorene ring, a phenanthrene ring, a dihydrophenanthrene ring, a chrysene ring, or a triphenylene ring, more preferably a benzene ring, a fluorene ring, or a spirobifluorene ring, further preferably a benzene ring, and these rings each may have a substituent.

Examples of the heterocyclic ring in the low molecular weight host materials include a pyrrole ring, a furan ring, a thiophene ring, an oxadiazole ring, a thiadiazole ring, a thiazole ring, an oxazole ring, a pyridine ring, a diazabenzene ring, a triazine ring, a quinoline ring, an isoquinoline ring, a quinazoline ring, a quinoxaline ring, a phenanthroline ring, a dibenzofuran ring, a dibenzothiophene ring, a dibenzosilole ring, a dibenzophosphole ring, a carbazole ring, an azacarbazole ring, a diazacarbazole ring, a phenoxazine ring, and a phenothiazine ring, the heterocyclic ring is preferably a pyridine ring, a diazabenzene ring, a triazine ring, a quinoline ring, an isoquinoline ring, a quinazoline ring, a quinoxaline ring, a phenanthroline ring, a dibenzofuran ring, a dibenzothiophene ring, a carbazole ring, an azacarbazole ring, or a diazacarbazole ring, more preferably a pyridine ring, a pyrimidine ring, a triazine ring, a quinoline ring, an isoquinoline ring, a quinazoline ring, a dibenzofuran ring, a dibenzothiophene ring, or a carbazole ring, further preferably a pyridine ring, a pyrimidine ring, a triazine ring, a dibenzofuran ring, a dibenzothiophene ring, or a carbazole ring, particularly preferably a dibenzofuran ring, a dibenzothiophene ring, or a carbazole ring, more particularly preferably a dibenzothiophene ring or a carbazole ring, and these rings each may have a substituent.

[Compound Represented by Formula (H-1)]

The low molecular weight host material is preferably a compound represented by formula (H-1).

$Ar^{H1}$ and $Ar^{H2}$ are preferably a phenyl group, a fluorenyl group, a spirobifluorenyl group, a pyridyl group, a pyrimidinyl group, a triazinyl group, a quinolinyl group, an isoquinolinyl group, a thienyl group, a benzothienyl group, a dibenzothienyl group, a furyl group, a benzofuryl group, a dibenzofuryl group, a pyrrolyl group, an indolyl group, an azaindolyl group, a carbazolyl group, an azacarbazolyl group, a diazacarbazolyl group, a phenoxazinyl group, or a phenothiazinyl group, more preferably a phenyl group, a spirobifluorenyl group, a pyridyl group, a pyrimidinyl group, a triazinyl group, a dibenzothienyl group, a dibenzofuryl group, a carbazolyl group, or an azacarbazolyl group, still more preferably a phenyl group, a pyridyl group, a carbazolyl group, or an azacarbazolyl group, particularly preferably a group represented by the above-mentioned formula (TDA-1) or (TDA-3), and especially preferably a group represented by the above-mentioned formula (TDA-3), and these groups each may have a substituent.

The optional substituent of $Ar^{H1}$ and $Ar^{H2}$ is preferably a fluorine atom, an alkyl group, a cycloalkyl group, an alkoxy group, a cycloalkoxy group, an aryl group, or a monovalent heterocyclic group, more preferably an alkyl group, a cycloalkyl group, an alkoxy group, or a cycloalkoxy group, and still more preferably an alkyl group or a cycloalkoxy group is more preferable, and these groups each may further have a substituent.

$n^{H1}$ is preferably 1. $n^{H2}$ is preferably 0.

$n^{H3}$ is usually an integer of 0 to 10, preferably an integer of 0 to 5, more preferably an integer of 1 to 3, and particularly preferably 1.

$n^{H11}$ is preferably an integer of 1 or more and 5 or less, more preferably an integer of 1 or more and 3 or less, and still more preferably 1.

$R^{H11}$ is preferably a hydrogen atom, an alkyl group, a cycloalkyl group, an aryl group, or a monovalent heterocyclic group, more preferably a hydrogen atom, an alkyl group, or a cycloalkyl group, and still more preferably a hydrogen atom or an alkyl group, and these groups each may have a substituent.

$L^{H1}$ is preferably an arylene group or a divalent heterocyclic group.

$L^{H1}$ is preferably a group represented by formulas (A-1) to (A-3), (A-8) to (A-10), (AA-1) to (AA-6), (AA-10) to (AA-21), or (A-24) to (AA-34), more preferably a group represented by formula (A-1), (A-2), (A-8), (A-9), (AA-1) to (AA-4), (AA-10) to (AA-15), or (A-29) to (AA-34), still more preferably a group represented by formula (A-1), (A-2), (A-8), (A-9), (AA-2), (AA-4), or (AA-10) to (AA-15), particularly preferably a group represented by formula (A-1), (A-2), (A-8), (AA-2), (AA-4), (AA-10), (AA-12), or (AA-14), and especially preferably a group represented by formula (A-1), (A-2), (AA-2), (AA-4), (AA-10), (AA-12), or (AA-14), yet more preferably a group represented by formula (AA-10), (AA-12), or (AA-14), and most preferably a group represented by formula (AA-14).

The optional substituent of $L^{H1}$ is preferably a fluorine atom, an alkyl group, a cycloalkyl group, an alkoxy group,

a cycloalkoxy group, an aryl group, or a monovalent heterocyclic group, more preferably an alkyl group, an alkoxy group, an aryl group, or a monovalent heterocyclic group, and still more preferably an alkyl group, an aryl group, or a monovalent heterocyclic group, and these groups each may further have a substituent.

$L^{H21}$ is preferably a single bond or an arylene group, and more preferably a single bond, and the arylene group may have a substituent.

The definition and examples of the arylene group or divalent heterocyclic group represented by $L^{H21}$ are the same as the definition and examples of the arylene group or divalent heterocyclic group represented by $L^{H1}$.

$R^{H21}$ is preferably an aryl group or a monovalent heterocyclic group, and these groups each may have a substituent.

The definition and examples of the aryl group or the monovalent heterocyclic group represented by $R^{H21}$ are the same as the definition and examples of the aryl group or the monovalent heterocyclic group represented by $Ar^{H1}$ and $Ar^{H2}$.

The definition and examples of the optional substituent of $R^{H21}$ are the same as the definitions and examples of the optional substituent of $Ar^{H1}$ and $Ar^{H2}$.

The compound represented by formula (H-1) is preferably a compound represented by formula (H-2).

[0216] [Chemical Formula 42]

$$Ar^{H1} - \left[ L^{H1} \right]_{n^{H3}} - Ar^{H2} \qquad \textbf{(H-2)}$$

[In the formula, $Ar^{H1}$, $Ar^{H2}$, $n^{H3}$, and $L^{H1}$ represent the same meanings as described above.]

Examples of the low molecular weight host material include the compounds represented by following formulas (H-101) to (H-118).

[Chemical Formula 43]

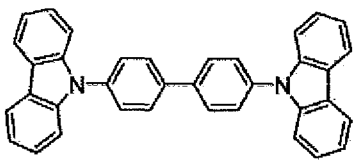

(H-101)

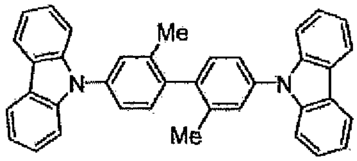

(H-102)

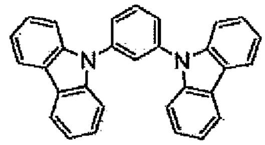

(H-103)

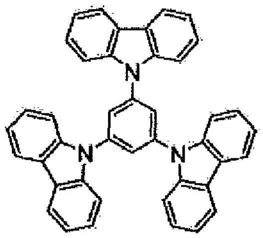

(H-104)

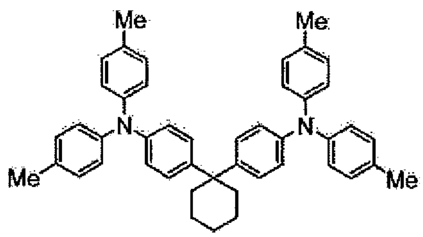

(H-105)

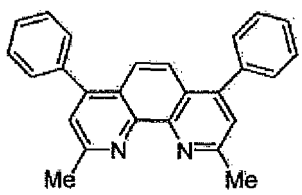

(H-106)

[Chemical Formula 44]

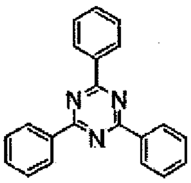

(H-107)

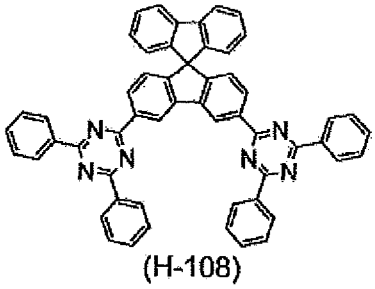

(H-108)

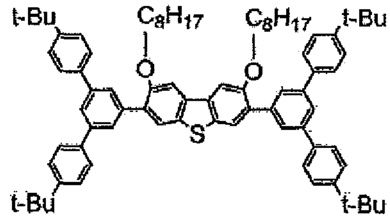

(H-109)

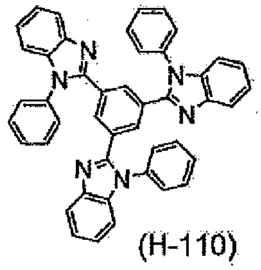

(H-110)

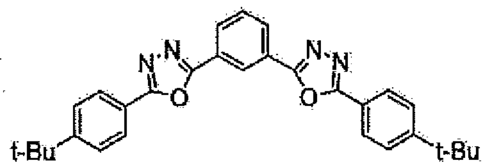

(H-111)

[Chemical Formula 45]

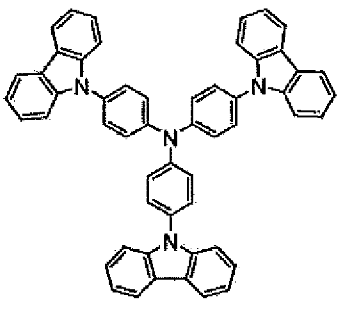

(H-112)

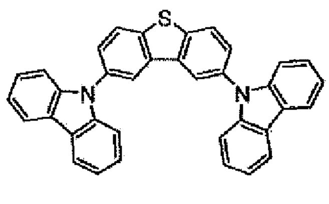

(H-113)

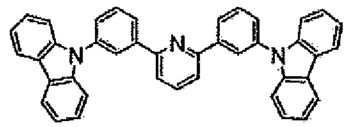

(H-114)

[Chemical Formula 46]

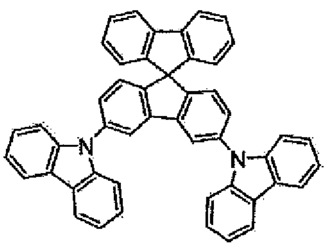

(H-115)

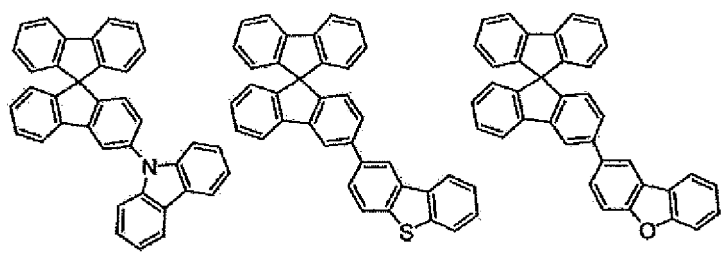

(H-116) (H-117) (H-118)

[Method for Obtaining Low Molecular Weight Host Material]

The low molecular weight host materials can be obtained from Aldrich, Luminescence Technology Corp.

Moreover, the low molecular weight host materials can also be obtained by the preparation by known methods described in literature such as International Publication No. WO 2006/121811, International Publication No. WO 2007/097153, International Publication No. WO 2009/086028, International Publication No. WO 2009/096202, Japanese Unexamined Patent Publication 2009-46408, and Japanese Unexamined Patent Publication 2009-267255.

[Method for Reducing Amount ($C^H$) of Chlorine Atoms Contained in Low Molecular Weight Host Material]

Examples of the method for reducing the amount ($C^H$) of chlorine atoms contained as impurities in the low molecular weight host materials include at least one method selected from among purification and treatment with a dehalogenating agent, and because the amount of chlorine atoms contained in the low molecular weight host materials can be reduced more, a preferable example is purification, more preferable examples are sublimation and/or recrystallization, and further preferable example is sublimation.

Examples, definitions, and preferable ranges of the purification and treatment with a dehalogenating agent in the method for reducing the amount of chlorine atoms contained in the low molecular weight host materials are the same as the examples, definitions, and preferable ranges of the purification and treatment with a dehalogenating agent in the method for reducing $C^1$ described above.

The amount of solvent used in the treatment with a dehalogenating agent in the method for reducing the amount of chlorine atoms contained in the low molecular weight host materials is usually 10 to 100000 parts by mass when 100 parts by mass is defined as the total amount of the low molecular weight host materials.

The amount of catalyst used in the treatment with a dehalogenating agent in the method for reducing the amount of chlorine atoms contained in the low molecular weight host materials is usually 0.00001 to 3 mol equivalent in terms of the amount of transition metal with respect to the total number of moles of the low molecular weight host materials.

The amounts of the base and the phase transfer catalyst used in the treatment with a dehalogenating agent in the method for reducing the amount of chlorine atoms contained in the low molecular weight host materials are each usually 0.001 to 100 mol equivalent with respect to the total number of moles of the low molecular weight host materials.

The amount ($C^H$) of chlorine atoms contained as impurities in the host materials is not particularly limited, but usually less than 50 ppm by mass, preferably less than 30 ppm by mass, more preferably 9 ppm by mass or less, further preferably 5 ppm by mass or less, particularly preferably 1 ppm by mass or less, and more particularly preferably 0 ppm by mass with respect to the total amount of the host materials because the initial degradation of the light-emitting device according to the present embodiment is suppressed.

A specific method for calculating $C^H$ that can determine $C^H$ is that similar to the specific methods for calculating $C^1$.

For example, similarly to the specific method for calculating $C^1$ in Example D1 as described above, $C^H$ in Example D1 is 0 ppm by mass.

<Composition>

The composition according to the present embodiment is a A composition in which a phosphorescent compound represented by formula (1) and a host material are blended with each other, wherein the amount of chlorine atoms blended in the phosphorescent compound as impurities is 3.5 ppm by mass or less with respect to the total amount of solid contents comprised in the composition.

In the composition according to the present embodiment, one phosphorescent compound represented by formula (1) may be blended alone, or two or more phosphorescent compounds represented by formula (1) may be blended in combination. Moreover, in the composition according to the present embodiment, one host material may be blended alone, or two or more host materials may be blended in combination.

In the composition according to the present embodiment, the total amount of chlorine atoms contained as impurities in the phosphorescent compound and chlorine atoms contained as impurities in the host material with respect to the total amount of solid contents blended in the composition is preferably 3.5 ppm by mass or less, more preferably 3.1 ppm by mass or less, further preferably 2.7 ppm by mass or less, particularly preferably 2.3 ppm by mass or less, more particularly preferably 1.8 ppm by mass or less, yet preferably 1.2 ppm by mass, yet more preferably 0.8 ppm by mass or less, yet further preferably 0.3 ppm by mass or less, and yet particularly preferably 0 ppm by mass, because the initial degradation of the light-emitting device according to the present embodiment is suppressed.

Moreover, in the composition according to the present embodiment, the total amount of chlorine atoms contained as impurities in the phosphorescent compound and chlorine atoms contained as impurities in the host material with respect to the total amount of solid contents blended in the composition is preferably 0.01 ppm by mass or more and 3.0 ppm by mass or less, more preferably 0.02 ppm by mass or more and 2.7 ppm by mass or less, further preferably 0.05 ppm by mass or more and 2.5 ppm by mass or less, particularly preferably 0.1 ppm by mass or more and 2.3 ppm by mass or less, and more particularly preferably 0.2 ppm by mass or more and 2.3 ppm by mass or less, because the light-emitting device according to the present embodiment has a good luminous efficiency.

Moreover, in the composition according to the present embodiment, the total amount of chlorine atoms contained as impurities in the phosphorescent compound and chlorine atoms contained as impurities in the host material with respect to the total amount of solid contents blended in the composition is preferably 0.01 ppm by mass or more and 2.3 ppm by mass or less, more preferably 0.02 ppm by mass or more and 1.8 ppm by mass or less, further preferably 0.05 ppm by mass or more and 1.2 ppm by mass or less, and particularly preferably 0.1 ppm by mass or more and 0.8

ppm by mass or less, because the initial degradation of the light-emitting device according to the present embodiment is suppressed and the light-emitting device according to the present embodiment has a good luminous efficiency.

For example, when the solid contents blended in the composition according to the present embodiment is the phosphorescent compound and host material only, the total amount (ppm by mass) of chlorine atoms contained as impurities in the phosphorescent compound and chlorine atoms contained as impurities in the host material is expressed as $C^1W^1+C^HW^H$ wherein $W^1$ is the ratio of the mass of the phosphorescent compound represented by formula (1) to the total mass of the phosphorescent compound represented by formula (1) and the host material, and $W^H$ is the ratio of the total mass of the host material to the total mass of the phosphorescent compound represented by formula (1) and the host material.

$W^1$ is usually 0.0001 to 0.90, preferably 0.01 to 0.60, and more preferably 0.10 to 0.40 because the initial degradation of the light-emitting device according to the present embodiment is more suppressed.

A specific method for calculating $W^1$ will be described with reference to Example D1 and Example D2 described below.

First, in Example D1, the mass ratio of the compound HM-1 (host material) and the phosphorescent compound MC3 is compound HM-1:phosphorescent compound MC3 = 75:25.

Accordingly, $W^1$ can be determined from the charged amount and is determined as follows.

$$W^1 = 25/(75 + 25) = 0.25$$

In Example D2, the mass ratio of the compound HM-1 and the phosphorescent compound MC3 and the phosphorescent compound MC2 is compound HM-1:phosphorescent compound MC3:phosphorescent compound MC2 = 75:22.5:2.5

Accordingly, $W^1$ can be determined from the charged amount and is determined as follows.

$$W^1 = (22.5 + 2.5)/(75 + 22.5 + 2.5) = 0.25$$

Similarly, $W^1$ in Example D3 is determined as follows.

$$W^1 = (12.5 + 12.5)/(75 + 12.5 + 12.5) = 0.25$$

Similarly, $W^1$ in Example D4 is determined as follows.

$$W^1 = 25/(75 + 25) = 0.25$$

Similarly, $W^1$ in Example D5 is determined as follows.

$$W^1 = (12.5 + 12.5)/(75 + 12.5 + 12.5) = 0.25$$

Similarly, $W^1$ in Comparative Example CD1 is determined as follows.

$$W^1 = 25/(75 + 25) = 0.25$$

$W^H$ is usually 0.1 to 0.9999, preferably 0.40 to 0.99, and more preferably 0.60 to 0.90, because the initial degradation of the light-emitting device according to the present embodiment is more suppressed.

In a specific method for calculating $W^H$, $W^H$ can be determined in the same manner as the specific methods for calculating $W^1$.

For example, similarly to the specific method for calculating $W^1$ in Example D1 as described above, $W^H$ in Example D1 is determined as follows.

$$W^H = 75/ (75 + 25) = 0.75$$

As described above, $C^1W^1 + C^HW^H$ can be calculated by calculating $C^1$, $C^H$, $W^1$, and $W^H$.

For example, $C^1W^1 + C^HW^H$ in Example D1 is determined as follows.

$$C^1W^1 + C^HW^H = (0 \times 0.25) + (0 \times 0.75) = 0 \text{ ppm by mass}$$

$C^1W^1 + C^HW^H$ in Example D2 is determined as follows.

$$C^1W^1 + C^HW^H = (0.9 \times 0.25) + (0 \times 0.75) = 0.23 \text{ ppm by mass}$$

$C^1W^1 + C^HW^H$ in Example D3 is determined as follows.

$$C^1W^1 + C^HW^H = (4.5 \times 0.25) + (0 \times 0.75) = 1.13 \text{ ppm by mass}$$

$C^1W^1 + C^HW^H$ in Example D4 is determined as follows.

$$C^1W^1 + C^HW^H = (9 \times 0.25) + (0 \times 0.75) = 2.25 \text{ ppm by mass}$$

$C^1 W^1 + C^HW^H$ in Example D5 is determined as follows.

$$C^1W^1 + C^HW^H = (12.5 \times 0.25) + (0 \times 0.75) = 3.13 \text{ ppm by mass}$$

$C^1W^1 + C^HW^H$ in Comparative Example CD1 is determined as follows.

$$C^1W^1 + C^HW^H = (16 \times 0.25) + (0 \times 0.75) = 4.00 \text{ ppm by mass}$$

$C^1W^1 + C^HW^H$ is 3.5 ppm by mass or less, preferably 3.1 ppm by mass or less, more preferably 2.7 ppm by mass or less, further preferably 2.3 ppm by mass or less, particularly preferably 1.8 ppm by mass or less, more particularly preferably 1.2 ppm by mass, yet preferably 0.8 ppm by mass or less, yet more preferably 0.3 ppm by mass or less, most preferably 0 ppm by mass, because the initial degradation of the light-emitting device according to the present embodiment is suppressed.

Moreover, $C^1W^1 + C^HW^H$ is preferably 0.01 ppm by mass or more and 3.0 ppm by mass or less, more preferably 0.02 ppm by mass or more and 2.7 ppm by mass or less, further preferably 0.05 ppm by mass or more and 2.5 ppm by mass or less, particularly preferably 0.1 ppm by mass or more and 2.3 ppm by mass or less, and more particularly preferably 0.2 ppm by mass or more and 2.3 ppm by mass or less, because the light-emitting device according to the present embodiment has a good luminous efficiency.

Moreover, $C^1W^1 + C^HW^H$ is preferably 0.01 ppm by mass or more and 2.3 ppm by mass or less, more preferably 0.02 ppm by mass or more and 1.8 ppm by mass or less, further preferably 0.05 ppm by mass or more and 1.2 ppm by mass or less, and particularly preferably 0.1 ppm by mass or more and 0.8 ppm by mass or less, because the initial degradation of the light-emitting device according to the present embodiment is suppressed and the light-emitting device according to the present embodiment has a good luminous efficiency.

Preferably, the composition according to the present embodiment further satisfy formula (2').

$$C^H \leq C^1 \leq 15 \text{ ppm by mass} \quad (2')$$

[In the formula, $C^1$ and $C^H$ represent the same meanings as those described above.]

The formula (2') is preferably formula (2'-1), more preferably formula (2'-2), further preferably formula (2'-3), particularly preferably formula (2'-4), and more particularly preferably formula (2'-5).

$$C^H \leq C^1 \leq 13 \text{ ppm by mass} \quad (2'\text{-}1)$$

$$C^H \leq C^1 \leq 9 \text{ ppm by mass} \quad (2'\text{-}2)$$

$$C^H \leq C^1 \leq 5 \text{ ppm by mass} \quad (2'\text{-}3)$$

$$C^H \leq C^1 \leq 1 \text{ ppm by mass} \quad (2'\text{-}4)$$

$$C^H = C^1 = 0 \text{ ppm by mass} \quad (2'\text{-}5)$$

[In the formula, $C^1$ and $C^H$ represent the same meanings as those described above.]

The amount of the phosphorescent compound represented by formula (1) blended in the composition according to the present embodiment is not particularly limited, but the amount may be 0.01 to 90% by mass, is preferably 1 to 60% by mass, and more preferably 10 to 40% by mass with respect to the total amount of solid contents contained in the composition.

The amount of the host material contained in the composition according to the present embodiment is not particularly limited, but the amount may be 10 to 99.99% by mass, is preferably 40 to 99% by mass, and more preferably 60 to 90% by mass with respect to the total amount of solid contents blended in the composition.

[Other Components]

The composition according to the present embodiment may further comprise at least one material selected from the group consisting of a hole transporting material, a hole injecting material, an electron transporting material, an electron injecting material, a luminescent material (different from the phosphorescent compound represented by formula (1) and the host material.), an antioxidant, and a solvent, provided that the hole transporting material, the hole injecting material, the electron transporting material, and the electron injecting material are different from the host material.

When the composition according to the present embodiment comprises at least one material selected from the group consisting of a hole transporting material, a hole injecting material, an electron transporting material, an electron injecting material, a luminescent material, and an antioxidant, the amounts of chlorine atoms contained in these materials are preferably reduced by at least one method selected from the purification as described above and the treatment with a dehalogenating agent as described above.

[Hole Transporting Material]

The hole transporting material is classified into a low molecular weight compound and a polymer compound, and a polymer compound is preferable. The hole transporting material may have a crosslinkable group.

Examples of the polymer compounds include polyvinylcarbazole and derivatives thereof; and polyarylene having an aromatic amine structure in a side chain or main chain and derivatives thereof. The polymer compound may be a compound to which an electron accepting site is bound. Examples of the electron accepting moiety include fullerene, tetrafluorotetracyanoquinodimethane, tetracyanoethylene, trinitrofluorenone and the like, and fullerene is preferable.

In the composition according to the present embodiment, the amount of the hole transporting material is, based on 100 parts by mass for the total of the phosphorescent compound represented by formula (1) and the host material, usually 1 to 400 parts by mass, and preferably 5 to 150 parts by mass.

One hole transporting material may be used alone, or two or more hole transporting materials may be used in combination.

[Electron Transporting Material]

The electron transporting materials are classified into a low molecular weight compound and a polymer compound. The electron transporting material may have a crosslinkable group.

Examples of the low molecular weight compound include metal complexes having 8-hydroxyquinoline as a ligand, oxadiazole, anthraquinodimethane, benzoquinone, naphthoquinone, anthraquinone, tetracyanoanthraquinodimethane, fluorenone, diphenyldicyanoethylene, and diphenoquinone, and derivatives of these.

Examples of the polymer compound include polyphenylene, polyfluorene, and derivatives thereof. The polymer compound may be doped with a metal.

In the composition according to the present embodiment, the amount of the electron transporting material blended is, based on 100 parts by mass for the total of the phosphorescent compound represented by formula (1) and the host material, usually 1 to 400 parts by mass, and preferably 5 to 150 parts by mass.

One electron transporting material may be used alone, or two or more hole transporting materials may be used in combination.

[Hole Injecting Material and Electron Injecting Material]

The hole injecting material and the electron injecting material are each classified into a low molecular weight compound and a polymer compound. The hole injecting material and the electron injecting material may have a crosslinkable group.

Examples of the low molecular weight compound include metal phthalocyanines such as copper phthalocyanine; carbon; metal oxides of molybdenum, tungsten, and the like; and metal fluorides such as lithium fluoride, sodium fluoride, cesium fluoride, and potassium fluoride.

Examples of the polymer compound include conductive polymers such as polyaniline, polythiophene, polypyrrole, polyphenylenevinylene, polythienylenevinylene, polyquinoline, and polyquinoxaline, and derivatives of these; and polymers comprising an aromatic amine structure in the main chain or side chain.

In the composition according to the present embodiment, the amount of the hole injecting material and the electron injecting material blended is respectively, based on 100 parts by mass for the total of the phosphorescent compound represented by formula (1) and the host material, usually 1 to 400 parts by mass, and preferably 5 to 150 parts by mass.

One of each of the electron transporting material and the hole transporting material may be used alone, or two or more of each of the electron transporting material and the hole transporting material may be used in combination.

[Ion Doping]

When the hole injecting material or the electron injecting material comprises a conductive polymer, the electrical conductivity of the conductive polymer is preferably $1 \times 10^{-5}$ S/cm to $1 \times 10^{3}$ S/cm. The conductive polymer can be doped with an appropriate amount of ions in order to set the electrical conductivity of the conductive polymer in such a range.

The type of ion to be doped is an anion for the hole injecting material and a cation for the electron injecting material. Examples of the anion include polystyrenesulfonic acid ions, alkylbenzenesulfonic acid ions, and camphorsulfonic acid ions. Examples of the cation include lithium ions, sodium ions, potassium ions, and tetrabutylammonium ions.

One type of ion to be doped may be used alone, or two or more types of ion to be doped may be used.

[Light-Emitting Material]

The light-emitting material (which is different from the phosphorescent compound represented by formula (1) and the host material) is classified into a low molecular weight compound and a polymer compound. The light-emitting material may have a crosslinkable group.

Examples of the low molecular weight compound include naphthalene and derivatives thereof, anthracene and derivatives thereof, and perylene and derivatives thereof.

Examples of the polymer compound include a polymer compound comprising a phenylene group, a naphthalenediyl group, an anthracenediyl group, a fluorenediyl group, a phenanthrenediyl group, a dihydrophenanthrenediyl group, a group represented by formula (X), a carbazolediyl group, a phenoxazinediyl group, a phenothiazinediyl group, a pirenediyl group, and the like.

The luminescent material preferably includes a triplet luminescence complex and a high molecular weight compound.

Examples of the triplet luminescence complex include the metal complexes illustrated below.

[Chemical Formula 47]

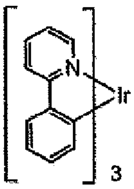
Ir(ppy)3

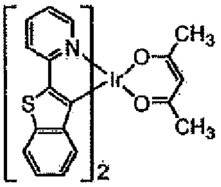
Btp2Ir(acac)

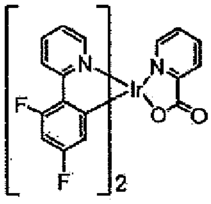
FIrpic

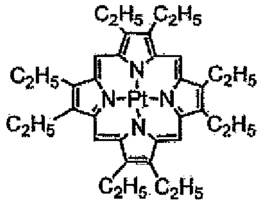
PtOEP

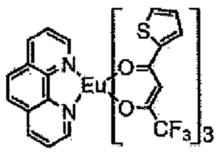
Eu(TTA)3phen

[Chemical Formula 48]

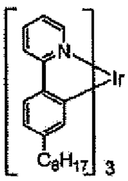
COM-1

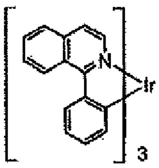
COM-2

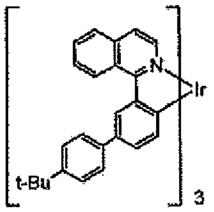
COM-3

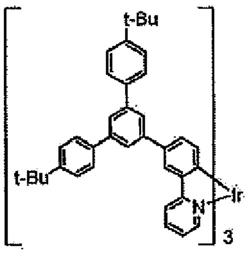
COM-4

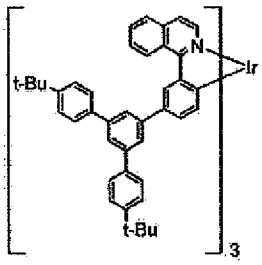
COM-5

[Chemical Formula 49]

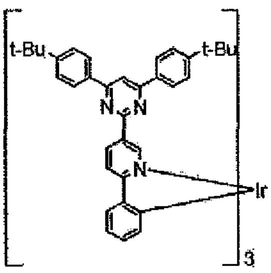
COM-6

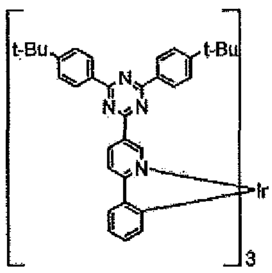
COM-7

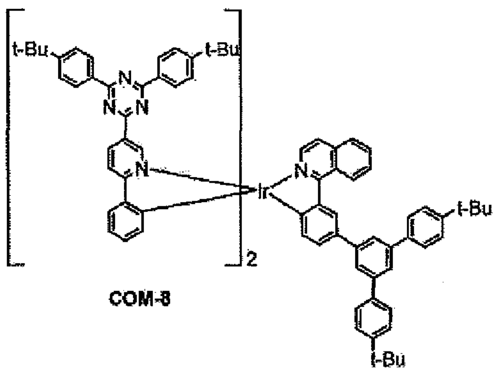
COM-8

In the composition according to the present embodiment, the amount of the luminescent material contained is usually 0.1 to 400 parts by mass and preferably 1 to 150 parts by mass when 100 parts by mass is defined as the total amount of the phosphorescent compound represented by formula (1) and the host material.

The luminescent material may be used singly or in combination of two or more.

[Antioxidant]

The antioxidant may be any compound that is soluble in the same solvent as the phosphorescent compound represent by formula (1) and the host material, and that does not inhibit light emission and charge transporting. Examples of the antioxidant include a phenol type antioxidant and a phosphorus type antioxidant.

In the composition according to the present embodiment, the content of the antioxidant blended is, based on 100 parts by mass for the total of the phosphorescent compound represented by formula (1) and the host material, usually 0.001 to 10 parts by mass.

One antioxidant may be used alone, or two or more antioxidants may be used in combination.

[Ink]

A composition (hereinafter also referred to as "ink") comprising a phosphorescent compound represented by formula (1), a host material, and a solvent can be suitably used in a coating method such as spin coating, casting, micro gravure coating, gravure coating, bar coating, roll coating, wire bar coating, dip coating, spray coating, screen printing, flexographic printing, an offset printing method, ink jet printing, capillary coating, and nozzle coating.

The viscosity of the ink may be adjusted according to the type of coating method. However, when a solution of an ink jet printing method is applied in a printing method that employs an ejection apparatus, the viscosity is preferably 1 to 20 mPa·s at 25°C in order to prevent clogging and curved flight during ejection.

The solvent contained in the ink is preferably a solvent capable of dissolving or uniformly dispersing the solid content in the ink. Examples of the solvent include chlorinated solvents such as 1,2-dichloroethane, 1,1,2-trichloroethane, chlorobenzene, and o-dichlorobenzene; ether solvents such as THF, dioxane, anisole, and 4-methylanisole; aromatic hydrocarbon solvents such as toluene, xylene, mesitylene, ethylbenzene, n-hexylbenzene, and cyclohexylbenzene; aliphatic hydrocarbon solvents such as cyclohexane, methylcyclohexane, n-pentane, n-hexane, n-heptane, n-octane, n-nonane, n-decane, n-dodecane, and bicyclohexyl; ketone solvents such as acetone, methyl ethyl ketone, cyclohexanone, and acetophenone; ester solvents such as ethyl acetate, butyl acetate, ethyl cellosolve acetate, methyl benzoate, and phenyl acetate; polyhydric alcohol solvents such as ethylene glycol, glycerin, and 1,2-hexanediol; alcohol solvents such as isopropyl alcohol and cyclohexanol; and sulfoxide solvents such as dimethylsulfoxide; amide solvents such as N-methyl-2-pyrrolidone and N,N-dimethylformamide. One solvent may be used alone, or two or more solvents may be used in combination.

In the ink, the content of the solvent blended is, based on 100 parts by mass for the total of the phosphorescent compound represented by formula (1) and the host material, usually 1000 to 100000 parts by mass, and preferably 2000 to 20000.

<Light-emitting device>

The light-emitting device according to the present embodiment is a light-emitting device comprising an organic layer comprising the composition according to the present embodiment or a light-emitting device comprising an organic layer in which the phosphorescent compound according to the present embodiment is blended.

The light-emitting device according to the present embodiment may be configured to have, for example, electrodes consisting of an anode and a cathode and an organic layer comprising the composition according to the present embodiment or an organic layer comprising the phosphorescent compound according to the present embodiment provided between the electrodes.

[Layer Configuration]

The organic layer comprising the composition according to the present embodiment and the organic layer in which the phosphorescent compound according to the present embodiment is blended are usually one or more layers selected from the group consisting of a light-emitting layer, a hole transporting layer, a hole injecting layer, an electron transporting layer, and an electron injecting layer, and the organic layers are preferably light-emitting layers. These layers respectively comprise a luminescent material, a hole transporting material, a hole injecting material, an electron transporting material, an electron injecting material. These layers can be formed by the method same as that in the preparation of a film described above, using inks prepared by respectively dissolving a luminescent material, a hole transporting material, a hole injecting material, an electron transporting material, an electron injecting material into the solvent described above.

A light-emitting device comprises a light-emitting layer between an anode and a cathode. From the viewpoints of hole injectability and the hole transportability, the light-emitting device according to the present embodiment preferably comprises at least one layer of a hole injecting layer and a hole transporting layer between the anode and the light-emitting layer, and from the viewpoints of electron injectability and electron transportability, preferably comprises at least one layer of an electron injecting layer and an electron transporting layer between the cathode and the light-emitting layer.

Examples of the materials for the hole transporting layer, the electron transporting layer, the light-emitting layer, the hole injecting layer and the electron injecting layer include, in addition to the composition according to the present embodiment and the phosphorescent compound according to the present embodiment, each of the various hole transporting materials, electron transporting materials, light-emitting materials, hole injecting materials, and electron injecting materials described above.

When the light-emitting device according to the present embodiment comprises a hole transporting layer, the hole transporting material used to form the hole transporting layer is preferably a polymer compound (hereinafter also referred to as "polymer compound of the hole transporting layer") comprising a constitutional unit represented by the

following formula (X) and at least one constitutional unit selected from the group consisting of a constitutional unit represented by formula (3) and a constitutional unit represented by formula (4). The polymer compound of the hole transporting layer may further comprise a constitutional unit represented by the following formula (Y).

[Chemical Formula 50]

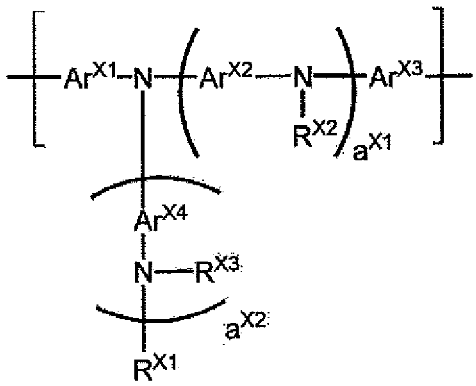

(X)

[In the formula,

$a^{X1}$ and $a^{X2}$ each independently represent an integer of 0 or more.

$Ar^{X1}$ and $Ar^{X3}$ each independently represent an arylene group or a divalent heterocyclic group, and these groups each may have a substituent.

$Ar^{X2}$ and $Ar^{X4}$ each independently represent an arylene group, a divalent heterocyclic group, or a divalent group in which at least one arylene group and at least one divalent heterocyclic group are directly bonded to each other, and these groups each may have a substituent. When there are a plurality of $Ar^{X2}$ and $Ar^{X4}$, they may be the same or different at each occurrence.

$R^{X1}$, $R^{X2}$, and $R^{X3}$ each independently represent a hydrogen atom, an alkyl group, a cycloalkyl group, an aryl group, or a monovalent heterocyclic group, and these groups each may have a substituent. When there are a plurality of $R^{X2}$ and $R^{X3}$, they may be the same or different at each occurrence.]

$a^{X1}$ is preferably 2 or less, and more preferably 1, because the initial degradation of the light-emitting device according to the present embodiment is more suppressed.

$a^{X2}$ is preferably 2 or less, and more preferably 0, because the initial degradation of the light-emitting device according to the present embodiment is more suppressed.

$R^{X1}$, $R^{X2}$ and $R^{X3}$ are preferably an alkyl group, a cycloalkyl group, an aryl group, or a monovalent heterocyclic group, and more preferably an aryl group, and these groups each may have a substituent.

The arylene group represented by $Ar^{X1}$ and $Ar^{X3}$ is more preferably a group represented by formula (A-1) or (A-9), and still more preferably a group represented by formula (A-1), and these groups each may have a substituent.

The divalent heterocyclic group represented by $Ar^{X1}$ and $Ar^{X3}$ is more preferably a group represented by formula (AA-1), (AA-2), or (AA-7) to (AA-26), and these groups each may have a substituent.

$Ar^{X1}$ and $Ar^{X3}$ are preferably an arylene group which may have a substituent.

The arylene group represented by $Ar^{X2}$ and $Ar^{X4}$ is more preferably an arylene group represented by formula (A-1), (A-6), (A-7), (A-9) to (A-11), or (A-19), and these groups each may have a substituent.

The more preferable range of the divalent heterocyclic group represented by $Ar^{X2}$ and $Ar^{X4}$ is the same as the more preferable range of the divalent heterocyclic group represented by $Ar^{X1}$ and $Ar^{X3}$.

The more preferable range and still more preferable range of the arylene group and the divalent heterocyclic group in the divalent group represented by $Ar^{X2}$ and $Ar^{X4}$ in which at least one arylene group and at least one divalent heterocyclic group are directly bonded to each other are respectively the same as the more preferable range and still more preferable range of the arylene group and the divalent heterocyclic group represented by $Ar^{X1}$ and $Ar^{X3}$.

Examples of the divalent group represented by $Ar^{X2}$ and $Ar^{X4}$ in which at least one arylene group and at least one divalent heterocyclic group are directly bonded to each other include the same divalent groups represented by $Ar^{Y1}$ of formula (Y) in which at least one arylene group and at least one divalent heterocyclic group are directly bonded to each other.

$Ar^{X2}$ and $Ar^{X4}$ are preferably an arylene group which may have a substituent.

The optional substituent of the groups represented by $Ar^{X1}$ to $Ar^{X4}$ and $R^{X1}$ to $R^{X3}$ is preferably an alkyl group,

a cycloalkyl group, or an aryl group, and these groups each may further have a substituent.

The constitutional unit represented by formula (X) is preferably a constitutional unit represented by formulas (X-1) to (X-7), more preferably a constitutional unit represented by formulas (X-1) to (X-6), and still more preferably a constitutional unit represented by formulas (X-3) to (X-6).

[Chemical Formula 51]

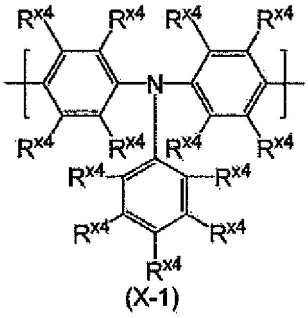

(X-1)

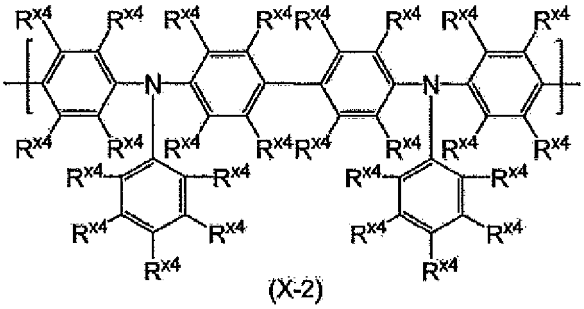

(X-2)

[Chemical Formula 52]

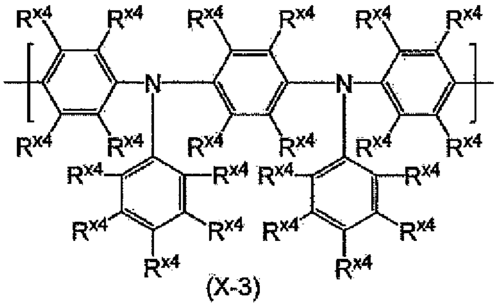

(X-3)

[Chemical Formula 53]

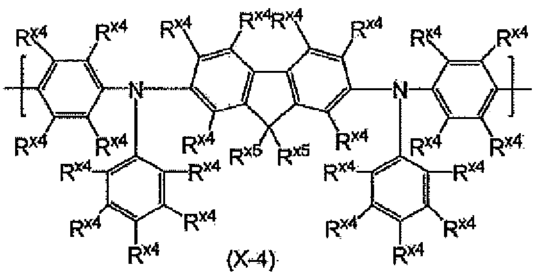

(X-4)

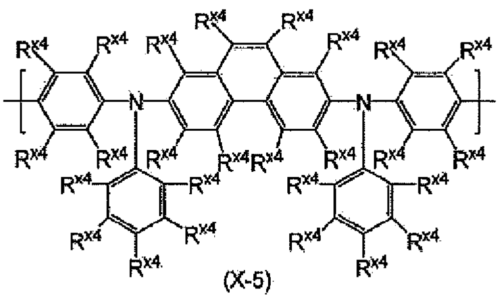

(X-5)

[Chemical Formula 54]

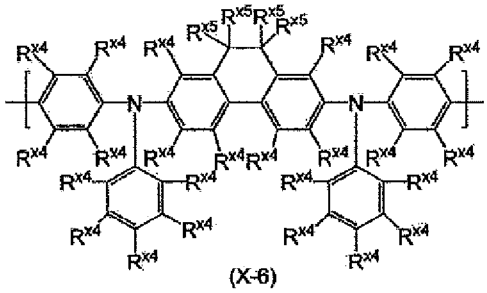
(X-6)

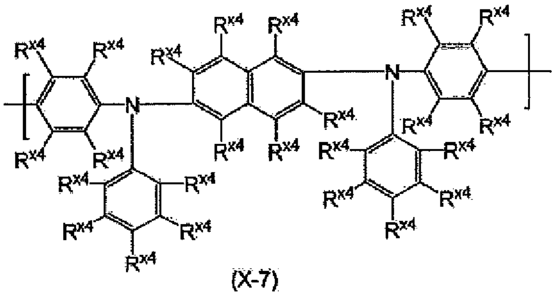
(X-7)

[In the formula, $R^{X4}$ and $R^{X5}$ each independently represent a hydrogen atom, an alkyl group, a cycloalkyl group, an alkoxy group, a cycloalkoxy group, an aryl group, an aryloxy group, a fluorine atom, a monovalent heterocyclic group, or a cyano group, and these groups each may have a substituent. The plurality of $R^{X4}$ may be the same or different. The plurality of $R^{X5}$ may be the same or different, and adjacent groups $R^{X5}$ may be bonded to each other to form a ring together with the carbon atom to which they are bonded.]

Examples of the constitutional unit represented by formula (X) include constitutional units represented by formulas (X1-1) to (X1-11). The constitutional unit represented by formula (X) is preferably a constitutional unit represented by formulas (X1-3) to (X1-10).

[0325] [Chemical Formula 55]

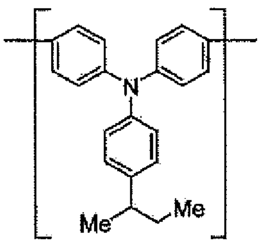
(X1-1)

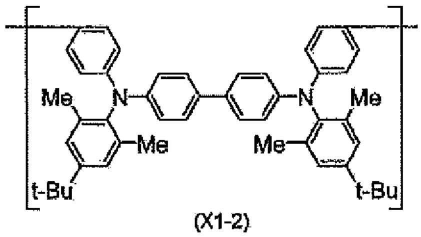
(X1-2)

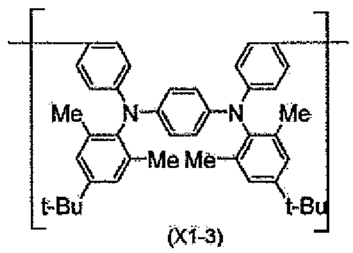
(X1-3)

[Chemical Formula 56]

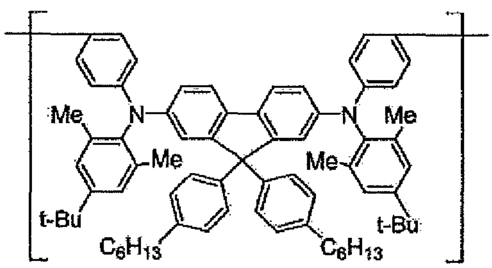
(X1-4)

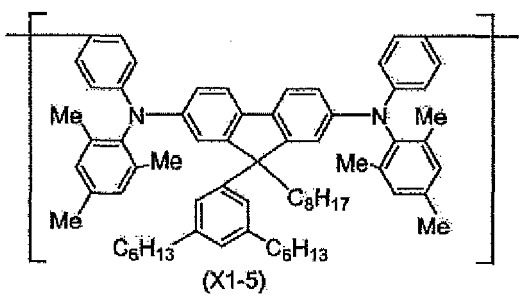
(X1-5)

[Chemical Formula 57]

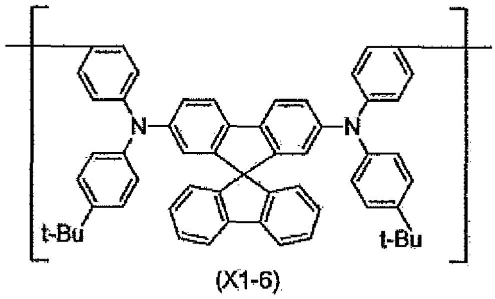

(X1-6)

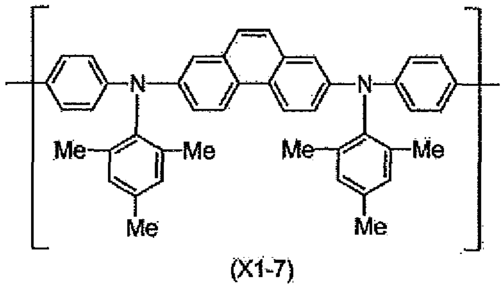

(X1-7)

[Chemical Formula 58]

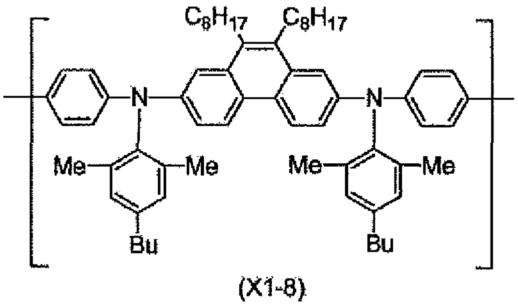

(X1-8)

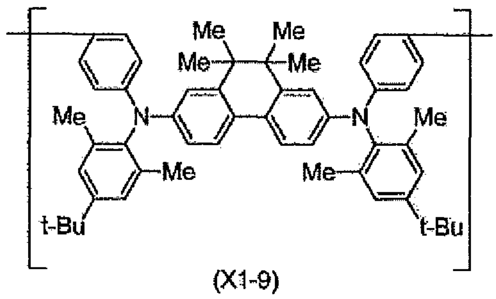

(X1-9)

[Chemical Formula 59]

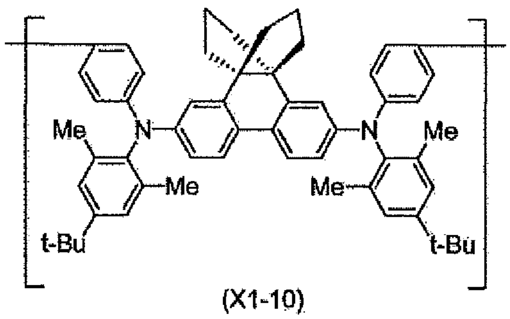

(X1-10)

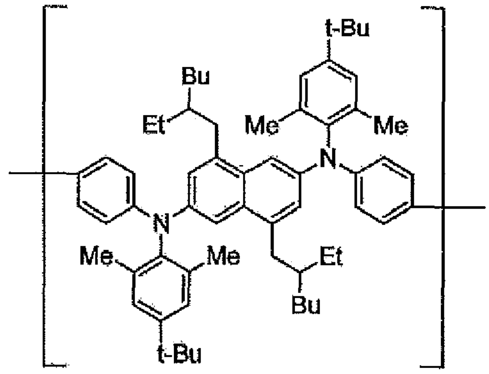

(X1-11)

In the polymer compound of the hole transporting layer, only one constitutional unit represented by formula (X) may be contained or two or more of them may be contained.

When the light-emitting device according to the present embodiment comprises a hole transporting layer obtained by using the polymer compound of the hole transporting layer, the polymer compound of the hole transporting layer may be contained in the hole transporting layer as it is, or in an intramolecularly, an intermolecularly, or an intramolecularly and intermolecularly crosslinked state (i.e., as a crosslinked product). The hole transporting layer is preferably a layer comprising a crosslinked product of the polymer compound of the hole transporting layer. The crosslinked product of the polymer compound of the hole transporting layer may be a product in which the polymer compound of the hole transporting layer and another material are intermolecularly crosslinked.

To achieve better hold transportability of the polymer compound of the hole transporting layer, the content of the constitutional unit represented by formula (X) is preferably 1 to 99 mol%, more preferably 10 to 80 mol%, and still more preferably 20 to 70 mol%, based on the total amount of constitutional units contained in the polymer compound of the hole transporting layer.

[Chemical Formula 60]

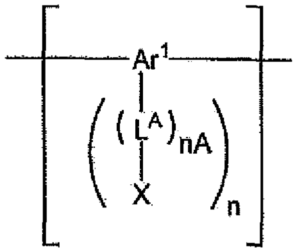

(3)

[In the formula,

nA represents an integer of 0 to 5, and n represents an integer of 1 to 4. $Ar^1$ represents an aromatic hydrocarbon group or a heterocyclic group, and these groups each may have a substituent.

$L^A$ represents an alkylene group, a cycloalkylene group, an arylene group, a divalent heterocyclic group, a group represented by -NR'-, an oxygen atom, or a sulfur atom, and these groups each may have a substituent. R' represents a hydrogen atom, an alkyl group, a cycloalkyl group, an aryl group, or a monovalent heterocyclic group, and these groups each may have a substituent. When there are a plurality of $L^A$, they may be the same or different.

X represents a crosslinkable group represented by any of the formulas (XL-1) to (XL-17). When there are a plurality of X, they may be the same or different.]

To achieve a better luminous efficiency of the light-emitting device according to the present embodiment, nA preferably represents an integer of 0 to 3, and more preferably an integer of 0 to 2.

To achieve a better luminous efficiency of the light-emitting device according to the present embodiment, n is preferably 1 or 2, and more preferably 2.

To achieve a better luminous efficiency of the light-emitting device according to the present embodiment, $Ar^1$ is preferably an aromatic hydrocarbon group that may have a substituent.

The aromatic hydrocarbon group represented by $Ar^1$ usually has 6 to 60 carbon atoms, preferably 6 to 30 carbon atoms, and more preferably 6 to 18 carbon atoms, not including the carbon atoms of the substituent.

The arylene group moiety excluding the n substituents of the aromatic hydrocarbon group represented by $Ar^1$ is preferably a group represented by formulas (A-1) to (A-20), more preferably a group represented by formula (A-1), (A-2), (A-6) to (A-10), (A-19), or (A-20), and still more preferably a group represented by formula (A-1), (A-2), (A-7), (A-9), or (A-19).

The heterocyclic group represented by $Ar^1$ usually has 2 to 60 carbon atoms, preferably 3 to 30 carbon atoms, and more preferably 4 to 18 carbon atoms, not including the carbon atoms of the substituent.

The divalent heterocyclic group moiety excluding the n substituents of the heterocyclic group represented by $Ar^1$ is preferably a group represented by formulas (AA-1) to (AA-34).

The aromatic hydrocarbon group and the heterocyclic group represented by $Ar^1$ may have a substituent. Examples of the substituent that the aromatic hydrocarbon group and the heterocyclic group may have include an alkyl group, a cycloalkyl group, an alkoxy group, a cycloalkoxy group, an aryl group, an aryloxy group, a fluorine atom, a monovalent heterocyclic group, or a cyano group.

The alkylene group represented by $L^A$ usually has 1 to 20 carbon atoms, preferably 1 to 15 carbon atoms, and more preferably 1 to 10 carbon atoms, not including the carbon atoms of the substituent. The cycloalkylene group represented by $L^A$ usually has 3 to 20 carbon atoms, not including the carbon atoms of the substituent.

The alkylene group and the cycloalkylene group each may have a substituent, and examples thereof include a methylene group, an ethylene group, a propylene group, a butylene group, a hexylene group, a cyclohexylene group, and an octylene group.

The alkylene group and the cycloalkylene group represented by $L^A$ each may have a substituent.

Examples of the optional substituents of the alkylene group and the cycloalkylene group include an alkyl group, a cycloalkyl group, an alkoxy group, a cycloalkoxy group, a fluorine atom, and a cyano group.

The arylene group represented by $L^A$ may have a substituent. The arylene group is preferably a phenylene group or a fluorenediyl group, and more preferably an m-phenylene group, a p-phenylene group, a fluorene-2,7-diyl group, or a fluorene-9,9-diyl group. Examples of the optional substituent of the arylene group include an alkyl group, a cycloalkyl group, an alkoxy group, a cycloalkoxy group, an aryl group, an aryloxy group, a monovalent heterocyclic group, a fluorine atom, a cyano group, and a crosslinkable group selected from the group of crosslinkable group A.

Preferable examples of the divalent heterocyclic group represented by $L^A$ are groups represented by formulas (AA-1) to (AA-34).

To facilitate synthesis of the polymer compound of the hole transporting layer, $L^A$ is preferably an arylene group or an alkylene group, and more preferably a phenylene group, a fluorenediyl group, or an alkylene group, and these groups each may have a substituent.

To achieve better crosslinkable properties of the polymer compound of the hole transporting layer, the crosslinkable group represented by X is preferably a crosslinkable group represented by formula (XL-1), (XL-3), (XL-7) to (XL-10), (XL-16), or (XL-17), more preferably a crosslinkable group represented by formula (XL-1), (XL-3), (XL-9,) (XL-16), or (XL-17), still more preferably a crosslinkable group represented by formula (XL-1), (XL-16), or (XL-17), and particularly preferably a crosslinkable group represented by formula (XL-1) or (XL-17).

To achieve better crosslinkable properties of the polymer compound of the hole transporting layer, the content of the constitutional unit represented by formula (3) is preferably 1 to 90 mol%, more preferably 3 to 75 mol%, still more preferably 5 to 60 mol%, based on the total amount of constitutional units contained in the polymer compound of the hole transporting layer.

Only one constitutional unit represented by formula (3) may be contained or two or more of them may be contained in the polymer compound of the hole transporting layer.

[Chemical Formula 61]

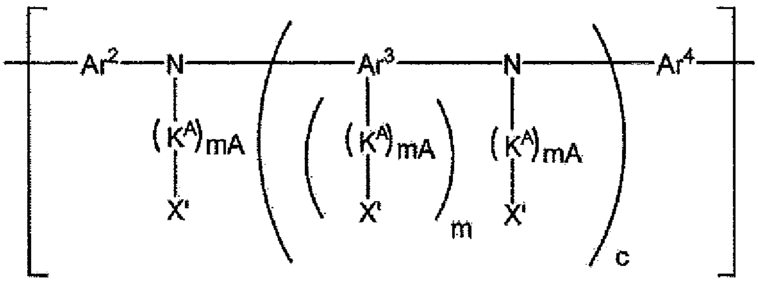

(4)

[In the formula,

mA represents an integer of 0 to 5, m represents an integer of 1 to 4, and c represents 0 or 1. When there are a plurality of mA, they may be the same or different.

$Ar^3$ represents an aromatic hydrocarbon group, a heterocyclic group, or a group in which at least one aromatic hydrocarbon ring and at least one heterocyclic ring are directly bonded to each other, and these groups each may have a substituent.

$Ar^2$ and $Ar^4$ each independently represent an arylene group or a divalent heterocyclic group, and these groups each may have a substituent.

$Ar^2$, $Ar^3$, and $Ar^4$ each may be bonded either directly or via an oxygen atom or a sulfur atom to a group other than the group bonded to the nitrogen atom to which that group is bonded to form a ring.

$K^A$ represents an alkylene group, a cycloalkylene group, an arylene group, a divalent heterocyclic group, a group represented by -NR"-, an oxygen atom, or a sulfur atom, and these groups each may have a substituent. R" represents a hydrogen atom, an alkyl group, a cycloalkyl group, an aryl group, or a monovalent heterocyclic group, and these groups each may have a substituent. When there are a plurality of $K^A$, they may be the same or different.

X' represents a crosslinkable group represented by any of the formulas (XL-1) to (XL-17), a hydrogen atom, an alkyl group, a cycloalkyl group, an aryl group, or a monovalent heterocyclic group, and these groups each may have a substituent. However, at least one X' is a crosslinkable group represented by any of the formulas (XL-1) to (XL-17).]

To achieve a better luminous efficiency of the light-emitting device according to the present embodiment, mA is preferably 0 to 2, more preferably 0 or 1, and still more preferably 0.

To achieve a better luminous efficiency of the light-emitting device according to the present embodiment, m is preferably 1 or 2, and more preferably 2.

To facilitate synthesis of the polymer compound of the hole transporting layer, and achieve a better luminous efficiency of the light-emitting device according to the present embodiment, c is preferably 0.

To achieve a better luminous efficiency of the light-emitting device according to the present embodiment, $Ar^3$ is preferably an aromatic hydrocarbon group which may have a substituent.

The definition and examples of the arylene group moiety excluding the m substituents of the aromatic hydrocarbon group represented by $Ar^3$ are the same as the definition and examples of the arylene group represented by $Ar^{X2}$ in the above-mentioned formula (X).

The definition and examples of the divalent heterocyclic group moiety excluding the m substituents of the heterocyclic group represented by $Ar^3$ are the same as the definition and examples of the divalent heterocyclic group moiety represented by $Ar^{X2}$ in the above-mentioned formula (X).

The definition and examples of the divalent group excluding the m substituents of the group represented by $Ar^3$ in which at least one aromatic hydrocarbon ring and at least one heterocyclic ring are directly bonded to each other are the same as the definition and examples of the divalent group represented by $Ar^{X2}$ in the above-mentioned formula (X) in which at least one arylene group and at least one divalent heterocyclic group are directly bonded to each other.

In $Ar^2$ and $Ar^4$, because the initial degradation of the light-emitting device according to the present embodiment is reduced, they are preferably arylene groups which may have a substituent.

The definition and examples of the arylene group represented by $Ar^2$ and $Ar^4$ are the same as the definition and examples of the arylene group represented by $Ar^{X1}$ and $Ar^{X3}$ in the above formula (X).

The definition and examples of the divalent heterocyclic group represented by $Ar^2$ and $Ar^4$ are the same as the definition and examples of the divalent heterocyclic group represented by $Ar^{X1}$ and $Ar^{X3}$ in the above formula (X).

The groups represented by $Ar^2$, $Ar^3$, and $Ar^4$ each may have a substituent. Preferable examples of the substituent include an alkyl group, a cycloalkyl group, an alkoxy group, a cycloalkoxy group, an aryl group, an aryloxy group, a fluorine atom, a monovalent heterocyclic group and a cyano group.

The definitions and examples of the alkylene group, the cycloalkylene group, the arylene group, and the divalent heterocyclic group represented by $K^A$ are each the same as the definitions and examples of the alkylene group, the cycloalkylene group, the arylene group, and the divalent heterocyclic group represented by $L^A$.

To facilitate the synthesis of the polymer compound of the hole transporting layer, $K^A$ is preferably a phenylene group or an alkylene group, and these groups each may have a substituent.

The definitions and examples of the crosslinkable group represented by X' are the same as the definition and examples of the crosslinkable group represented by X described above.

To achieve better crosslinkable properties of the polymer compound of the hole transporting layer, the content of the constitutional unit represented by formula (4) is preferably 1 to 90 mol%, more preferably 3 to 50 mol%, still more preferably 5 to 20 mol%, based on the total amount of constitutional units contained in the polymer compound of the hole transporting layer.

Only one constitutional unit represented by formula (4) may be contained or two or more of them may be contained in the polymer compound of the hole transporting layer.

Examples of constitutional unit represented by formula (3) include constitutional units represented by formulas (3-1) to (3-30), and examples of the constitutional unit represented by formula (4) include constitutional units represented by formulas (4-1) to (4-9). Among these, to achieve better crosslinkable properties of the polymer compound of the hole transporting layer, such constitutional unit is preferably a constitutional unit represented by formulas (3-1) to (3-30), more preferably a constitutional unit represented by formulas (3-1) to (3-15), (3-19), (3-20), (3-23), (3-25), or (3-30), still more preferably a constitutional unit represented by formulas (3-1) to (3-13) or (3-30), and particularly preferable a constitutional unit represented by formulas (3-1) to (3-9) or (3-30).

[Chemical Formula 62]

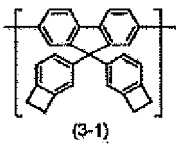
(3-1)

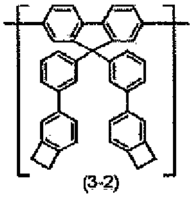
(3-2)

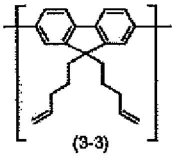
(3-3)

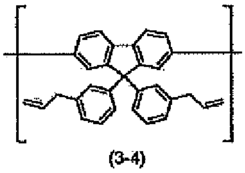
(3-4)

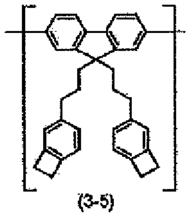
(3-5)

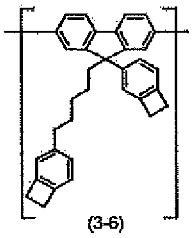
(3-6)

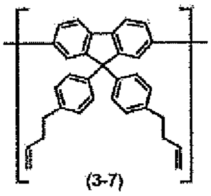
(3-7)

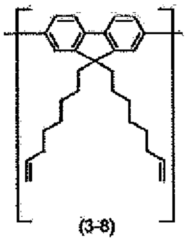
(3-8)

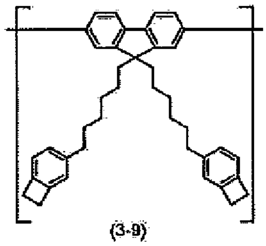
(3-9)

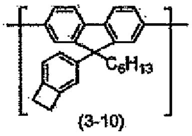
(3-10)

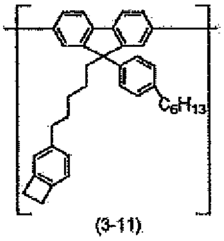
(3-11)

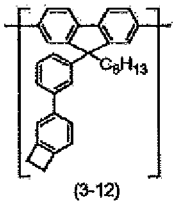
(3-12)

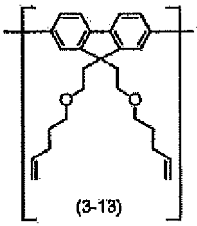
(3-13)

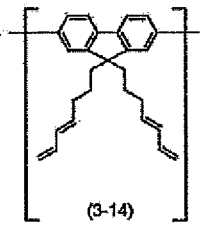
(3-14)

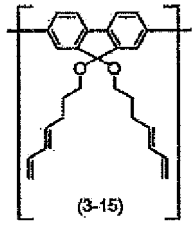
(3-15)

[Chemical Formula 63]

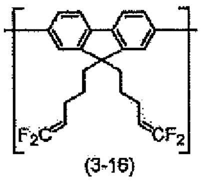
(3-16)

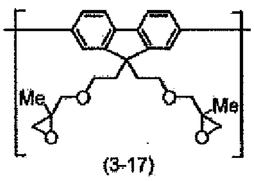
(3-17)

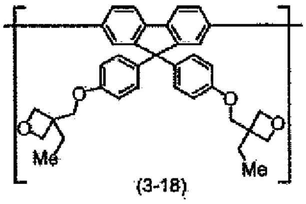
(3-18)

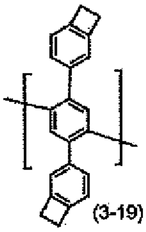
(3-19)

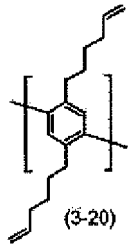
(3-20)

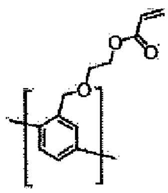
(3-21)

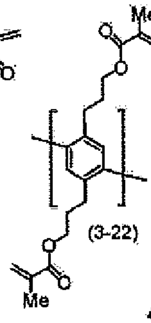
(3-22)

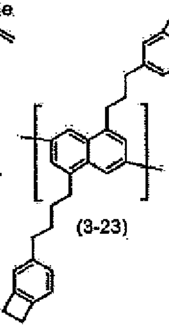
(3-23)

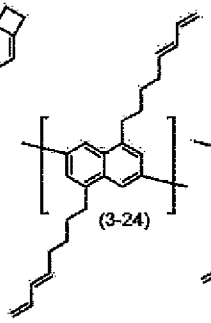
(3-24)

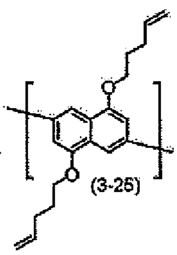
(3-25)

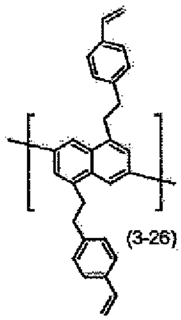
(3-26)

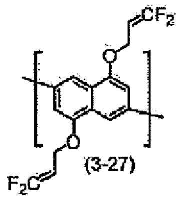
(3-27)

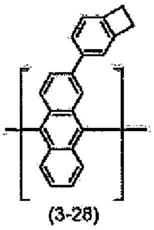
(3-28)

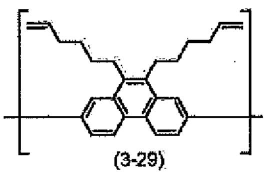
(3-29)

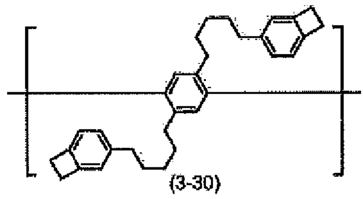
(3-30)

[Chemical Formula 64]

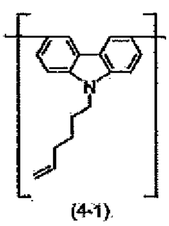
(4-1)
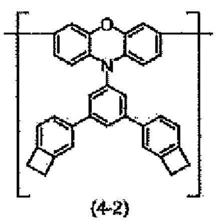
(4-2)
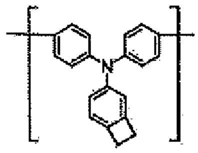
(4-3)
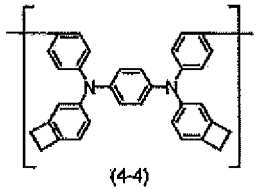
(4-4)
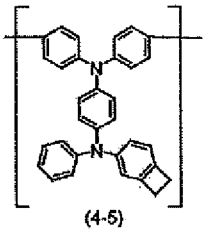
(4-5)
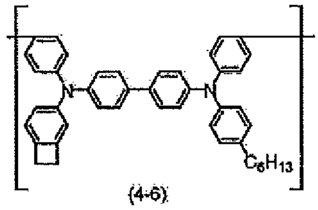
(4-6)
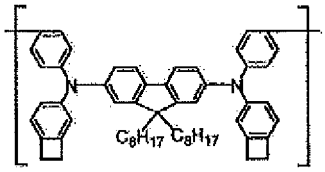
(4-7)
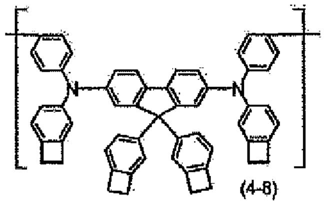
(4-8)
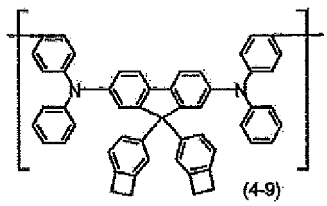
(4-9)

[Chemical Formula 65]

$$-[-Ar^{Y1}-]- \qquad (Y)$$

[In the formula, $Ar^{Y1}$ represents an arylene group, a divalent heterocyclic group, or a divalent group in which at least one arylene group and at least one divalent heterocyclic group are directly bonded to each other, and these groups each may have a substituent.]

The arylene group represented by $Ar^{Y1}$ is more preferably an arylene group represented by formula (A-1), (A-2), (A-6) to (A-10), (A-19), or (A-20), and still more preferably a group represented by formula (A-1), (A-2), (A-7), (A-9), or (A-19), and these groups each may have a substituent.

More preferably, the divalent heterocyclic group represented by $Ar^{Y1}$ is a group represented by formula (AA-1) to (AA-4), (AA-10) to (AA-15), (AA-18) to (AA-21), (AA-33), or (AA-34), and still more preferably a group represented by formula (AA-4), (AA-10), (AA-12), (AA-14), or (AA-33), and these groups each may have a substituent.

The more preferable range and still more preferable range of the arylene group and divalent heterocyclic group in the divalent group represented by $Ar^{Y1}$ in which at least one arylene group and at least one divalent heterocyclic group are directly bonded to each other are respectively the same as the more preferable range and still more preferable range of the arylene group and divalent heterocyclic group represented by $Ar^{Y1}$ described above.

Examples of the "divalent group in which at least one arylene group and at least one divalent heterocyclic group are directly bonded to each other" include groups represented by the following formulas, and these groups each may have a substituent.

[Chemical Formula 66]

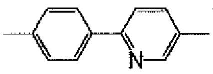
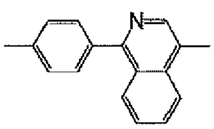
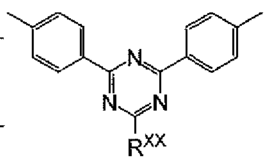
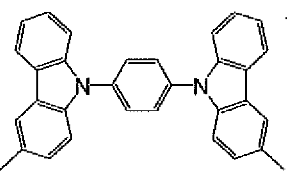
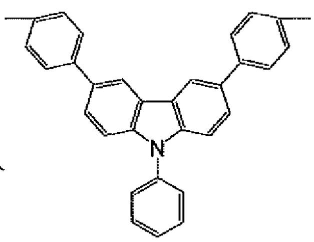

[In the formula, $R^{XX}$ represents a hydrogen atom, an alkyl group, a cycloalkyl group, an aryl group, or a monovalent heterocyclic group, and these groups each may have a substituent.]

$R^{XX}$ is preferably an alkyl group, a cycloalkyl group, or an aryl group, and these groups each may have a substituent.

The optional substituent of the group represented by $Ar^{Y1}$ is preferably an alkyl group, a cycloalkyl group, or an aryl group, and these groups each may further have a substituent.

Examples of the constitutional unit represented by formula (Y) include the constitutional units represented by formulas (Y-1) to (Y-10). From the viewpoint of the initial degradation of the light-emitting device according to the present embodiment, the constitutional unit is preferably a constitutional unit represented by formulas (Y-1) to (Y-3). From the viewpoint of hole transportability, the constitutional unit is preferably a constitutional unit represented by formulas (Y-8) to (Y-10).

[Chemical Formula 67]

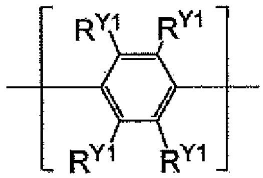

(Y-1)

[In the formula, $R^{Y1}$ represents a hydrogen atom, an alkyl group, a cycloalkyl group, an alkoxy group, a cycloalkoxy group, an aryl group, or a monovalent heterocyclic group, and these groups each may have a substituent. The plurality of $R^{Y1}$ may be the same or different and adjacent groups $R^{Y1}$ may be bonded to each other to form a ring together with the carbon atom to which they are bonded.]

$R^{Y1}$ is preferably a hydrogen atom, an alkyl group, a cycloalkyl group, or an aryl group, and these groups each may have a substituent.

The constitutional unit represented by formula (Y-1) is preferably a constitutional unit represented by formula (Y-1').

[Chemical Formula 68]

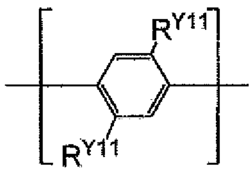

(Y-1')

[In the formula, $R^{Y11}$ represents an alkyl group, a cycloalkyl group, an alkoxy group, a cycloalkoxy group, an aryl group, or a monovalent heterocyclic group, and these groups each may have a substituent; and the plurality of $R^{Y11}$ may be the same or different.]

$R^{Y11}$ is preferably an alkyl group, a cycloalkyl group, or an aryl group, and more preferably an alkyl group or a cycloalkyl group, and these groups each may have a substituent.

[Chemical Formula 69]

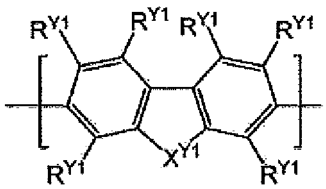

(Y-2)

[In the formula, $R^{Y1}$ has the same meaning as described above. $X^{Y1}$ represents a group represented by $-C(R^{Y2})_2-$, $-C(R^{Y2})=C(R^{Y2})-$, or $-C(R^{Y2})_2-C(R^{Y2})_2-$. $R^{Y2}$ represents a hydrogen atom, an alkyl group, a cycloalkyl group, an alkoxy group, a cycloalkoxy group, an aryl group, or a monovalent heterocyclic group, and these groups each may have a substituent. The plurality of $R^{Y2}$ may be the same or different, and $R^{Y2}$ may be bonded to another $R^{Y2}$ to form a ring

together with the carbon atom to which they are bonded.]

$R^{Y2}$ is preferably an alkyl group, a cycloalkyl group, an aryl group, or a monovalent heterocyclic group, and more preferably an alkyl group, a cycloalkyl group, or an aryl group, and these groups each may have a substituent.

In $X^{Y1}$, the combination of the two $R^{Y2}$ groups in the group represented by $-C(R^{Y2})_2-$ is preferably a combination in which both groups are alkyl groups or cycloalkyl groups, a combination in which both groups are aryl groups, a combination in which both groups are monovalent heterocyclic groups, or a combination in which one group is an alkyl group or a cycloalkyl group and the other group is an aryl group or a monovalent heterocyclic group, and more preferably is a combination in which one group is an alkyl group or a cycloalkyl group and the other group is an aryl group, and these groups each may have a substituent. Two present $R^{Y2}$ groups may be bonded to each other to form a ring together with the atoms to which they are bonded. When groups $R^{Y2}$ forms a ring, the group represented by $-C(R^{Y2})_2-$ is preferably a group represented by formulas (Y-A1) to (Y-A5), and more preferably a group represented by formula (Y-A4), and these groups each may have a substituent.

[Chemical Formula 70]

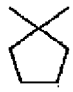
(Y-A1)

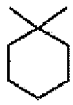
(Y-A2)

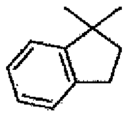
(Y-A3)

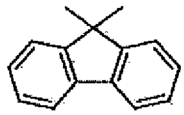
(Y-A4)

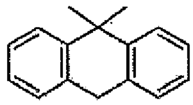
(Y-A5)

In $X^{Y1}$, the combination of the two $R^{Y2}$ groups in the group represented by $-C(R^{Y2})=C(R^{Y2})-$ is preferably a combination in which both groups are alkyl groups or cycloalkyl groups or a combination in which one group is an alkyl group or a cycloalkyl group and the other group is an aryl group, and these groups each may have a substituent.

In $X^{Y1}$, the four $R^{Y2}$ in the group represented by $-C(R^{Y2})_2-C(R^{Y2})_2-$ are preferably an optionally substituted alkyl group or cycloalkyl group. The plurality of $R^{Y2}$ may be bonded to each other to form a ring together with the atoms to which they are bonded, and when groups $R^{Y2}$ forms a ring, a group represented by $-C(R^{Y2})_2-C(R^{Y2})_2-$ is preferably a group represented by formulas (Y-B1) to (Y-B5), and more preferably a group represented by formula (Y-B3), and these groups each may have a substituent.

[Chemical Formula 71]

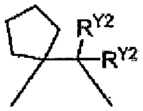
(Y-B1)

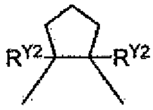
(Y-B2)

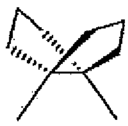
(Y-B3)

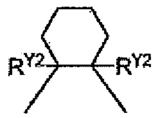
(Y-B4)

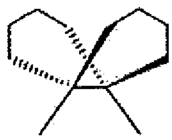
(Y-B5)

[In the formula, $R^{Y2}$ has the same meaning as described above.]

The constitutional unit represented by formula (Y-2) is preferably a constitutional unit represented by formula (Y-2').

[Chemical Formula 72]

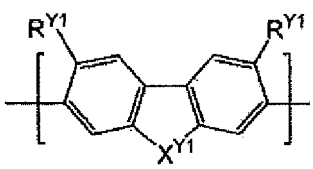
(Y-2')

[In the formula, $R^{Y1}$ and $X^{Y1}$ have the same meanings as described above.]

[Chemical Formula 73]

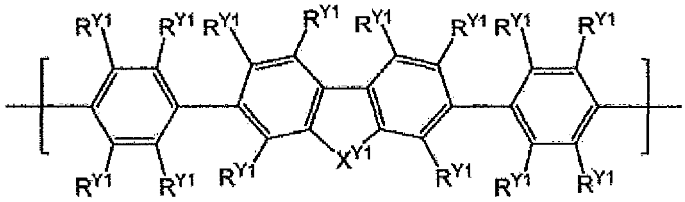

(Y-3)

[In the formula, $R^{Y1}$ and $X^{Y1}$ have the same meanings as described above.]

The constitutional unit represented by formula (Y-3) is preferably a constitutional unit represented by formula (Y-3').

[Chemical Formula 74]

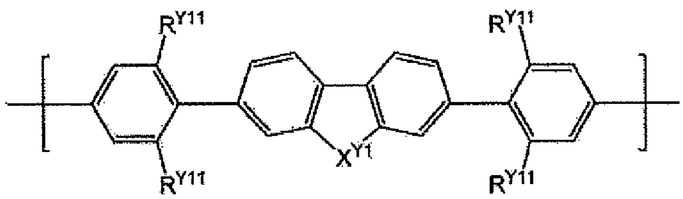

(Y-3')

[In the formula, $R^{Y11}$ and $X^{Y1}$ have the same meanings as described above.]

[Chemical Formula 75]

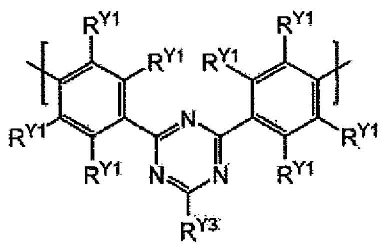

(Y-4)

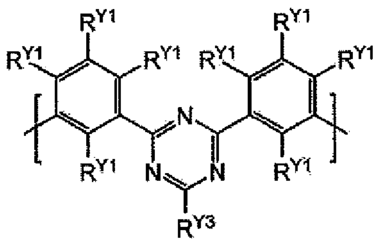

(Y-5)

[Chemical Formula 76]

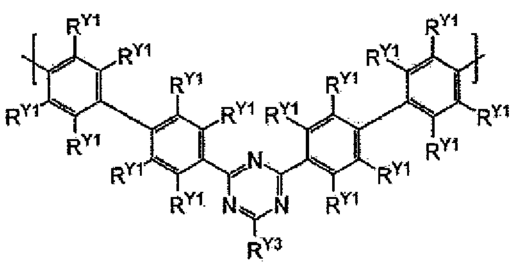

(Y-6)

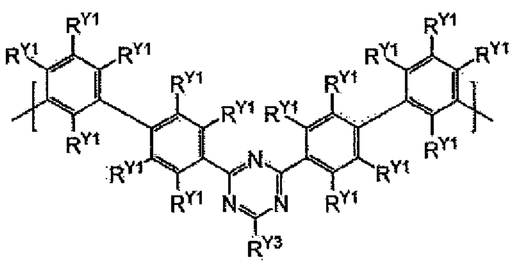

(Y-7)

[In the formula, $R^{Y1}$ represents the same meaning as described above. $R^{Y3}$ represents a hydrogen atom, an alkyl group, a cycloalkyl group, an alkoxy group, a cycloalkoxy group, an aryl group, or a monovalent heterocyclic group, and these groups each may have a substituent.]

$R^{Y3}$ is preferably an alkyl group, a cycloalkyl group, an alkoxy group, a cycloalkoxy group, an aryl group, or a monovalent heterocyclic group, and more preferably an aryl group, and these groups each may have a substituent.

The constitutional unit represented by formula (Y-4) is preferably a constitutional unit represented by formula (Y-4'), and the constitutional unit represented by formula (Y-6) is preferably a constitutional unit represented by formula (Y-6').

[Chemical Formula 77]

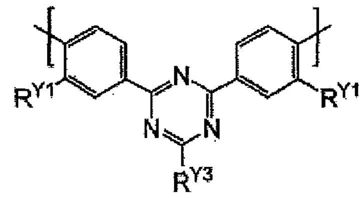

(Y-4')

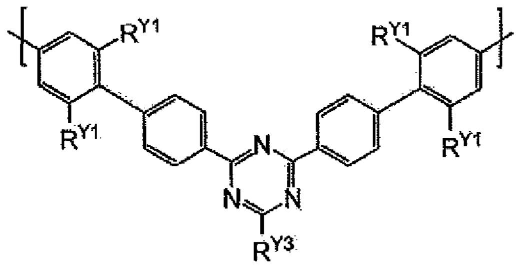

(Y-6')

[In the formula, $R^{Y1}$ and $R^{Y3}$ represent the same meanings as described above.]

[Chemical Formula 78]

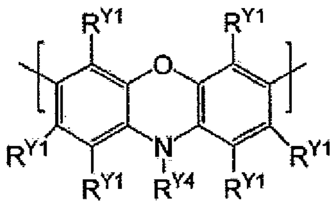

(Y-8)

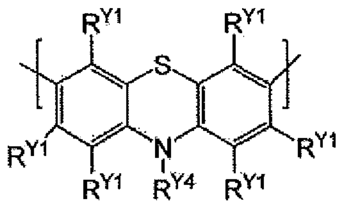

(Y-9)

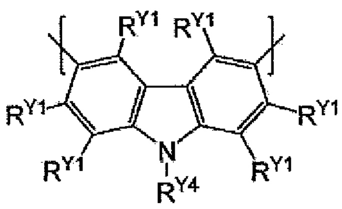

(Y-10)

[In the formula, $R^{Y1}$ represents the same meaning as described above. $R^{Y4}$ represents a hydrogen atom, an alkyl group, a cycloalkyl group, an alkoxy group, a cycloalkoxy group, an aryl group, or a monovalent heterocyclic group, and these groups each may have a substituent.]

$R^{Y4}$ is preferably an alkyl group, a cycloalkyl group, an alkoxy group, a cycloalkoxy group, an aryl group, or a monovalent heterocyclic group, and more preferably an aryl group. These groups each may have a substituent.

Examples of the constitutional unit represented by formula (Y) include constitutional units composed of arylene groups represented by formulas (Y-101) to (Y-121), constitutional units composed of divalent heterocyclic groups represented by formulas (Y-201) to (Y-206), and constitutional units composed of a divalent group represented by formulas (Y-300) to (Y-304) in which at least one arylene group and at least one divalent heterocyclic group are directly bonded to each other, and preferably constitutional units composed of arylene groups represented by formulas (Y-101) to (Y-121), constitutional units composed of divalent heterocyclic groups represented by formulas (Y-201) to (Y-206), and constitutional units composed of a divalent group represented by formulas (Y-301) to (Y-304) in which at least one arylene group and at least one divalent heterocyclic group are directly bonded to each other.

[Chemical Formula 79]

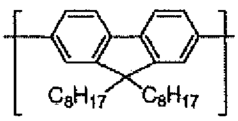
(Y-101)

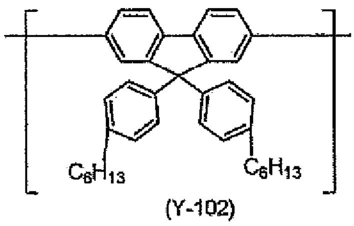
(Y-102)

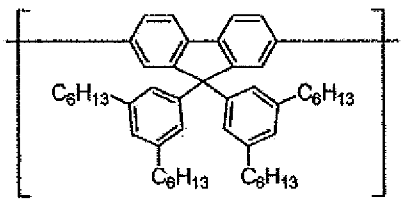
(Y-103)

[Chemical Formula 80]

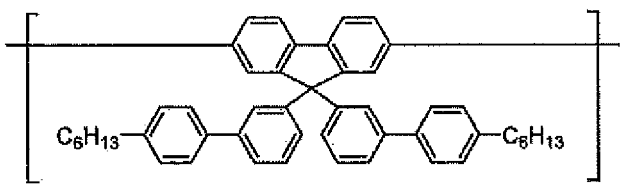
(Y-104)

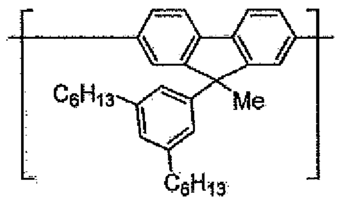
(Y-105)

[Chemical Formula 81]

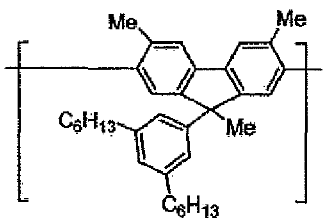
(Y-106)

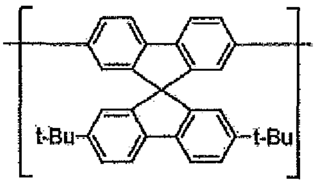
(Y-107)

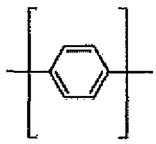
(Y-108)

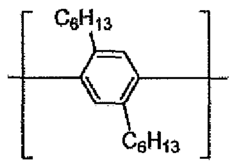
(Y-109)

[Chemical Formula 82]

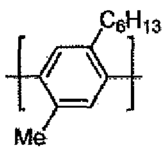
(Y-110)

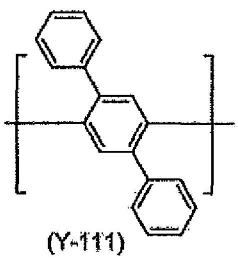
(Y-111)

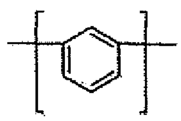
(Y-112)

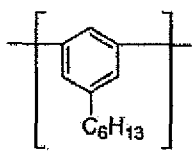
(Y-113)

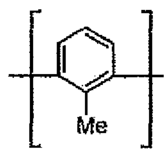
(Y-114)

[Chemical Formula 83]

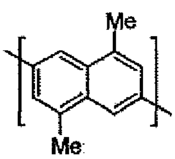
(Y-115)

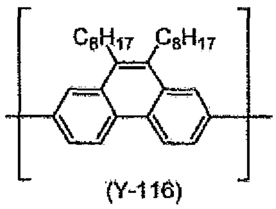
(Y-116)

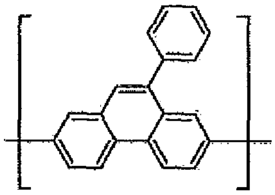
(Y-117)

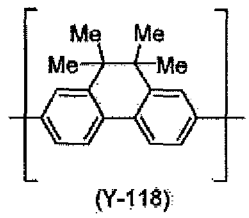
(Y-118)

[Chemical Formula 84]

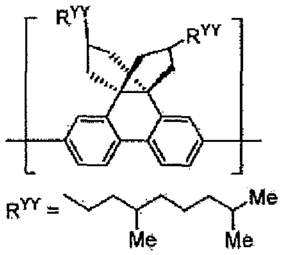
(Y-119)

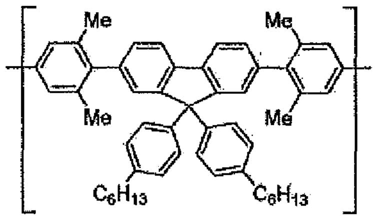
(Y-120)

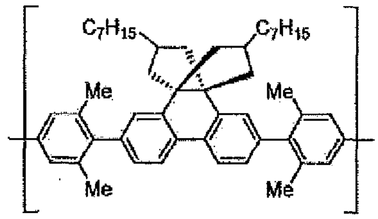
(Y-121)

[Chemical Formula 85]

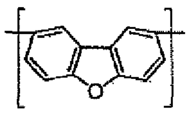
(Y-201)

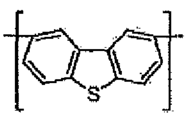
(Y-202)

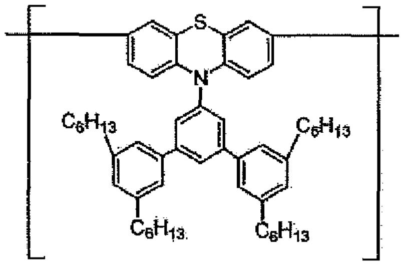
(Y-203)

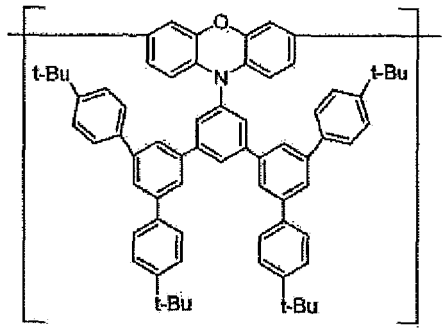
(Y-204)

[Chemical Formula 86]

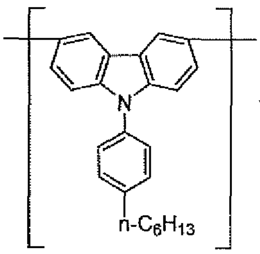
(Y-205)

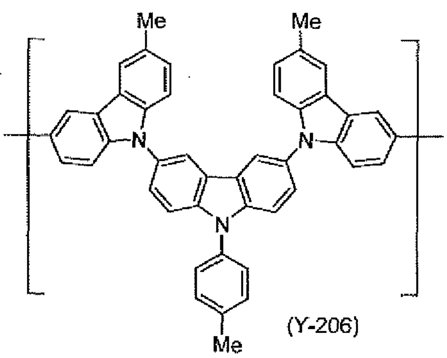
(Y-206)

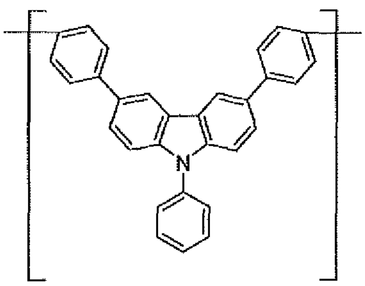
(Y-300)

[Chemical Formula 87]

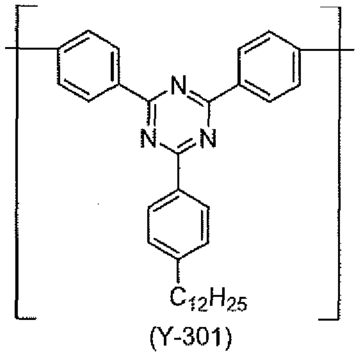
(Y-301)

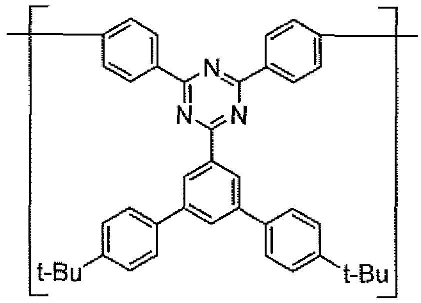
(Y-302)

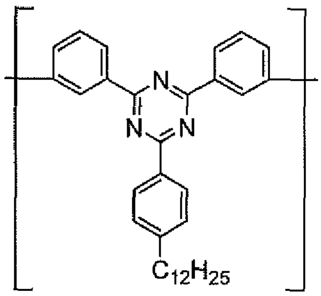
(Y-303)

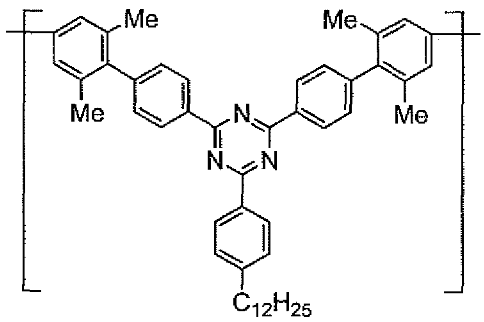
(Y-304)

A constitutional unit represented by formula (Y), wherein $Ar^{Y1}$ is an arylene group, is preferably 0.5 to 90 mol%, and more preferably 30 to 80 mol% with respect to the total amount of the constitutional units contained in the high molecular weight compound in the hole transporting layer because the initial degradation of the light-emitting device according to the present embodiment is more suppressed.

To achieve better hole transportability of the high molecular weight compound in the hole transport layer, the content of the constitutional unit represented by formula (Y) in which $Ar^{Y1}$ is a divalent heterocyclic group or a divalent group in which at least one arylene group and at least one divalent heterocyclic group are directly bonded to each other is preferably 0.5 to 40 mol%, and more preferably 3 to 30 mol%, based on the total amount of constitutional units contained in the high molecular weight compound in the hole transport layer.

Only one constitutional unit represented by formula (Y) may be contained or two or more of them may be contained in the high molecular weight compound in the hole transport layer.

When the light-emitting device according to the present embodiment comprises an electron transporting layer, the electron transporting material contained in the electron transporting layer is preferably a polymer compound (hereinafter also referred to as "polymer compound of the electron transporting layer") comprising at least one constitutional unit selected from the group consisting of a constitutional unit represented by formula (ET-1) and a constitutional unit represented by formula (ET-2).

[Chemical Formula 88]

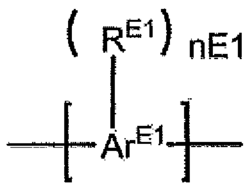
(ET-1)

[In the formula,

nE1 represents an integer of 1 or more,
$Ar^{E1}$ represents an aromatic hydrocarbon group or a heterocyclic group, and these groups each may have a substituent other than $R^{E1}$.

$R^{E1}$ represents a group represented by formula (ES-1). When there are a plurality of $R^{E1}$, they may be the same or different.]

$$-R^{E3}-\{(Q^{E1})_{nE3}-Y^{E1}(M^{E1})_{aE1}(Z^{E1})_{bE1}\}_{mE1} \quad (ES-1)$$

[In the formula,

nE3 represents an integer of 0 or more, aE1 represents an integer of 1 or more, bE1 represents an integer of 0 or more, and mE1 represents an integer of 1 or more. When there are a plurality of nE3, aE1, and bE1, they may be the same or different at each occurrence. When $R^{E3}$ is a single bond, mE1 is 1. aE1 and bE1 are selected so that the charge of the group represented by formula (ES-1) is zero.

$R^{E3}$ represents a single bond, a hydrocarbon group, a heterocyclic group or -O-$R^{E3'}$ ($R^{E3'}$ represents a hydrocarbon group or a heterocyclic group), and these groups each may have a substituent.

$Q^{E1}$ represents an alkylene group, a cycloalkylene group, an arylene group, an oxygen atom, or a sulfur atom, and these groups each may have a substituent. When there are a plurality of $Q^{E1}$, they may be the same or different.

$Y^{E1}$ represents $-CO_2^-$, $-SO_3^-$, $-SO_2^-$, or $-PO_3^{2-}$. When there are a plurality of $Y^{E1}$, they may be the same or different.

$M^{E1}$ represents an alkali metal cation, an alkali earth metal cation, or an ammonium cation, and the ammonium cation may have a substituent. When there are a plurality of $M^{E1}$, they may be the same or different. $Z^{E1}$ represents $F^-$, $OH^-$ $B(R^{E4})_4^-$, $R^{E4}SO_3^-$, $R^{E4}COO^-$, $NO_3^-$, $SO_4^{2-}$, $HSO_4^-$, $PO_4^{3-}$, $HPO_4^{2-}$, $H_2PO_4^-$, $BF4^-$, or $PF_6^-$. $R^{E4}$ represents an alkyl group, a cycloalkyl group, or an aryl group, and these groups each may have a substituent. When there are a plurality of $Z^{E1}$, they may be the same or different.]

nE1 is usually an integer of 1 to 4, and preferably 1 or 2.

Preferable examples of the aromatic hydrocarbon group or heterocyclic group represented by $Ar^{E1}$ are a group remaining after removing from a 1,4-phenylene group, a 1,3-phenylene group, a 1,2-phenylene group, a 2,6-naphthalenediyl group, a 1,4-naphthalenediyl group, a 2,7-fluorenediyl group, a 3,6-fluorenediyl group, a 2,7-phenanthrenediyl group, or a 2,7-carbazolediyl group, nE1 atoms of hydrogen directly bonded to the atoms constituting the ring, and $Ar^{E1}$ may have a substituent other than $R^{E1}$.

Examples of the optional substituent other than $R^{E1}$ of $Ar^{E1}$ include a fluorine atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group, a cycloalkynyl group, a carboxyl group, and a group represented by formula (ES-3).

$$-O-(C_{n'}H_{2'}O)_{nx}-C_{m'}H_{2m'+1} \quad (ES-3)$$

[In the formula, n', m', and nx each independently represent an integer of 1 or more.]

nE3 is usually an integer of 0 to 10, preferably an integer of 0 to 8, and more preferably an integer of 0 to 2.

aE1 is usually an integer of 1 to 10, preferably an integer of 1 to 5, and more preferably 1 or 2.

bE1 is usually an integer of 0 to 10, preferably an integer of 0 to 4, and more preferably 0 or 1.

mE1 is usually an integer of 1 to 5, preferably 1 or 2, and more preferably 1.

When $R^{E3}$ is -O-$R^{E3'}$, the group represented by formula (ES-1) is a group represented by the following formula.

$$-O-R^{E3'}-\{(Q^{E1})_{nE3}-Y^{E1}(M^{E1})_{aE1}(Z^{E1})_{bE1}\}_{mE1}$$

$R^{E3}$ is preferably a hydrocarbon group or a heterocyclic group, more preferably an aromatic hydrocarbon group or an aromatic heterocyclic group, and still more preferably an aromatic hydrocarbon group.

Examples of the optional substituent of $R^{E3}$ include an alkyl group, a cycloalkyl group, an aryl group, a monovalent heterocyclic group, and a group represented by formula (ES-3), and a group represented by formula (ES-3) is preferable.

$Q^{E1}$ is preferably an alkylene group, an arylene group, or an oxygen atom, and more preferably an alkylene group or an oxygen atom.

$Y^{E1}$ is preferably $-CO_2^-$, $-SO_2^-$, or $PO_3^{2-}$, and more preferably $-CO_2^-$.

Examples of the alkali metal cation represented by $M^{E1}$ include $Li^+$, $Na^+$, $K^+$, $Rb^+$, and $Cs^+$, and $K^+$, $Rb^+$, or $Cs^+$ is preferable and $Cs^+$ is more preferable.

Examples of the alkali earth metal cation represented by $M^{E1}$ include $Be^{2+}$, $Mg^{2+}$, $Ca^{2+}$, $Sr^{2+}$, $Ba^{2+}$, and $Mg^{2+}$,

$Ca^{2+}$, $Sr^{2+}$, and $Ba^{2+}$ are preferable, and $Ba^{2+}$ is more preferable.

Preferable examples of $M^{E1}$ include an alkali metal cation or an alkali earth metal cation, and an alkali metal cation is more preferable.

$Z^{E1}$ is preferably $F^-$, $OH^-$, $B(R^{E4})_4^-$, $R^{E4}SO_3^-$, $R^{E4}COO^-$, or $NO_3^-$, and more preferably $F^-$, $OH^-$, $R^{E4}SO_3^-$, or $R^{E4}COO^-$. $R^{E4}$ is preferably an alkyl group.

Examples of the group represented by formula (ES-1) include groups represented by the following formulas.

[Chemical Formula 89]

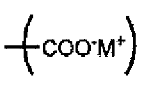
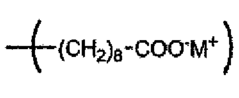
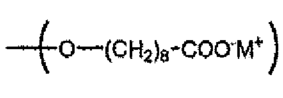
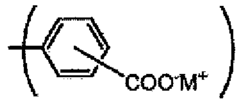
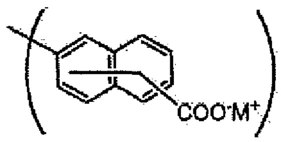
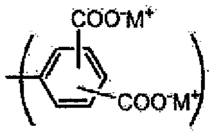
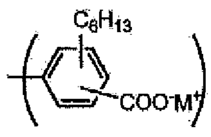
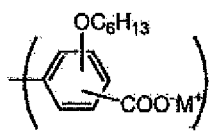

[Chemical Formula 90]

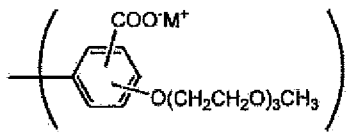
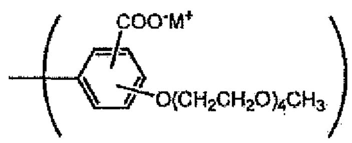
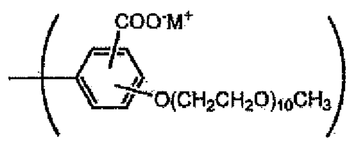
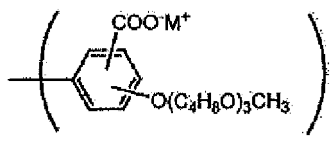
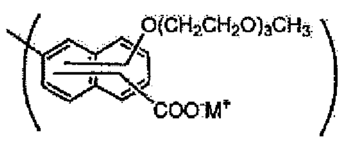
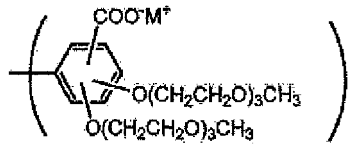
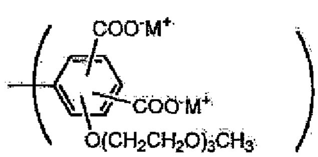
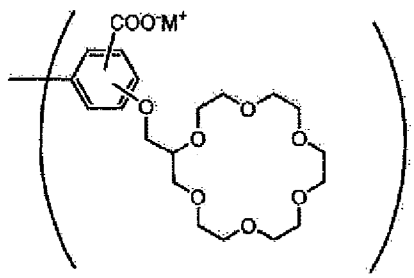

[In the formula, $M^+$ represents $Li^+$, $Na^+$, $K^+$, $Cs^+$, or $N(CH^3)_4^+$. When there are a plurality of $M^+$, they may be the same or different.]

[Chemical Formula 91]

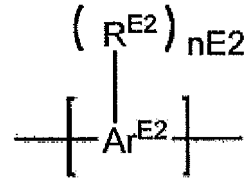
(ET-2)

[In the formula,

nE2 represents an integer of 1 or more,

$Ar^{E2}$ represents an aromatic hydrocarbon group or a heterocyclic group, and these groups each may have a substituent other than $R^{E2}$, and $R^{E2}$ represents a group represented by formula (ES-2); when there are a plurality of $R^{E2}$, they may be the same or different.]

$$-R^{E5}-\{(Q^{E2})_{nE4}-Y^{E2}(M^{E2})_{aE2}(Z^{E2})_{bE2}\}_{mE2} \quad (ES-2)$$

[In the formula,

nE4 represents an integer of 0 or more, aE2 represents an integer of 1 or more, bE2 represents an integer of 0 or more, and mE2 represents an integer of 1 or more. When there are a plurality of nE4, aE2, and bE2, they may be the same or different at each occurrence. When $R^{E5}$ is a single bond, mE2 is 1. aE2 and bE2 are selected so that the charge of the group represented by formula (ES-2) is zero.

$R^{E5}$ represents a single bond, a hydrocarbon group, a heterocyclic group or -O-$R^{E5'}$ ($R^{E5'}$ represents a hydrocarbon group or a heterocyclic group), and these groups each may have a substituent.

$Q^{E2}$ represents an alkylene group, a cycloalkylene group, an arylene group, an oxygen atom, or a sulfur atom, and these groups each may have a substituent. When there are a plurality of $Q^{E2}$, they may be the same or different.

$Y^{E2}$ represents $-C^+R^{E6}_2$, $-N^+R^{E6}_3$, $-P^+R^{E6}_3$, $-S^+R^{E6}_2$, or $-I^+R^{E6}_2$. $R^{E6}$ represents a hydrogen atom, an alkyl group, a cycloalkyl group, or an aryl group, and these groups each may have a substituent. The plurality of $R^{E6}$ may be the same or different. When there are a plurality of $Y^{E2}$, they may be the same or different.

$M^{E2}$ represents $F^-$, $OH^-$, $B(R^{E7})_4^-$, $R^{E7}SO_3^-$, $R^{E7}COO^-$, $BF_4^-$, or $SbF_6^-$. $R^{E7}$ represents an alkyl group, a cycloalkyl group, or an aryl group, and these groups each may have a substituent. When there are a plurality of $M^{E2}$, they may be the same or different.

$Z^{E2}$ represents an alkali metal cation or an alkali earth metal cation. When there are a plurality of $Z^{E2}$, they may be the same or different.]

nE2 is usually an integer of 1 to 4, and preferably 1 or 2.

Preferable examples of the aromatic hydrocarbon group or heterocyclic group represented by $Ar^{E2}$ are a group remaining after removing from a 1,4-phenylene group, a 1,3-phenylene group, a 1,2-phenylene group, a 2,6-naphthalenediyl group, a 1,4-naphthalenediyl group, a 2,7-fluorenediyl group, a 3,6-fluorenediyl group, a 2,7-phenanthrenediyl group, or a 2,7-carbazolediyl group, nE2 atoms of hydrogen directly bonded to the atoms constituting the ring, and $Ar^{E2}$ may have a substituent other than $R^{E2}$.

The examples of the optional substituent other than $R^{E2}$ of $Ar^{E2}$ are the same as the examples of the optional substituent other than $R^{E1}$ of $Ar^{E1}$.

nE4 is usually an integer of 0 to 10, preferably an integer of 0 to 8, and more preferably an integer of 0 to 2.

aE2 is usually an integer of 1 to 10, preferably an integer of 1 to 5, and more preferably 1 or 2.

bE2 is usually an integer of 0 to 10, preferably an integer of 0 to 4, and more preferably 0 or 1.

mE2 is usually an integer of 1 to 5, preferably 1 or 2, and more preferably 1.

When $R^{E5}$ is -O-$R^{E5'}$, the group represented by formula (ES-2) is a group represented by the following formula.

$$-O-R^{E5'}-\{(Q^{E1})_{nE3}-Y^{E1}(M^{E1})_{aE1}(Z^{E1})_{bE1}\}_{mE1}$$

$R^{E5}$ is preferably a hydrocarbon group or a heterocyclic group, more preferably an aromatic hydrocarbon group or an aromatic heterocyclic group, and still more preferably an aromatic hydrocarbon group.

Examples of the optional substituent of $R^{E5}$ include an alkyl group, a cycloalkyl group, an aryl group, a monovalent heterocyclic group, and a group represented by formula (ES-3), and a group represented by formula (ES-3) is preferable.

$Q^{E2}$ is preferably an alkylene group, an arylene group, or an oxygen atom, and more preferably an alkylene group or an oxygen atom.

$Y^{E2}$ is preferably $-C^+R^{E6}_2$, $\sim N^+R^{E6}_3$, $-P^+R^{E6}_3$, or $-S^+R^{E6}_2$, and more preferably $-N^+R^{E6}_3$. $R^{E6}$ is preferably a hydrogen atom, an alkyl group, or an aryl group, and more preferably a hydrogen atom or an alkyl group.

$M^{E2}$ is preferably $F^-$, $B(R^{E7})_4^-$, $R^{E7}SO_3^-$, $R^{E7}COO^-$, $BF_4^-$, or $SbF_6^-$, and more preferably $B(R^{E7})_4^-$, $R^{E7}COO^-$, or $SbF_6^-$. $R^{E7}$ is preferably an alkyl group.

Examples of the alkali metal cation represented by $Z^{E2}$ include $Li^+$, $Na^+$, $K^+$, $Rb^+$, and $Cs^+$, and $Li^+$, $Na^+$, or $K^+$ is preferable.

Examples of the alkali earth metal cation represented by $Z^{E2}$ include $Be^{2+}$, $Mg^{2+}$, $Ca^{2+}$, $Sr^{2+}$, and $Ba^{2+}$, and $Mg^{2+}$ or $Ca^{2+}$ is more preferable.

$Z^{E2}$ is preferably an alkali metal cation.

Examples of the group represented by formula (ES-2) include groups represented by the following formulas.

[Chemical Formula 92]

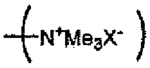
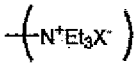
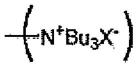
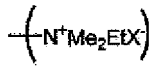
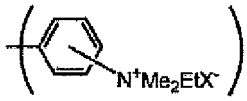
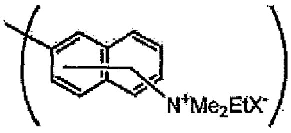
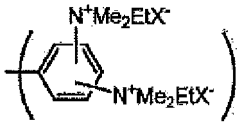
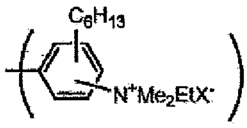
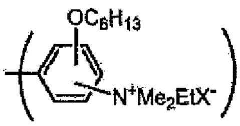

[Chemical Formula 93]

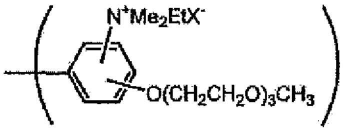
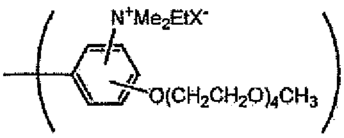
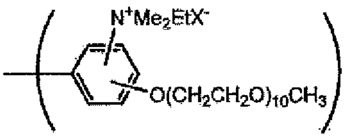
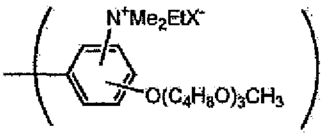
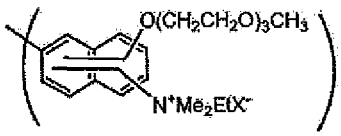
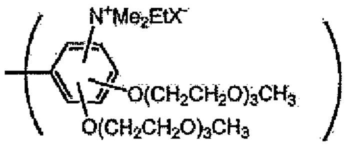
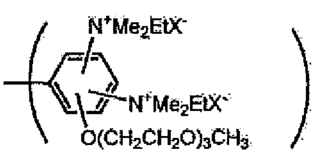
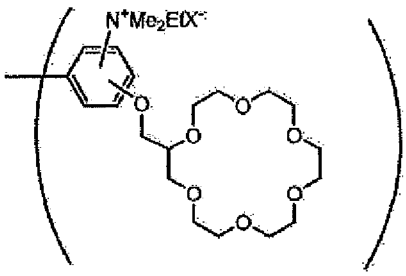

[In the formula, $X^-$ represents $F^-$, $B(C_6H_5)_4^-$, $CH_3COO^-$, or $CF_3SO_3^-$. When there are a plurality of $X^-$, they may be the same or different.]

Examples of the constitutional unit represented by formulas (ET-1) and (ET-2) include the constitutional units represented by the following formulas (ET-31) to (ET-38).

[Chemical Formula 94]

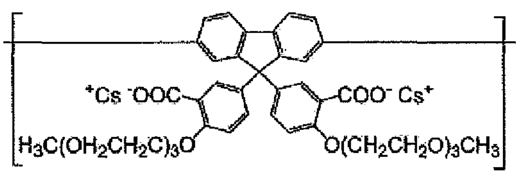
(ET-31)

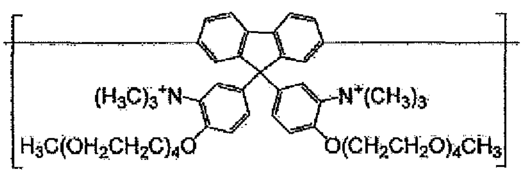
(ET-32)

[Chemical Formula 95]

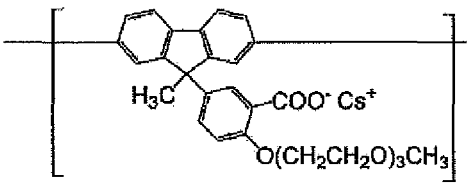

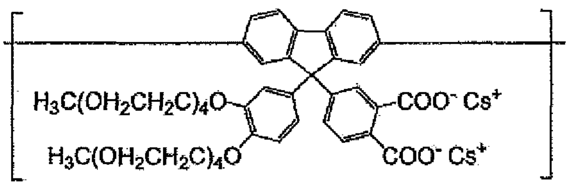

(ET-33) (ET-34)

[Chemical Formula 96]

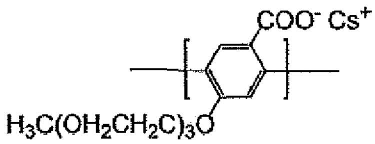

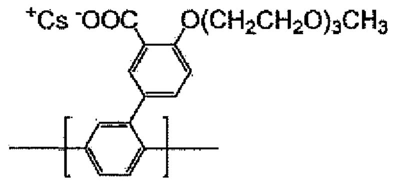

(ET-35) (ET-36)

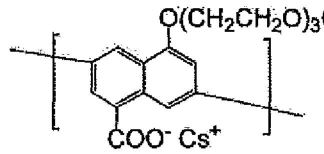

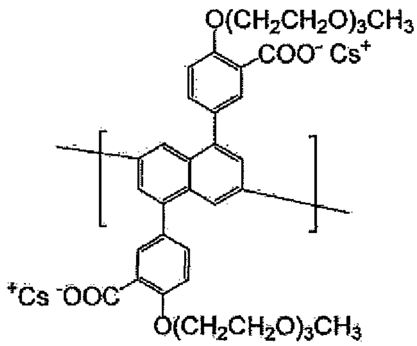

(ET-37) (ET-38)

The polymer compound of the electron transporting layer can be synthesized according to the methods described in, for example, Japanese Unexamined Patent Publication No. 2009-239279, Japanese Unexamined Patent Publication No. 2012-033845, Japanese Unexamined Patent Publication No. 2012-216821, Japanese Unexamined Patent Publication No. 2012-216822, and Japanese Unexamined Patent Publication No. 2012-216815.

The material of the hole transporting layer, the material of the electron transporting layer, and the material of the light-emitting layer preferably have a crosslinkable group to avoid the dissolution of the materials into solvents used in the formation of layers respectively adjacent to the hole transporting layer, the electron transporting layer, and the light-emitting layer in the fabrication of the light-emitting device when they are dissolved in the solvents. After each layer has been formed using a material having a crosslinkable group, the layer can be insolubilized by cross-linking the crosslinkable group.

Examples of methods for forming the light-emitting layer, the hole transporting layer, the electron transporting layer, the hole injecting layer, the electron injecting layers, and the like in the light-emitting device according to the present embodiment include vacuum deposition from powder and methods involving the film formation from a solution or molten state when a low molecular weight compound is used and the film formation from a solution or molten state when a high molecular weight compound is used.

The order, the number, and the thickness of forming layers are adjusted according to the external quantum efficiency and the luminescence lifetime.

[Substrate/Electrodes]

The substrate in the light-emitting device may be any material that is capable of forming an electrode and that does not chemically change when forming an organic layer. The substrate may be made of a material such as glass,

plastic, and silicon. In the case of an opaque substrate, it is preferable that the electrode farthest from the substrate be transparent or translucent.

Examples of the material of the anode include conductive metal oxides and translucent metals. The anode material is preferably indium oxide, zinc oxide, or tin oxide; a conductive compound such as indium tin oxide (ITO) or indium zinc oxide; a complex of silver, palladium, and copper (APC); NESA, gold, platinum, silver, or copper.

Examples of the material of the cathode include metals such as lithium, sodium, potassium, rubidium, cesium, beryllium, magnesium, calcium, strontium, barium, aluminum, zinc, and indium; alloys of two or more of those metals; alloys of one or more of those metals with one or more of silver, copper, manganese, titanium, cobalt, nickel, tungsten, and tin; and graphite and graphite intercalation compounds. Examples of the alloys include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.

Each of the anode and the cathode may have a stacked structure of two or more layers.

[Applications]

To obtain planar light emission using a light-emitting device, a planar anode and a planar cathode may be arranged so as to overlap each other. Examples of methods for obtaining patterned light emission include a method in which a mask having a patterned window is disposed on the surface of a planar light-emitting device, a method in which a layer that is not intended to emit light is formed very thickly so as to substantially prevent light emission, and a method in which the anode or the cathode, or both electrodes, are formed in a pattern shape. By forming a pattern by any of these methods and arranging such that several electrodes can be independently switched ON/OFF, a segment type display device capable of displaying numerals, letters, and the like is obtained. To obtain a dot matrix display device, the anodes and the cathodes are formed in a striped shape so as to be orthogonal to each other. Partial color display and multi-color display can be achieved by a method in which a plurality of polymer compounds having different emission colors are used for separate paining or a method in which a color filter or a fluorescence conversion filter are used. A dot matrix display device can be driven passively, or can be driven actively in combination with TFT and the like. These display devices can be used as a display in computers, television sets, mobile terminals, and the like. A planar light-emitting device can be suitably used as a planar light source for a backlight in a liquid crystal display or as a planar illumination light source. When a flexible substrate is used, the light-emitting device can also be used as a curved light source and a curved display.

One preferable embodiment of the present invention has so far been described, but the present invention is not limited to the embodiment described above.

For example, one aspect of the present invention may relate to a composition in which a phosphorescent compound represented by formula (1) at a residual chlorine concentration 15 ppm by mass or less is blended with a host material. The residual chlorine concentration of the phosphorescent compound is equal to the amount ($C_1$) of chlorine atoms contained in the phosphorescent compound represented by formula (1).

In one embodiment, the composition may one that satisfies the following formula (i), wherein $C_1$ (ppm) is the residual chlorine concentration of the phosphorescent compound and $W_1$ is the ratio (mass ratio) of the amount of the phosphorescent compound contained to the total amount of solid contents blended in the composition.

$$C_1 \times W_1 \leq 3.5 \text{ (ii)}$$

Moreover, in one embodiment, the composition may be the one that satisfies the following formula (ii), wherein $C_2$ (ppm by mass) is the residual chlorine concentration of the host material and $W_2$ is the ratio (mass ratio) of the amount of the host material contained with respect to the total amount of solid contents blended in the composition.

$$C_1 \times W_1 + C_2 \times W_2 \leq 3.5 \text{ (ii)}$$

Moreover, one aspect of the present invention may relate to a method for purifying a composition comprising the steps of preparing a crude product of a phosphorescent compound represented by formula (1) with a residual chlorine concentration higher than 15 ppm by mass, obtaining a purified product of the phosphorescent compound with a residual chlorine concentration 15 ppm by mass or less from the crude product, and obtaining a composition in which the purified product is blended with a host material.

## Examples

The present invention will now be described in more detail by the following Examples, but the present invention is not limited to these Examples.

In the Examples, the polystyrene-equivalent number-average molecular weight (Mn) and the polystyrene-equivalent weight-average molecular weight (Mw) of the polymer compounds were determined by the below size-exclusion chromatography (SEC) columns using tetrahydrofuran for the mobile phase. The SEC measurement conditions were as follows.

The polymer compound to be measured was dissolved at a concentration of about 0.05% by mass in tetrahydrofuran, and 10 μL of the solution was injected into the SEC column. The mobile phase flow rate was 2.0 mL/min. The column used was PLgel MIXED-B (a product manufactured by Polymer Laboratories Ltd.) was used. The detector used was a UV-VIS detector (a product manufactured by Shimadzu Corporation, trade name: SPD-10Avp)

LC-MS was measured by the following method.

The measurement sample was dissolved in chloroform or tetrahydrofuran so as to have a concentration of about 2 mg/mL, and about 1 μL was injected into an LC-MS (trade name: 1100 LC-MSD, manufactured by Agilent). The LC-MS mobile phase was flowed at a rate of 0.2 mL/min while varying the ratio of acetonitrile and tetrahydrofuran. An L-column 2 ODS (3 μm) (manufactured by the Chemicals Evaluation and Research Institute, inner diameter: 2.1 mm, length: 100 mm, particle diameter 3 μm) was used for the column.

TLC-MS was measured by the following method.

The measurement sample was dissolved in a solvent of either toluene, tetrahydrofuran, or chloroform at an arbitrary concentration, and the solution was coated on a TLC plate for DART (trade name: YSK5-100, manufactured by Techno Applications), and then measurement was carried out using TLC-MS (trade name: JMS-T100TD (The AccuTOF TLC), manufactured by JEOL Ltd.). The helium gas temperature during the measurement was adjusted in the range of 200 to 400°C.

NMR was measured by the following method.

A measurement sample of 5 to 10 mg was dissolved in about 0.5 mL of deuterated chloroform ($CDCl_3$), heavy tetrahydrofuran, heavy dimethyl sulfoxide, heavy acetone, heavy N,N-dimethylformamide, heavy toluene, heavy methanol, heavy ethanol, heavy 2-propanol, or heavy methylene chloride, and measured using an NMR apparatus (trade name: INOVA 300 or MERCURY 400 VX, manufactured by Agilent).

As an index of the purity of the compound, the value of the high-performance liquid chromatography (HPLC) area percentage was used. Unless noted otherwise, this value is the value at UV=254 nm in the HPLC apparatus (product name: LC-20A, manufactured by Shimadzu Corporation). At this time, the compound to be measured was dissolved in tetrahydrofuran or chloroform so as to have a concentration of 0.01 to 0.2% by mass, and 1 to 10 μL was injected into the HPLC apparatus in accordance with the concentration. For the HPLC mobile phase, the ratio of acetonitrile/tetrahydrofuran was varied between 100/0 to 0/100 (volume ratio) while flowing at a flow rate of 1.0 mL/min. As the column, a Kaseisorb LC ODS 2000 (manufactured by Tokyo Chemical Industry) or an ODS column having equivalent performance was used. For the detector, a photodiode array detector (trade name: SPD-M20A, manufactured by Shimadzu Corporation) was used.

In this Example, the maximum peak wavelength of the emission spectrum of the phosphorescent compound was measured at room temperature by a spectrophotometer (FP-6500, manufactured by JASCO Corporation). A xylene solution in which the phosphorescent compound was dissolved in xylene at a concentration of about $0.8 \times 10^{-4}$ % by mass was used as the sample. For the excitation light, UV light with a wavelength of 325 nm was used.

The amounts of bromine and chlorine atoms contained in phosphorescent compounds and host materials were measured by automatic combustion-ion chromatography. In this measurement, combustion decomposition was performed using the automatic sample combustion apparatus Model AQF-2100H manufactured by Mitsubishi Chemical Analytech Co., Ltd. and the following chromatography was performed using the ion chromatography system ICS-2100 manufactured by Thermo Fisher Scientific K.K.

<Synthesis Example 1> Synthesis of Compound M1, Compound M2, and Compound M3

Compound M1 was synthesized according to the method described in International Publication No. WO 2015/145871.

Compound M2 was synthesized according to the method described in International Publication No. WO 2013/146806.

Compound M3 was synthesized according to the method described in International Publication No. WO 2005/049546.

[Chemical Formula 97]

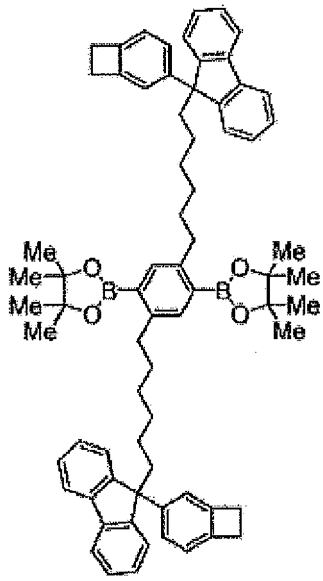

Compound M1

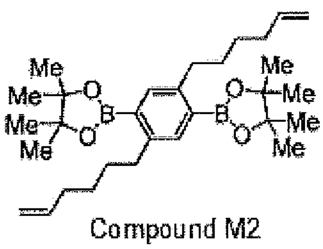

Compound M2

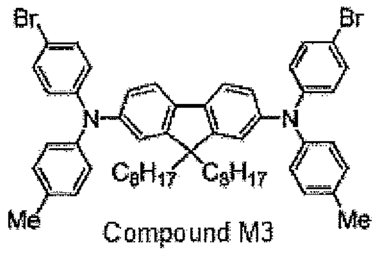

Compound M3

<Synthesis Example 2> Synthesis of Polymer Compound HTL-1

(Step 1) The atmosphere within a reaction vessel was replaced with inert gas, then the reaction vessel was charged with Compound M1 (0.923 g), Compound M2 (0.0496 g), Compound M3 (0.917 g), dichlorobis(tris-o-methoxyphenylphosphine)palladium (1.76 mg), and toluene (34 ml), and the mixture was heated to 105°C.

(Step 2) A 20% by mass solution of aqueous tetraethylammonium hydroxide (6.7 mL) was added dropwise to the reaction solution, and the mixture was refluxed for 6 hours.

(Step 3) After the reaction, phenylboronic acid (48.8 mg), dichlorobis(tris-o-methoxyphenylphosphine)palladium (0.88 mg) were added, and the mixture was refluxed for 14.5 hours.

(Step 4) Then, an aqueous sodium diethyldithiocarbamate solution was added, and the mixture was stirred at 80°C for 2 hours. The reaction solution was cooled, then washed twice with water, twice with 3% by mass aqueous acetic acid solution, and twice with water, and the resultant solution was dropped into methanol, which caused a precipitate to form. The resultant precipitate was dissolved in toluene and purified by passing through an alumina column and a silica gel column in that order. The obtained solution was dropped into methanol, and the mixture was stirred, and then the obtained precipitate was collected by filtration and dried to obtain 1.23 g of the polymer compound HTL-1.

The polystyrene-equivalent number-average molecular weight of the polymer compound HTL-1 was $2.3 \times 10^4$, and the polystyrene-equivalent weight-average molecular weight was $1.2 \times 10^5$.

The polymer compound HTL-1 was a copolymer having, based on the theoretical values obtained from the amounts of the charged raw materials, a molar ratio among the constitutional unit derived from compound M1, the constitutional unit derived from compound M2, and the constitutional unit derived from compound M3 of 45:5:50.

<Synthesis Example 3> Synthesis of Compound M4 and Compound M5

Compound M4 was synthesized according to the method described in Japanese Unexamined Patent Publication 2012-33845.

Compound M5 was synthesized according to the method described in Japanese Unexamined Patent Publication 2010-189630.

[Chemical Formula 98]

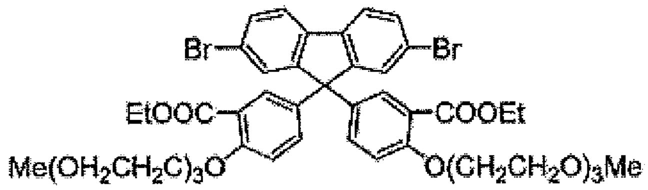

Compound M4

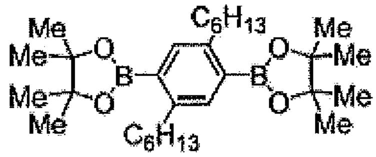

Compound M5

<Synthesis Example 4> Synthesis of Polymer Compound ET1

(Step 1) The atmosphere within a reaction vessel was replaced with inert gas, then the reaction vessel was charged with Compound M4 (9.23 g), Compound M5 (4.58 g), dichloro-bis[tris-o-methoxyphenylphosphine] palladium (8.6 mg), methyltrioctylammonium chloride (product manufactured by Sigma-Aldrich Co. LLC., trade name Aliquat336 (R)) (0.098 g), and toluene (175 mL), and the mixture was heated to 105°C.

(Step 2) Then, a 12% by mass aqueous solution of sodium carbonate (40.3 mL) was added dropwise thereto and the mixture was refluxed for 29 hours.

(Step 3) Then, phenylboronic acid (0.47 g) and dichloro-bis[tris-o-methoxyphenylphosphine] palladium (8.7 mg) were added and the mixture was refluxed for 14 hours.

(Step 4) Then, an aqueous solution of sodium diethyldithiocarbamate was added thereto and the mixture was stirred at 80°C for 2 hours. The obtained reaction solution was cooled and then the resultant solution was dropped into methanol, which caused a precipitate to form. The precipitate was collected by filtration, washed with methanol and water, and then dried to obtain a solid, which was dissolved in chloroform to obtain a solution. The solution was purified by passing through an alumina column and a silica gel column pretreated with chloroform in that order. The obtained purified solution was added dropwise to methanol and the mixture was stirred, which caused a precipitate to form. The precipitate was collected by filtration and dried to obtain Polymer Compound ET1a (7.15 g). The Mn and Mw of Polymer Compound ET1a were found to be $3.2 \times 10^4$ and $6.0 \times 10^4$, respectively.

Polymer Compound ET1a is a copolymer composed of a constitutional unit derived from Compound M4 and a constitutional unit derived from Compound M5 at a molar ratio of 50:50 based on the theoretical values obtained from the amounts of the charged raw materials.

(Step 5) The atmosphere within a reaction vessel was replaced with argon gas, then the reaction vessel was charged with Polymer Compound ET1a (3.1 g), tetrahydrofuran (130 mL), methanol (66 mL), cesium hydroxide monohydrate (2.1 g) and water (12.5 mL), and the mixture was stirred at 60°C for 3 hours.

(Step 6) Then, methanol (220 mL) was added thereto and the mixture was stirred for 2 hours. The obtained reaction mixture was concentrated, then isopropyl alcohol was added dropwise, and the mixture was stirred, which caused a precipitate to form. The precipitate was collected by filtration and dried to obtain Polymer Compound ET1 (3.5 g). $^1$H-NMR analysis of Polymer Compound ET1 indicated that the signal derived from the ethyl ester site in Polymer Compound ET1 had disappeared and confirmed that the reaction had been completed.

Polymer Compound ET1 is a copolymer composed of a constitutional unit represented by the following formula and a constitutional unit derived from Compound M5 at a molar ratio of 50:50 based on the theoretical values obtained from the amounts of the charged raw materials of Polymer Compound ET1a.

[Chemical Formula 99]

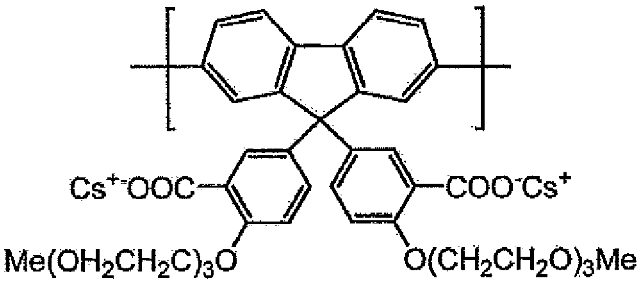

The values obtained by the elementary analysis of Polymer Compound ET1 were C, 54.1% by mass; H, 5.6% by mass; N, < 0.3% by mass; Cs, 22.7% by mass (theoretical values: C, 57.29% by mass; H, 5.70% by mass; Cs, 21.49% by mass; O, 15.52% by mass).

<Synthesis Example 5> Synthesis of Compound HM-1

[Chemical Formula 100]

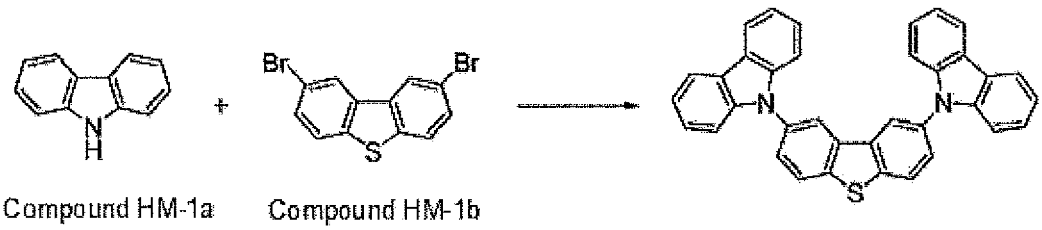

Compound HM-1a Compound HM-1b

Low Molecular Weight Compound HM-1

The atmosphere within a reaction vessel was replaced with nitrogen gas, then the reaction vessel was charged with Compound HM-1a (324 g), Compound HM-1b (300 g), xylene (12L), palladium acetate (II) (11.5 g), tri-tert-butylphosphonium tetrafluoroborate (29.8 g), and sodium tert-butoxide (555 g) and the mixture was stirred with heating to reflux for 40 hours. Then, the obtained reaction solution was filtered with a filter made of layers of silica gel and celite and the filter made of layers of silica gel and celite was further washed with toluene (10 L). The obtained filtrate was washed with ion exchanged water (4 L) five times and then the obtained organic layer was dried over anhydrous sodium sulfate and filtered. The obtained filtrate was concentrated under reduced pressure to obtain a solid. The obtained solid was recrystallized in toluene and then dried under reduced pressure at 50°C to obtain the crude product HM-1 (361 g). The HPLC area percent of the crude product HM-1 was 99.5% or more.

The obtained crude product HM-1 (86 g) was purified by submission five times to obtain Compound HM-1 (21 g). In the purification by sublimation, the degree of vacuum was $5 \times 10^{-3}$ Pa and the sublimation temperature was 290°C.

The HPLC area percent of Compound HM-1 was 99.5% or more. Moreover, the amount ($C^H$) of chlorine atoms contained in Compound HM-1 was below the detection limit (0 ppm by mass). Moreover, the amount of bromine atoms contained in Compound HM-1 was below the detection limit (0 ppm by mass). Compound HM-1 was used as a host material.

<Comparative Example 1 and Example 1> Synthesis of Phosphorescent compounds MC1 and MC2

[Chemical Formula 101]

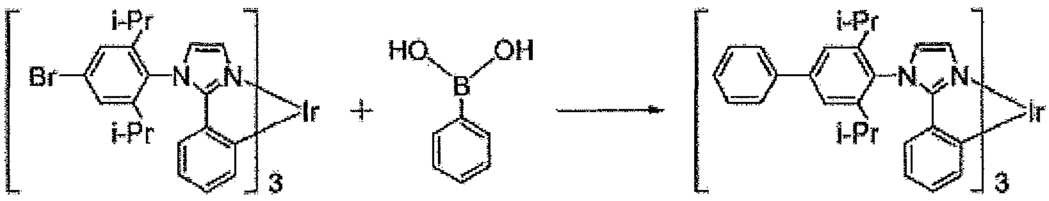

Metal Complex MC1a

Metal Complex MC1 and MC2

The atmosphere within a lightproof reaction vessel was replaced with argon gas, then the reaction vessel was charged with the phosphorescent compound MC1a (210 g), phenylboronic acid (63.1 g), dichloro-bis[tris-o-methoxyphenylphosphine] palladium (II) (0.69 g), and toluene (2.1 kg) and the mixture was heated to 70°C. A 20% by mass aqueous solution of tetraethylammonium hydroxide (1.39 kg) was added thereto and then the mixture was stirred at 90°C for 19 hours. Then, the reaction solution was cooled to room temperature, and then 10% by mass brine was added and filtered with a filter made of a layer of celite. The obtained filtrate was extracted with toluene and 10% by mass brine to obtain an organic layer. The obtained organic layer was dried over anhydrous magnesium sulfate and then filtered with a filter made of a layer of amino silica gel. The obtained filtrate was concentrated under reduced pressure to obtain a solid. The obtained solid was recrystallized in a mixed solvent of toluene and acetonitrile and then filtered to obtain the filtrate MC1' and the residue MC2'. The obtained filtrate MC1' was concentrated under reduced pressure to obtain the solid MC1'. The obtained solid MC1' was dried under reduced pressure at 50°C to obtain the phosphorescent compound MC1 (81.8 g) of Comparative Example 1. Moreover, the obtained residue MC2' was dried over reduced pressure at 50°C to obtain the phosphorescent compound MC2 (125 g) of Example 1.

The HPLC area percent of the phosphorescent compound MC1 was 99.2%. The HPLC area percent of the phosphorescent compound MC2 was 99.5% or more.

The amount ($C^1$) of chlorine atoms contained in the phosphorescent compound MC1 was 16 ppm by mass. The amount ($C^1$) of chlorine atoms contained in the phosphorescent compound MC2 was 9 ppm by mass.

The amount of bromine atoms contained in the phosphorescent compound MC1 was 2 ppm by mass. The amount of bromine atoms contained in the phosphorescent compound MC2 was below the detection limit (0 ppm by mass).

The maximum peak wavelength of emission spectrum of the phosphorescent compounds MC1 and MC2 was 471 nm.

<Example 2> Synthesis of Phosphorescent Compound MC3

[Chemical Formula 102]

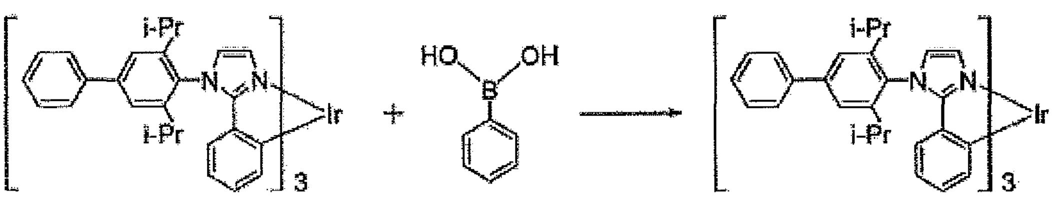

Metal Complex MC2

Metal Complex MC3

The phosphorescent compound MC2 was dehalogenated. Specifically, the atmosphere within a lightproof reaction vessel was replaced with argon gas, then the reaction vessel was charged with the phosphorescent compound MC2 (40.0 g), phenylboronic acid (3.67 g), (di-tert-butyl-(4-dimethylaminophenyl)phosphine)dichloropalladium (II) (0.64 g), and toluene (210 mL) and the mixture was heated to 90°C. A 40% by mass aqueous solution of tetrabutylammonium hydroxide (97 mL) was added thereto and then the mixture was stirred at 90°C for 120 hours. Then, the reaction solution was cooled to room temperature, and then an aqueous layer was removed to obtain an organic layer. The obtained organic layer was washed with ion exchanged water (100 mL) twice, dried over anhydrous magnesium sulfate, and then filtered and the residue was washed with toluene (250 mL). The obtained filtrate was concentrated under reduced pressure to obtain a solid. The obtained solid was purified by silica gel column chromatography (a mixed solvent of hexane and dichloromethane) and then recrystallized in a mixed solvent of toluene and acetonitrile. The obtained solid

was dried under reduced pressure at 50°C to obtain the phosphorescent compound MC3 (34.2 g) as a yellow solid.

The HPLC area percent of the phosphorescent compound MC3 was 99.5% or more. Moreover, the amount ($C^1$) of chlorine atoms contained in the phosphorescent compound MC3 was below the detection limit (0 ppm by mass). The amount of bromine atoms contained in the phosphorescent compound MC3 was below the detection limit (0 ppm by mass).

$^1$H-NMR of the phosphorescent compounds MC3 and LC-MS was as follows.

$^1$H-NMR (300 MHz, $CD_2Cl_2$-$d_2$) $\delta$ (ppm) = 7.83-7.85 (m, 6H), 7.67-7.54 (m, 12H), 7.52-7.43 (m, 3H), 6.94 (d, 6H), 6.80 (d, 3H), 6.67 (t, 3H), 6.52 (t, 3H), 6.44-6.36 (m, 3H), 2.94-2.74 (m, 3H), 2.55-2.36 (m, 3H), 1.35 (d, 9H), 1.19-1.09 (m, 18H), 1.06 (d, 9H).

LC-MS (APCI, positive): m/z = 1331.6 $[M+H]^+$

The maximum peak wavelength of emission spectrum of the phosphorescent compound MC3 was 471 nm.

<Example D1> Fabrication and Evaluation of Light-Emitting Device D1

(Fabrication of Light-Emitting Device D1)

(Formation of Anode and Hole Injecting Layer)

The anode was formed by depositing an ITO film with a thickness of 45 nm on a glass substrate by sputtering. On the anode, ND-3202 (a product manufactured by Nissan Chemical Industries, Ltd.), a hole injecting material, was deposited to a thickness of 35 nm by spin coating. A hole injecting layer was formed by heating in the atmosphere on a hot plate at 50°C for 3 minutes and further at 230°C for 15 minutes.

(Formation of Hole Transporting Layer)

A polymer compound HTL-1 was dissolved at a concentration of 0.7% by mass in xylene. The resultant xylene solution was spin-coated on the hole injecting layer to form a film with a thickness of 20 nm, and the film was heated on a hot plate at 180°C for 60 minutes under a nitrogen gas atmosphere to form a hole transporting layer.

(Formation of Light-Emitting Layer D1)

Compound HM-1 and the phosphorescent compound MC3 (Compound HM-1/Phosphorescent compound MC3 = 75% by mass/25% by mass) were dissolved at a concentration of 2.0% by mass to toluene (a product manufactured by Kanto Chemical Co., Inc.: for electronics (EL grade)). Using the obtained toluene solution, a film with a thickness of 75 nm was formed on a hole transporting layer by spin coating and heating at 130°C for 10 minutes under a nitrogen gas atmosphere to form a light-emitting layer.

$C^1$ is 0 ppm by mass, $C^H$ is 0 ppm by mass, $W^1$ is 0.25, $W^H$ is 0.75, and $C^1W^1 + C^HW^H$ is 0 ppm by mass in the theoretical values calculated from the charged amounts.

(Formation of Electron Transporting Layer)

A polymer compound ETL-1 was dissolved at a concentration of 0.25% by mass in 2,2,3,3,4,4,5,5-octafluoro-1-pentanol. The resultant 2,2,3,3,4,4,5,5-octafluoro-1-pentanol solution was spin-coated on the light-emitting layer to form a film with a thickness of 10 nm, and the film was heated at 130°C for 10 minutes under a nitrogen gas atmosphere to form an electron transporting layer.

(Formation of Cathode)

The substrate on which the electron transporting layer had been formed was placed in a vapor deposition machine, then reducing the pressure to $1.0 \times 10^{-4}$ Pa or less, followed by vapor-depositing sodium fluoride to a thickness of about 4 nm on the electron transporting layer, and then vapor-deposited aluminum to a thickness of about 80 nm on the sodium fluoride layer. After the vapor deposition, sealing was performed using a glass substrate to fabricate a light-emitting device D1.

(Evaluation of Light-Emitting Device)

EL luminescence was observed by applying voltage to the light-emitting device D1. At 100 cd/$m^2$, the luminous efficiency was 4.98 [lm/W] and the CIE chromaticity coordinate (x, y) was (0.19, 0.42). At 1000 cd/$m^2$, the CIE chromaticity

coordinate (x, y) was (0.20, 0.44). AT 5000 cd/$m^2$, the CIE chromaticity coordinate (x, y) was (0.19, 0.42). After setting the current value so that the initial luminance was 1000 cd/$m^2$, the light-emitting device was driven at a constant current and the time until the luminance reached 95% of the initial luminance (hereinafter, also referred to as "LT95") was measured to be 229 hours. After setting the current value so that the initial luminance was 5000 cd/$m^2$, the light-emitting device was driven at a constant current and the time until the luminance reached 50% of the initial luminance (hereinafter, also referred to as "LT50") was measured to be 25.3 hours. The result is illustrated in Table 5.

<Example D2> Fabrication and Evaluation of Light-Emitting Device D2

(Fabrication of Light-Emitting Device D2)

A light-emitting device D2 was fabricated in the same manner as in Example D1 except that (Formation of Light-Emitting Layer D2) was conducted in place of (Formation of Light-Emitting Layer D1) in Example D1.

(Formation of Light-Emitting Layer D2)

Compound HM-1, the phosphorescent compound MC3, and the phosphorescent compound MC2 (Compound HM-1/phosphorescent compound MC3/phosphorescent compound MC2 = 75% by mass/22.5% by mass/2.5% by mass) were dissolved at a concentration of 2.0% by mass to toluene (a product manufactured by Kanto Chemical Co., Inc.: for electronics (EL grade)). Using the obtained toluene solution, a film with a thickness of 75 nm was formed on a hole transporting layer by spin coating and heating at 130°C for 10 minutes under a nitrogen gas atmosphere to form a light-emitting layer.

$C^1$ is 0.90 ppm by mass, $C^H$ is 0 ppm by mass, $W^1$ is 0.25, $W^H$ is 0.75, and $C^1W^1 + C^HW^H$ is 0.23 ppm by mass in the theoretical values calculated from the charged amounts.

(Evaluation of Light-Emitting Device)

EL luminescence was observed by applying voltage to the Light-Emitting Device D2. At 100 cd/$m^2$, the luminous efficiency was 7.60 [lm/W] and the CIE chromaticity coordinate (x, y) was (0.20, 0.44). At 1000 cd/$m^2$, the CIE chromaticity coordinate (x, y) was (0.20, 0.44). At 5000 cd/$m^2$, the CIE chromaticity coordinate (x, y) was (0.19, 0.42). After setting the current value so that the initial luminance was 1000 cd/$m^2$, the light-emitting device was driven at a constant current and the time until the luminance reached 95% of the initial luminance was measured to be 124 hours. After setting the current value so that the initial luminance was 5000 cd/$m^2$, the light-emitting device was driven at a constant current and the time until the luminance reached 50% of the initial luminance was measured to be 24.5 hours. The result is illustrated in Table 5.

<Example D3> Fabrication and Evaluation of Light-Emitting Device D3

(Fabrication of Light-Emitting Device D3)

A light-emitting device D3 was fabricated in the same manner as in Example D1 except that (Formation of Light-Emitting Layer D3) was conducted in place of (Formation of Light-Emitting Layer D1) in Example D1.

(Formation of Light-Emitting Layer D3)

Compound HM-1, the phosphorescent compound MC3, and the phosphorescent compound MC2 (Compound HM-1/phosphorescent compound MC3/phosphorescent compound MC2 = 75% by mass/12.5% by mass/12.5% by mass) were dissolved at a concentration of 2.0% by mass to toluene (a product manufactured by Kanto Chemical Co., Inc.: for electronics (EL grade)). Using the obtained toluene solution, a film with a thickness of 75 nm was formed on a hole transporting layer by spin coating and heating at 130°C for 10 minutes under a nitrogen gas atmosphere to form a light-emitting layer.

$C^1$ is 4.50 ppm by mass, $C^H$ is 0 ppm by mass, $W^1$ is 0.25, $W^H$ is 0.75, and $C^1W^1 + C^HW^H$ is 1.13 ppm by mass in the theoretical values calculated from the charged amounts.

(Evaluation of Light-Emitting Device)

EL luminescence was observed by applying voltage to the light-emitting device D3. At 100 cd/$m^2$, the luminous efficiency was 8.07 [lm/W] and the CIE chromaticity coordinate (x, y) was (0.19, 0.43). At 1000 cd/$m^2$, the CIE chromaticity

coordinate (x, y) was (0.19, 0.43). At 5000 cd/$m^2$, the CIE chromaticity coordinate (x, y) was (0.19, 0.42). After setting the current value so that the initial luminance was 1000 cd/$m^2$, the light-emitting device was driven at a constant current and the time until the luminance reached 95% of the initial luminance was measured to be 41 hours. After setting the current value so that the initial luminance was 5000 cd/$m^2$, the light-emitting device was driven at a constant current and the time until the luminance reached 50% of the initial luminance was measured to be 24.8 hours. The result is illustrated in Table 5.

<Example D4> Fabrication and Evaluation of Light-Emitting Device D4

(Fabrication of Light-Emitting Device D4)

A light-emitting device D4 was fabricated in the same manner as in Example D1 except that (Formation of Light-Emitting Layer D4) was conducted in place of (Formation of Light-Emitting Layer D1) in Example D1.

(Formation of Light-Emitting Layer D4)

Compound HM-1 and the phosphorescent compound MC2 (Compound HM-1/Phosphorescent compound MC2 = 75% by mass/25% by mass) were dissolved at a concentration of 2.0% by mass to toluene (a product manufactured by Kanto Chemical Co., Inc.: for electronics (EL grade)). Using the obtained toluene solution, a film with a thickness of 75 nm was formed on a hole transporting layer by spin coating and heating at 130°C for 10 minutes under a nitrogen gas atmosphere to form a light-emitting layer.

$C^1$ is 9.00 ppm by mass, $C^H$ is 0 ppm by mass, $W^1$ is 0.25, $W^H$ is 0.75, and $C^1W^1 + C^HW^H$ is 2.25 ppm by mass in the theoretical values calculated from the charged amounts.

(Evaluation of Light-Emitting Device)

EL luminescence was observed by applying voltage to the light-emitting device D4. At 100 cd/$m^2$, the luminous efficiency was 8.48 [lm/W] and the CIE chromaticity coordinate (x, y) was (0.20, 0.44). At 1000 cd/$m^2$, the CIE chromaticity coordinate (x, y) was (0.20, 0.44). At 5000 cd/$m^2$, the CIE chromaticity coordinate (x, y) was (0.19, 0.41). After setting the current value so that the initial luminance was 1000 cd/$m^2$, the light-emitting device was driven at a constant current and the time until the luminance reached 95% of the initial luminance was measured to be 18 hours. After setting the current value so that the initial luminance was 5000 cd/$m^2$, the light-emitting device was driven at a constant current and the time until the luminance reached 50% of the initial luminance was measured to be 24.4 hours. The result is illustrated in Table 5.

<Example D5> Fabrication and Evaluation of Light-Emitting Device D5

(Fabrication of Light-Emitting Device D5)

A light-emitting device D5 was fabricated in the same manner as in Example D1 except that (Formation of Light-Emitting Layer D5) was conducted in place of (Formation of Light-Emitting Layer D1) in Example D1.

(Formation of Light-Emitting Layer D5)

Compound HM-1, the phosphorescent compound MC2, and the phosphorescent compound MC1 (Compound HM-1/phosphorescent compound MC2/phosphorescent compound MC1 = 75% by mass/12.5% by mass/12.5% by mass) were dissolved at a concentration of 2.0% by mass to toluene (a product manufactured by Kanto Chemical Co., Inc.: for electronics (EL grade)). Using the obtained toluene solution, a film with a thickness of 75 nm was formed on a hole transporting layer by spin coating and heating at 130°C for 10 minutes under a nitrogen gas atmosphere to form a light-emitting layer.

$C^1$ is 12.5 ppm by mass, $C^H$ is 0 ppm by mass, $W^1$ is 0.25, $W^H$ is 0.75, and $C^1W^1 + C^HW^H$ is 3.13 ppm by mass in the theoretical values calculated from the charged amounts.

(Evaluation of Light-Emitting Device)

EL luminescence was observed by applying voltage to the light-emitting device D5. At 100 cd/$m^2$, the luminous efficiency was 6.34 [lm/W] and the CIE chromaticity coordinate (x, y) was (0.19, 0.42). At 1000 cd/$m^2$, the CIE chromaticity coordinate (x, y) was (0.19, 0.43). At 5000 cd/$m^2$, the CIE chromaticity coordinate (x, y) was (0.19, 0.41). After setting

the current value so that the initial luminance was 1000 cd/$m^2$, the light-emitting device was driven at a constant current and the time until the luminance reached 95% of the initial luminance was measured to be 8.3 hours. After setting the current value so that the initial luminance was 5000 cd/$m^2$, the light-emitting device was driven at a constant current and the time until the luminance reached 50% of the initial luminance was measured to be 24.0 hours. The result is illustrated in Table 5.

<Comparative Example CD1> Fabrication and Evaluation of Light-Emitting Device CD1

(Fabrication of Light-Emitting Device CD1)

A light-emitting device CD1 was fabricated in the same manner as in Example D1 except that (Formation of Light-Emitting Layer CD1) was conducted in place of (Formation of Light-Emitting Layer D1) in Example D1.

(Formation of Light-Emitting Layer CD1)

Compound HM-1 and the phosphorescent compound MC1 (Compound HM-1/Phosphorescent compound MC1 = 75% by mass/25% by mass) were dissolved at a concentration of 2.0% by mass to toluene (a product manufactured by Kanto Chemical Co., Inc.: for electronics (EL grade)). Using the obtained toluene solution, a film with a thickness of 75 nm was formed on a hole transporting layer by spin coating and heating at 130°C for 10 minutes under a nitrogen gas atmosphere to form a light-emitting layer.

$C^1$ is 16.0 ppm by mass, $C^H$ is 0 ppm by mass, $W^1$ is 0.25, $W^H$ is 0.75, and $C^1W^1 + C^HW^H$ is 4.00 ppm by mass in the theoretical values calculated from the charged amounts.

(Evaluation of Light-Emitting Device)

EL luminescence was observed by applying voltage to the light-emitting device CD1. At 100 cd/$m^2$, the luminous efficiency was 6.33 [lm/W] and the CIE chromaticity coordinate (x, y) was (0.19, 0.41). At 1000 cd/$m^2$, the CIE chromaticity coordinate (x, y) was (0.20, 0.43). At 5000 cd/$m^2$, the CIE chromaticity coordinate (x, y) was (0.19, 0.41). After setting the current value so that the initial luminance was 1000 cd/$m^2$, the light-emitting device was driven at a constant current and the time until the luminance reached 95% of the initial luminance was measured to be 5.3 hours. After setting the current value so that the initial luminance was 5000 cd/$m^2$, the light-emitting device was driven at a constant current and the time until the luminance reached 50% of the initial luminance was measured to be 26.3 hours. The result is illustrated in Table 5.

[Table 5]

| | Light emitting device | Light-Emitting Layer | | | |
|---|---|---|---|---|---|
| | | Material | Material Ratio (weight%) | $C^1$ (ppm) | $C^1W^1+C^HW^H$ (ppm) |
| Example D1 | D1 | HM-1/MC3 | 75/25 | 0 | 0 |
| Example D2 | D2 | HM-1/MC3/MC2 | 75/22.5/2.5 | 0.90 | 0.23 |
| Example D3 | D3 | HM-1/MC3/MC2 | 75/12.5/12.5 | 4.50 | 1.13 |
| Example D4 | D4 | HM-1/MC2 | 75/25 | 9.00 | 2.25 |
| Example D5 | D5 | HM-1/MC2/MC1 | 75/12.5/12.5 | 12.5 | 3.13 |
| Comparative Example CD1 | CD1 | HM-1/MC1 | 75/25 | 16.0 | 4.00 |

[Table 5](continued)

| | Light emitting device | LT95 (Time) | LT50 (Time) | Luminous Efficiency [lm/w] |
|---|---|---|---|---|
| Example D1 | D1 | 229 | 25.3 | 4.98 |
| Example D2 | D2 | 124 | 24.5 | 7.60 |
| Example D3 | D3 | 41 | 24.8 | 8.07 |
| Example D4 | D4 | 18 | 24.4 | 8.48 |
| Example D5 | D5 | 8.3 | 24.0 | 6.34 |
| Comparative Example CD1 | CD1 | 5.3 | 26.3 | 6.33 |

## Claims

**1.** A composition in which a phosphorescent compound represented by formula (1) and a host material are blended with each other, wherein the amount of chlorine atoms contained as impurities in the phosphorescent compound is 3.5 ppm by mass or less with respect to the total amount of solid contents blended in the composition:

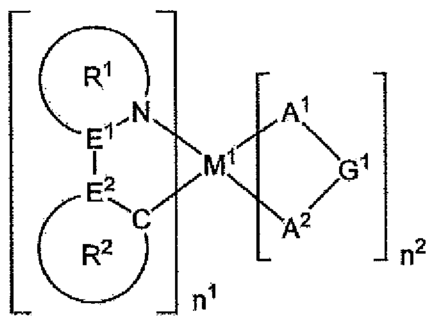

(1)

wherein,

$M^1$ represents a rhodium atom, a palladium atom, an iridium atom or a platinum atom;
$n^1$ represents an integer of 1 or more, $n^2$ represents an integer of 0 or more, and $n^1 + n^2$ is 2 or 3;
when $M^1$ is a rhodium atom or an iridium atom, $n^1 + n^2$ is 3;
when $M^1$ is a palladium atom or a platinum atom, $n^1 + n^2$ is 2;

$E^1$ and $E^2$ each independently represent a carbon atom or a nitrogen atom, provided that at least one of $E^1$ and $E^2$ is a carbon atom;
$R^1$ ring represents a 5-membered aromatic heterocyclic ring, and the ring may have a substituent;
when there are a plurality of the substituents, they may be the same or different, or may be bonded to each other to form a ring together with the atoms to which the substituents are bonded;
when there are a plurality of $R^1$ rings, they may be the same or different;
$R^2$ ring represents an aromatic hydrocarbon ring or an aromatic heterocyclic ring, and these rings each may have a substituent;
when there are a plurality of the substituents, they may be the same or different, or may be bonded to each other to form a ring together with the atoms to which they are bonded;
when there are a plurality of $R^2$ rings, they may be the same or different;
the substituent that the $R^1$ ring may have and the substituent that the $R^2$ ring may have may be bonded to each other to form a ring together with the atoms to which the substituents are bonded;
$A^1$-$G^1$-$A^2$ represents an anionic bidentate ligand;
$A^1$ and $A^2$ each independently represent a carbon atom, an oxygen atom or a nitrogen atom, and these atoms each may be atoms constituting a ring;
$G^1$ represents a single bond or an atomic group constituting the bidentate ligand together with $A^1$ and $A^2$; and when there are a plurality of $A^1$-$G^1$-$A^2$, they may be the same or different.

2. The composition according to claim 1, wherein the total amount of chlorine atoms contained as impurities in the phosphorescent compound and chlorine atoms contained as impurities in the host material is 3.5 ppm by mass or less with respect to the total amount of solid contents blended in the composition.

3. The composition according to claim 2, wherein the total amount of chlorine atoms is 0.8 ppm by mass or less with respect to the total amount of solid contents blended in the composition.

4. The composition according to claim 2, wherein the total amount of chlorine atoms is 0.01 ppm by mass or more and 3.0 ppm by mass or less with respect to the total amount of solid contents blended in the composition.

5. The composition according to any one of claims 2 to 4, wherein the total amount of chlorine atoms is 0.1 ppm by mass or more and 0.8 ppm by mass or less with respect to the total amount of solid contents blended in the composition.

6. The composition according to any one of claims 1 to 5, wherein the phosphorescent compound is a compound represented by formula (1-A):

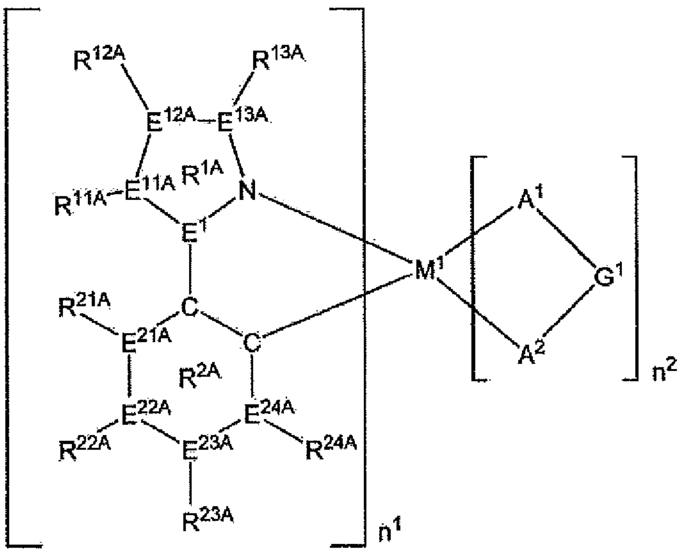

(1-A)

wherein,

$M^1$, $n^1$, $n^2$, $E^1$, and $A^1$-$G^1$-$A^2$ represent the meanings the same as described above;

$E^{11A}$, $E^{12A}$, $E^{13A}$, $E^{21A}$, $E^{22A}$, $E^{23A}$, and $E^{24A}$ each independently represent a nitrogen atom or a carbon atom; when there are a plurality of $E^{11A}$, $E^{12A}$, $E^{13A}$, $E^{21A}$, $E^{22A}$, $E^{23A}$, and $E^{24A}$, they may each be the same or different at each occurrence;
when $E^{11A}$ is a nitrogen atom, $R^{11A}$ may be present or absent;
when $E^{12A}$ is a nitrogen atom, $R^{12A}$ may be present or absent;
when $E^{13A}$ is a nitrogen atom, $R^{13A}$ may be present or absent;
when $E^{21A}$ is a nitrogen atom, $R^{21A}$ is absent;
when $E^{22A}$ is a nitrogen atom, $R^{22A}$ is absent;
when $E^{23A}$ is a nitrogen atom, $R^{23A}$ is absent;
when $E^{24A}$ is a nitrogen atom, $R^{24A}$ is absent;
$R^{11A}$, $R^{12A}$, $R^{13A}$, $R^{21A}$, $R^{22A}$, $R^{23A}$, and $R^{24A}$ each independently represent a hydrogen atom, an alkyl group, a cycloalkyl group, an alkoxy group, a cycloalkoxy group, an aryl group, an aryloxy group, a monovalent heterocyclic group, a substituted amino group, or a fluorine atom, and these groups each may have a substituent; when there are a plurality of $R^{11A}$, $R^{12A}$, $R^{13A}$, $R^{21A}$, $R^{22A}$, $R^{23A}$, and $R^{24A}$, they may each be the same or different at each occurrence;
$R^{11A}$ and $R^{12A}$, $R^{12A}$ and $R^{13A}$, $R^{11A}$ and $R^{21A}$, $R^{21A}$ and $R^{22A}$, $R^{22A}$ and $R^{23A}$, and $R^{23A}$ and $R^{24A}$ each may be bonded to each other to form a ring together with the atoms to which they are bonded;
$R^{1A}$ ring represents a triazole ring or a diazole ring, constituted of a nitrogen atom, $E^1$, $E^{11A}$, $E^{12A}$, and $E^{13A}$ ; and $R^{2A}$ ring represents a benzene ring, a pyridine ring, or a pyrimidine ring, constituted of two carbon atoms, $E^{21A}$, $E^{22A}$, $E^{23A}$, and $E^{24A}$.

7. The composition according to claim 6, wherein the phosphorescent compound is a compound represented by formula (1-A1), a compound represented by formula (1-A2), a compound represented by formula (1-A3), or a compound represented by formula (1-A4):

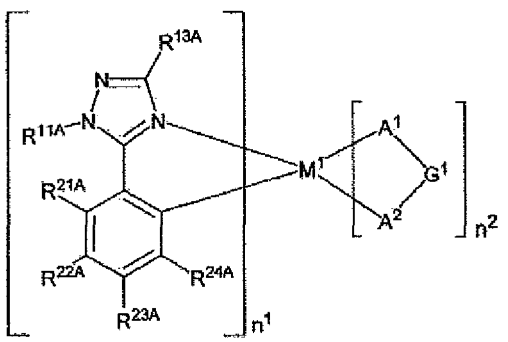

(1-A1)

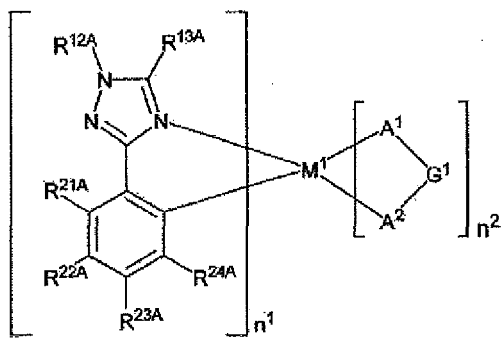

(1-A2)

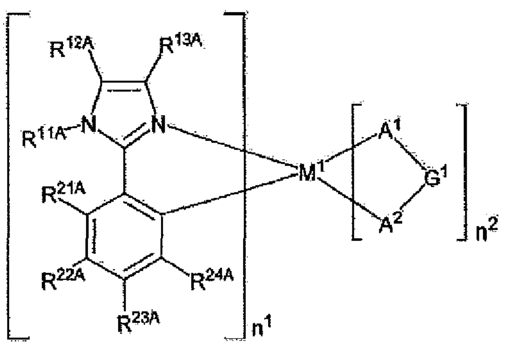

(1-A3)

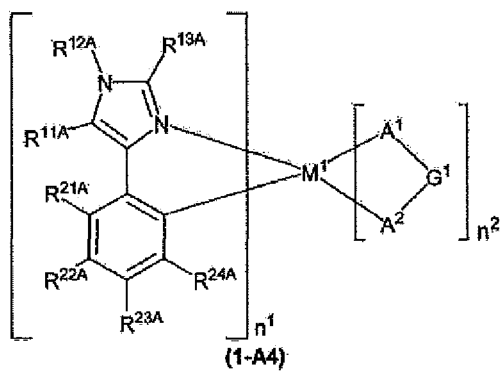

(1-A4)

wherein $M^1$, $n^1$, $n^2$, $R^{11A}$, $R^{12A}$, $R^{13A}$, $R^{21A}$, $R^{22A}$, $R^{23A}$, $R^{24A}$, and $A^1$-$G^1$-$A^2$ represent the same meanings as described above.

8. The composition according to claim 7,
wherein,
the phosphorescent compound is a compound represented by formula (1-A3); and
the $R^{11A}$ is an aryl group that may have a substituent.

**9.** The composition according to any one of claims 1 to 8, wherein the host material is a compound represented by formula (H-1):

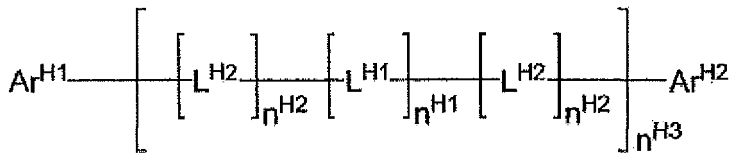

**(H-1)**

wherein,

$Ar^{H1}$ and $Ar^{H2}$ each independently represent an aryl group or a monovalent heterocyclic group, and these groups each may have a substituent;
$n^{H1}$ and $n^{H2}$ each independently represent 0 or 1;
when there are a plurality of $n^{H1}$, they may be the same or different;
the plurality of $n^{H2}$ may be the same or different;
$n^{H3}$ represents an integer of 0 or more;
$L^{H1}$ represents an arylene group, a divalent heterocyclic group, or a group represented by $-[C(R^{H11})_2]n^{H11}-$, and these groups each may have a substituent;
when there are a plurality of $L^{H1}$, they may be the same or different;
$n^{H11}$ represents an integer of 1 or more and 10 or less;
$R^{H11}$ represents a hydrogen atom, an alkyl group, a cycloalkyl group, an alkoxy group, a cycloalkoxy group, an aryl group, or a monovalent heterocyclic group, and these groups each may have a substituent;
the plurality of $R^{H11}$ may be the same or different, or may be bonded to each other to form a ring together with the carbon atom to which they are bonded;
$L^{H2}$ represents a group represented by $-N(-L^{H21}-R^{H21})-$;
when there are a plurality of $L^{H2}$, they may be the same or different;
$L^{H21}$ represents a single bond, an arylene group, or a divalent heterocyclic group, and these groups each may have a substituent; and
$R^{H11}$ represents a hydrogen atom, an alkyl group, a cycloalkyl group, an aryl group, or a monovalent heterocyclic group, and these groups each may have a substituent.

**10.** The composition according to any one of claims 1 to 8, further comprising at least one material selected from the group consisting of a hole transporting material, a hole injecting material, an electron transporting material, an electron injecting material, a light emitting material, an antioxidant, and a solvent.

**11.** A phosphorescent compound represented by formula (1),
wherein the amount of chlorine atoms contained as impurities is 15 ppm by mass or less with respect to the total amount of the phosphorescent compound:

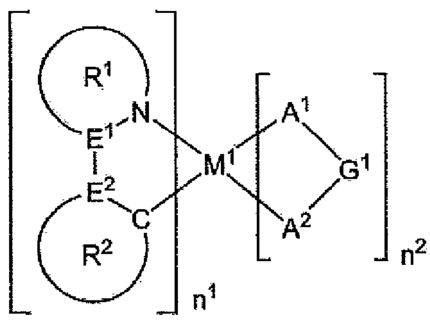

**(1)**

wherein,

$M^1$ represents a rhodium atom, a palladium atom, an iridium atom, or a platinum atom;
$n^1$ represents an integer of 1 or more, $n^2$ represents an integer of 0 or more, and $n^1 + n^2$ is 2 or 3;
when $M^1$ is a rhodium atom or an iridium atom, $n^1 + n^2$ is 3;
when $M^1$ is a palladium atom or a platinum atom, $n^1 + n^2$ is 2;

$E^1$ and $E^2$ each independently represent a carbon atom or a nitrogen atom, provided that at least one of $E^1$ and $E^2$ is a carbon atom;
$R^1$ ring represents a 5-membered aromatic heterocyclic ring, the ring may have a substituent;
when there are a plurality of the substituents, they may be the same or different, or may be bonded to each other to form a ring together with the atoms to which they are bonded;
when there are a plurality of $R^1$ rings, they may be the same or different;
$R^2$ ring represents an aromatic hydrocarbon ring or an aromatic heterocyclic ring, and these rings each may have a substituent;
when there are a plurality of the substituents, they may be the same or different, or may be bonded to each other to form a ring together with the atoms to which they are bonded;
when there are a plurality of $R^2$ rings, they may be the same or different;
the substituent that the $R^1$ ring may have and the substituent that the $R^2$ ring may have may be bonded to each other to form a ring together with the atoms to which they are bonded;
$A^1$-$G^1$-$A^2$ represents an anionic bidentate ligand;
$A^1$ and $A^2$ each independently represent a carbon atom, an oxygen atom, or a nitrogen atom, and these atoms each may be atoms constituting a ring;
$G^1$ represents a single bond or an atomic group constituting the bidentate ligand together with $A^1$ and $A^2$; and when there are a plurality of $A^1$-$G^1$-$A^2$, they may be the same or different.

**12.** The phosphorescent compound according to claim 11, wherein the phosphorescent compound represented by formula (1) is a compound represented by formula (1-A):

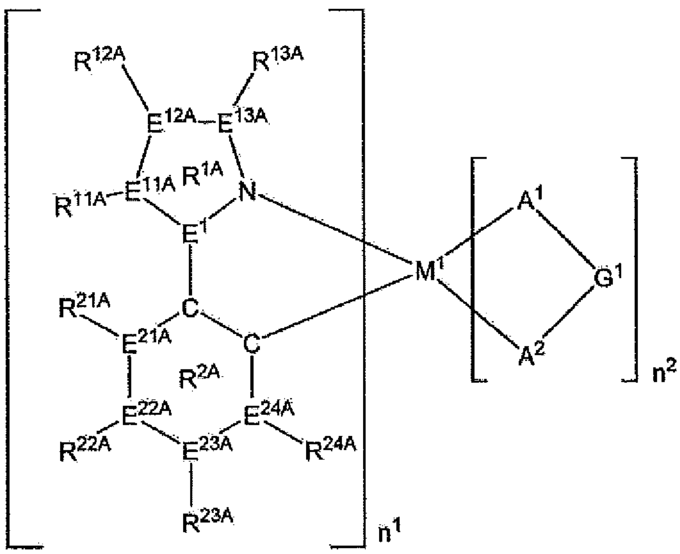

(1-A)

wherein,

$M^1$, $n^1$, $n^2$, $E^1$, and $A^1$-$G^1$-$A^2$ represent the meanings the same as described above;
$E^{11A}$, $E^{12A}$, $E^{13A}$, $E^{21A}$, $E^{22A}$, $E^{23A}$, and $E^{24A}$ each independently represent a nitrogen atom or a carbon atom;
when there are a plurality of $E^{11A}$, $E^{12A}$, $E^{13A}$, $E^{21A}$, $E^{22A}$, $E^{23A}$, and $E^{24A}$, they may each be the same or different at each occurrence;
when $E^{11A}$ is a nitrogen atom, $R^{11A}$ may be present or absent;
when $E^{12A}$ is a nitrogen atom, $R^{12A}$ may be present or absent;
when $E^{13A}$ is a nitrogen atom, $R^{13A}$ may be present or absent;
when $E^{21A}$ is a nitrogen atom, $R^{21A}$ is absent;
when $E^{22A}$ is a nitrogen atom, $R^{22A}$ is absent;
when $E^{23A}$ is a nitrogen atom, $R^{23A}$ is absent;
when $E^{24A}$ is a nitrogen atom, $R^{24A}$ is absent;
$R^{11A}$, $R^{12A}$, $R^{13A}$, $R^{21A}$, $R^{22A}$, $R^{23A}$, and $R^{24A}$ each independently represent a hydrogen atom, an alkyl group, a cycloalkyl group, an alkoxy group, a cycloalkoxy group, an aryl group, an aryloxy group, a monovalent heterocyclic group, a substituted amino group, or a fluorine atom, and these groups each may have a substituent;
when there are a plurality of $R^{11A}$, $R^{12A}$, $R^{13A}$, $R^{21A}$, $R^{22A}$, $R^{23A}$ and $R^{24A}$, they may each be the same or

different at each occurrence;
$R^{11A}$ and $R^{12A}$, $R^{12A}$ and $R^{13A}$, $R^{11A}$ and $R^{21A}$, $R^{21A}$ and $R^{22A}$, $R^{22A}$ and $R^{23A}$, and $R^{23A}$ and $R^{24A}$ each may be bonded to each other to form a ring together with the atoms to which they are bonded;
$R^{1A}$ ring represents a triazole ring or a diazole ring, constituted of a nitrogen atom, $E^1$, $E^{11A}$, $E^{12A}$, and $E^{13A}$ ; and
$R^{2A}$ ring represents a benzene ring, a pyridine ring, or a pyrimidine ring, constituted of two carbon atoms, $E^{21A}$, $E^{22A}$, $E^{23A}$, and $E^{24A}$

**13.** The phosphorescent compound according to claim 12, wherein the phosphorescent compound is a compound represented by formula (1-A1), a compound represented by formula (1-A2), a compound represented by formula (1-A3), or a compound represented by formula (1-A4):

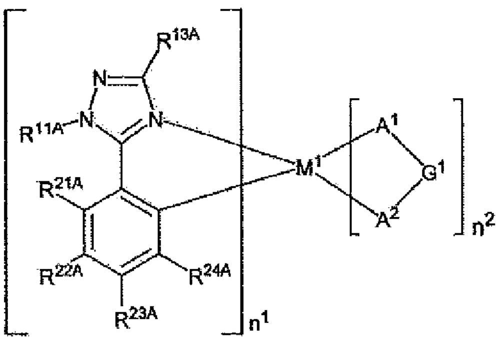

(1-A1)

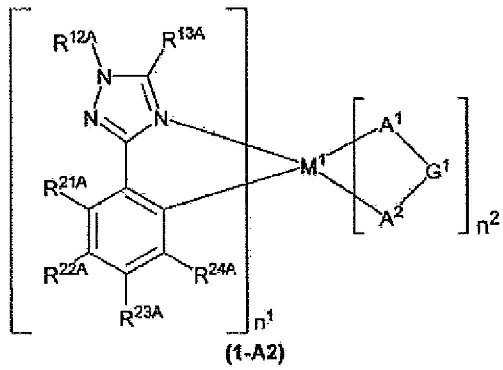

(1-A2)

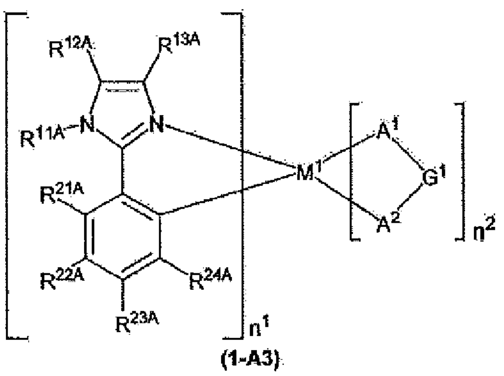

(1-A3)

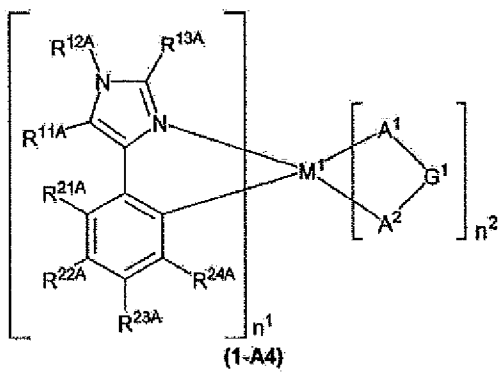

(1-A4)

wherein, $M^1$, $n^1$, $n^2$, $R^{11A}$, $R^{12A}$, $R^{13A}$, $R^{21A}$, $R^{22A}$, $R^{23A}$, $R^{24A}$, and $A^1$-$G^1$-$A^2$ represent the meanings the same as described above.

**14.** The phosphorescent compound according to claim 13,
wherein,
the phosphorescent compound is a compound represented by formula (1-A3); and
the $R^{11A}$ is an aryl group that may have a substituent.

**15.** The phosphorescent compound according to any one of claims 11 to 14, wherein the amount of chlorine atoms contained as the impurities is 0.9 ppm by mass or less.

**16.** The phosphorescent compound according to any one of claims 11 to 14, wherein the amount of chlorine atoms contained as the impurities is 0.01 ppm by mass or more and 12 ppm by mass or less.

**17.** The phosphorescent compound according to claim 15 or 16, wherein the amount of chlorine atoms contained as the impurities is 0.1 ppm by mass or more and 0.9 ppm by mass or less.

**18.** A light-emitting device comprising an organic layer comprising a composition according to any one of claims 1 to 10.

**19.** A light-emitting device comprising an organic layer in which a phosphorescent compound according to any one of claims 11 to 17 is blended.

## INTERNATIONAL SEARCH REPORT

International application No.
PCT/JP2017/002377

A. CLASSIFICATION OF SUBJECT MATTER

*H01L51/50*(2006.01)i, *C07D233/58*(2006.01)i, *C07F15/00*(2006.01)i, *C09K11/06*(2006.01)i, *C07D409/14*(2006.01)n

According to International Patent Classification (IPC) or to both national classification and IPC

B. FIELDS SEARCHED

Minimum documentation searched (classification system followed by classification symbols)
H01L51/50, C07D233/58, C07F15/00, C09K11/06, C07D409/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2011-216903 A (Idemitsu Kosan Co., Ltd.), 27 October 2011 (27.10.2011), paragraphs [0010], [0038], [0052], [0060], [0067]<br>& US 2007/0172698 A1<br>paragraphs [0028] to [0029], [0100], [0146], [0169], [0194] to [0201]<br>& WO 2005/084083 A1 & EP 1722603 A1<br>& KR 10-2007-0004678 A & CN 1926925 A<br>& KR 10-1120393 B1 & TW 200534745 A | 1-7,9-13, 15-19<br>8,14 |
| Y | WO 2013/031794 A1 (Konica Minolta Holdings, Inc.), 07 March 2013 (07.03.2013), paragraph [0289]<br>(Family: none) | 8,14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 April 2017 (18.04.17) | 25 April 2017 (25.04.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/002377

C (Continuation). DOCUMENTS CONSIDERED TO BE RELEVANT

| Category* | Citation of document, with indication, where appropriate, of the relevant passages | Relevant to claim No. |
|---|---|---|
| Y | US 2011/057559 A1 (UNIVERSAL DISPLAY CORP.), 10 April 2011 (10.04.2011), paragraph [0061] & JP 2011-509247 A & JP 5575661 B2 & JP 2014-196342 A & WO 2009/085344 A2 & WO 2009/086028 A2 & EP 2240446 A2 & KR 10-2010-0099327 A & CN 101939296 A & TW 200927742 A & TW 200940504 A & TW 201422601 A & KR 10-2015-0087423 A & TW 201602090 A | 8,14 |
| A | JP 2014-13910 A (Konica Minolta, Inc.), 23 January 2014 (23.01.2014), paragraphs [0102], [0107] & WO 2007/108327 A1 | 6-8,12-14 |
| A | JP 2013-147551 A (Sumitomo Chemical Co., Ltd.), 01 August 2013 (01.08.2013), paragraphs [0065] to [0073] (Family: none) | 6-7,12-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006121811 A
- WO 2007097153 A
- JP 2013048190 A
- WO 2004101707 A
- JP 2013147449 A
- JP 2013147450 A
- JP 2013147551 A
- WO 2006037458 A
- JP 2007220772 A
- JP 2007077078 A
- WO 2005084083 A
- WO 2009086028 A
- WO 2009096202 A
- JP 2009046408 A
- JP 2009267255 A
- JP 2009239279 A
- JP 2012033845 A
- JP 2012216821 A
- JP 2012216822 A
- JP 2012216815 A
- WO 2015145871 A
- WO 2013146806 A
- WO 2005049546 A
- JP 2010189630 A


### Non-patent literature cited in the description


- The fourth series of experimental chemistry (in Japanese). MaruzenJunkudo Bookstores Co., Ltd, 1993
- The fifth series of experimental chemistry (in Japanese). MaruzenJunkudo Bookstores Co., Ltd, 2007
- New series of experimental chemistry (in Japanese). MaruzenJunkudo Bookstores Co., Ltd, 1975
- Manual for organic chemistry experiments (in Japanese). Kagaku-Dojin Publishing Co., Inc, 1988